(19) Europäisches Patentamt
European Patent Office
Office européen des brevets

(11) EP 1 392 676 B1

(12) **EUROPEAN PATENT SPECIFICATION**

(45) Date of publication and mention of the grant of the patent:
**13.12.2006 Bulletin 2006/50**

(21) Application number: **02714914.5**

(22) Date of filing: **15.02.2002**

(51) Int Cl.:
*C07D 333/62* (2006.01) *C07D 333/54* (2006.01)
*C07D 498/04* (2006.01) *C07D 495/04* (2006.01)
*A61K 31/381* (2006.01) *A61P 25/18* (2006.01)

(86) International application number:
**PCT/US2002/004560**

(87) International publication number:
**WO 2002/066468 (29.08.2002 Gazette 2002/35)**

(54) **HETEROCYCLIC UREA DERIVATIVES AND THEIR USE AS DOPAMINE D3 RECEPTOR LIGANDS**

HETEROCYCLISCHE HARNSTOFFDERIVATE UND DEREN VERWENDUNG ALS DOPAMINE D3 RECEPTOR LIGANDEN

NOUVEAUX DERIVES D'UREE HETEROCYCLIQUES ET LEUR UTILISATION EN TANT QUE LIGANDS DES RECEPTEURS DE LA DOPAMINE D3

(84) Designated Contracting States:
**AT BE CH CY DE DK ES FI FR GB GR IE IT LI LU MC NL PT SE TR**

(30) Priority: **16.02.2001 US 269654 P**
**23.07.2001 GB 0117950**

(43) Date of publication of application:
**03.03.2004 Bulletin 2004/10**

(73) Proprietor: **AVENTIS PHARMACEUTICALS INC.**
**Bridgewater, NJ 08807 (US)**

(72) Inventors:
- **HENDRIX, James, A.**
  **Hillsborough, NJ 08876 (US)**
- **STRUPCZEWSKI, Joseph, T.**
  **Flemington, NJ 08822 (US)**
- **BORDEAU, Kenneth, J.**
  **Kintnersville, PA 18930 (US)**
- **BROOKS, Sarah**
  **Arlington, MA 02174 (US)**
- **HEMMERLE, Horst**
  **D-65812 Bad Soden (DE)**
- **URMANN, Matthias**
  **D-65760 Eschborn (DE)**
- **ZHAO, Xu-Yang**
  **Bridgewater, NJ 08807 (US)**
- **MUELLER, Paul, Justin.**
  **Hoboken, NJ 07030 (US)**

(74) Representative: **Fischer, Hans-Jürgen et al**
**Sanofi-Aventis Deutschland GmbH**
**Patent- und Lizenzabteilung**
**Industriepark Höchst**
**Gebäude K 801**
**65926 Frankfurt am Main (DE)**

(56) References cited:
**EP-A- 0 395 313** **WO-A-00/67847**
**US-A- 5 393 761** **US-A- 5 990 114**

- **DATABASE MEDLINE [Online] December 1995 (1995-12) ISHIZUMI K ET AL: "Succinimide derivatives. II. Synthesis and antipsychotic activity of N-[4-[4-(1,2-benzisothiazol-3-yl)-1-pipera zinyl]butyl]-1,2-cis-cyclohexanedicarboximide (SM-9018) and related compounds." Database accession no. NLM8582016 XP002204864 -& DATABASE CROSSFIRE BEILSTEIN [Online] BEILSTEIN INSTITUT ZUR FOERDERUNG DER WISSENSCHAFTEN, FRANKFURT AM MAIN, DE; BRN 7469776, XP002204865 & ISHIZUMI K ET AL: "Succinimide derivatives. II. Synthesis and antipsychotic activity of N-(4-(4-(1,2-benzisothiazol-3-yl)-1-pipera zinyl)butyl)1,2-cis-cyclohexanedicarboximi de (SM-9018) and related compounds" CHEMICAL & PHARMACEUTICAL BULLETIN, JAPAN DEC 1995, vol. 43, no. 12, December 1995 (1995-12), pages 2139-2151,**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- **MURRAY P J ET AL: "A novel series of arylpiperazines with high affinity and selectivity for the dopamine D3 receptor" BIOORGANIC & MEDICINAL CHEMISTRY LETTERS, OXFORD, GB, vol. 5, no. 3, 2 February 1995 (1995-02-02), pages 219-222, XP004135762 ISSN: 0960-894X**
- **SOKOLOFF P ET AL: "MOLECULAR CLONING AND CHARACTERIZATION OF A NOVEL DOPAMINE RECEPTOR(D3) AS A TARGET FOR NEUROLEPTICS" NATURE, MACMILLAN JOURNALS LTD. LONDON, GB, vol. 347, 13 September 1990 (1990-09-13), pages 146-151, XP000652293 ISSN: 0028-0836**


Remarks:

The file contains technical information submitted after the application was filed and not included in this specification

## Description

BACKGROUND OF THE INVENTION

The subject invention relates to novel heterocyclic derivatives that selectively bind to the dopamine $D_3$ receptor. The therapeutic effects of currently available antipsychotic agents (neuroleptics) are generally believed to be exerted via blockade of $D_2$ receptors; however this mechanism is also thought to be responsible for undesireable extrapyramidal side effects (eps) associated with many neuroleptic agents. Without wishing to be bound by theory, it has been suggested that blockade of the dopamine $D_3$ receptor may give rise to beneficial antipsychotic activity without significant eps. (see for example Sokoloff et al, Nature, 1990; 347: 146-151; and Schwartz et al, Clinical Neuropharmacology, Vol 16, No. 4, 295-314, 1993). This receptor is found in high abundance in brain regions associated with emotional and cognitive functions. Compounds that selectively bind to the dopamine $D_3$ receptor are useful in treating certain central nervous system disorders. These central nervous system disorders include the following indications:

1) Psychoses (including schizophrenia) - See, for example, *Biochem Pharmacol,* 1992, 3(4), 659-66; *Clin Neuropharmacol,* 1993,16(4), 295-314; *Neuropsychopharmacology,* 1997, 16(6), 375-84; *Am J Psychiatry,* 1999,156(4), 610-616;*Psychopharmacology (Berl),* 1995, 120(1), 67-74.

2) Substance dependence and substance abuse - See, for example, *Neuroreport,* 1997, 8(9-10), 2373-2377; *J Pharmacol Exp Ther,* 1996, 278(3), 1128-37; *Brain Res Mol Brain Res,* 1997, 45(2), 335-9.

3) Mood Disorders (including mania, depressive disorders and bipolar disorders) - See, for example, *Clin Neuropharmacol,* 1998, 21 (3),176-80; *Am J Med Genet,* 1998, 81 (2), 192-4; *J Clin Psychiatry,* 1995, 56(11), 514-518; *J Clin Psychiatry,* 1995, 56(9), 423-429; *Am J Med Genet,* 1995, 60(3), 234-237; *Pharmacopsychiatry,* 1999, 32(4), 127-135; *J Affect Disord,* 1999, 52(1-3), 275-290; *Am J Psychiatry,* 1999,156(4), 610-616.

4) dyskinetic disorders (including Parkinson's Disease, Parkinsonism, Neuroleptic-Induced Tardive Dyskinesia and Gilles de la Tourette Syndrome) - See, for example, *Clin Neuropharmacol,* 2000, 23(1), 34-44; *Eur J Pharmacol,* 1999, 385(1), 39-46.

5) sleep disorders (including narcolepsy) - The $D_3$ agonist pramipexole causes narcolepsy. A $D_3$ antagonist would be useful for reversing this undesireable side effect. See *Aust Fam Physician,* 1999, 28(7), 737; *Neurology,* 1999, 52(9), 1908-1910.

6) anxiety disorders (including obsessive compulsive disorders) - See, for example, *Physiol Behav,* 1997, 63(1), 137-141; *J Clin Psychiatry,* 1995, 56(9), 423-429; *J Psychiatry Neurosci,* 2000, 25(2),185; *J Affect Disord,* 1999, 56(2-3), 219-226.

7) nausea - Dopamine antagonists are used alone and in combination with 5HT3 antagonists. See, for example, *Support Care Cancer,* 1998, 6(1), 8-12; *Support Care Cancer,* 2000, 8(3), 233-237; *Eur J Anaesthesiol,* 1999, 16 (5), 304-307.

8) dementia - See, for example, *Behav Brain Res,* 2000, 109(1), 99-111; *Neuroscience,* 1999, 89(3), 743-749.

Dopamine $D_3$ receptor ligands are also useful for the treatment of renal dysfunction. (See WO 200067847)

SUMMARY OF THE INVENTION

This invention relates to a class of compounds and pharmaceutically acceptable salts thereof which are selective modulators of dopamine $D_3$ receptors. The compounds may act as agonists, partial agonists, antagonists or allosteric modulators of dopamine $D_3$ receptors, and are useful for a variety of therapeutic applications.

In another aspect, the invention relates to a method for treating central nervous system disorders associated with the dopamine $D_3$ receptor activity in a patient in need of such treatment comprising administering to the subject a therapeutically effective amount of a compound described herein for alleviation of such disorder. The central nervous system conditions or disorders that may be treated with these compounds include Psychotic Disorders, Substance Dependence, Substance Abuse, Dyskinetic Disorders (e.g. Parkinson's Disease, Parkinsonism, Neuroleptic-Induced Tardive Dyskinesia, Gilles de la Tourette Syndrome and Huntington's Disease), Nausea, Dementia, Anxiety Disorders, Sleep Disorders, Circadian Rhythm Disorders and Mood Disorders.

In yet another aspect, the subject invention is directed toward a pharmaceutical composition comprising an effective amount of a compound described herein with a pharmaceutically-acceptable carrier or diluent optionally in conjunction with one or more dopamine $D_1$, $D_2$, $D_4$, $D_5$ or 5HT receptor antagonists.

In yet another aspect, the subject invention is directed towards processes for the preparation of the class of compounds described herein.

Also within the scope of this invention are methods for using these novel compounds as imaging agents for dopamine $D_3$ receptors. Methods of using these compounds as imaging agents are presented, as are intermediates and methods for making the imaging agents.

DETAILED DESCRIPTION OF THE INVENTION

In accordance.with the present invention, there are provided compounds of formula I A compound of the formula (I):

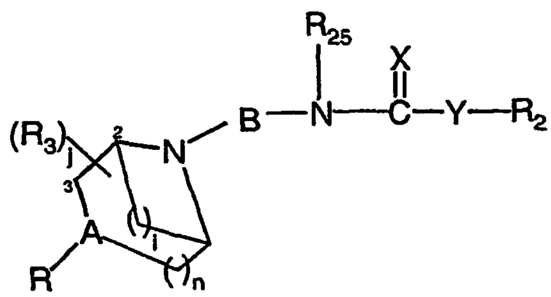

I

wherein

A is CH or N;
Y is O or $NR_1$
wherein $R_1$ is

a) hydrogen;
b) $C_1$-$C_6$alkyl; or
c)

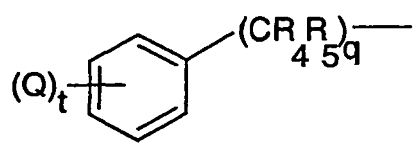

wherein

each Q is independently hydrogen, $C_1$-$C_6$alkyl, halogen or trifluoromethyl;
each $R_4$, and $R_5$ is independently hydrogen or $C_1$-$C_6$alkyl;
t is 0 or 1; and
q is 0 or 1;

j is 0, 1 or 2;
n is 1 or 2;
when n is 1, i is 0 or 2;
when n is 2, i is 0;
$R_3$ is hydrogen, $C_1$-$C_6$alkyl or

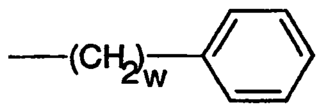

wherein w is 1, 2 or 3;
X is O or S;
$R_{25}$ is hydrogen or

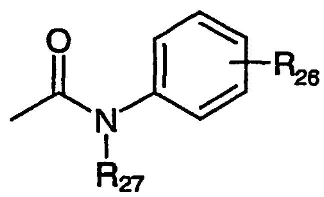

wherein $R_{27}$ is hydrogen or $C_1$-$C_6$alkyl and $R_{26}$ is hydrogen, $C_1$-$C_6$alkoxy, halogen or $C_1$-$C_6$alkyl that is unsaturated or saturated;
-B- is a group selected from (a) - (e):

(a) $-(CR_{21}R_{22})_m-$
(b)

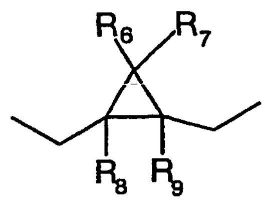

(c)

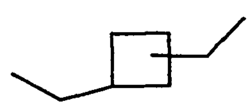

(d)

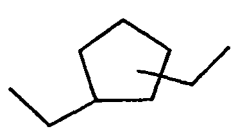

(e)

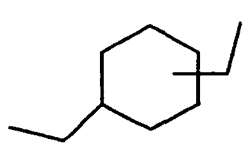

wherein

m is 3, 4, 5, or 6;
each $R_{21}$ and $R_{22}$ is independently hydrogen, $C_1$-$C_6$alkyl or -$(CH_2)_a$OH wherein a is 0,1 or 2;
$R_6$, $R_7$, $R_8$ and $R_9$ are each independently hydrogen, halogen or $C_1$-$C_3$alkyl with the proviso that when $R_8$ is $C_1$-$C_3$alkyl, $R_9$ is not $C_1$-$C_3$alkyl;

R is a group selected from (a) - (c): .

a)

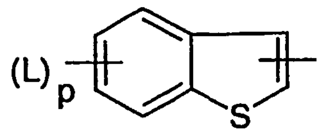

b)

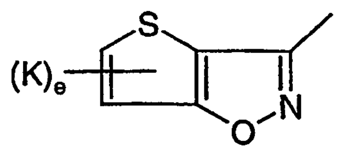

c)

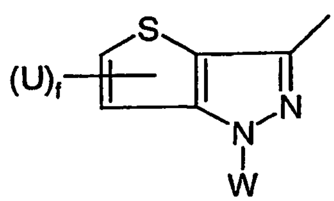

wherein

each L is independently hydrogen, $C_1$-$C_6$alkyl, halogen or trifluoromethyl;
each U is independently hydrogen, $C_1$-$C_6$alkyl, CHO, halogen, -$(CH_2)_b$OH, or -$(CH_2)_b$O$C_1$-$C_6$alkyl wherein b is 1 or 2;
each K is independently hydrogen, $C_1$-$C_6$alkyl, CHO, halogen, -$(CH_2)_b$OH, or -$(CH_2)_b$O$C_1$-$C_6$alkyl wherein b is as hereinbefore defined; W is hydrogen or

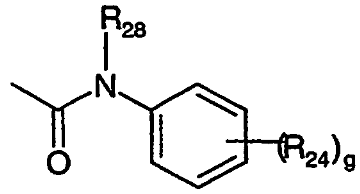

wherein $R_{28}$ is hydrogen or $C_1$-$C_6$alkyl;

each $R_{24}$ is independently trifluoromethyl, trifluoromethoxy, $C_1$-$C_6$alkoxy or halogen; and
g is 0, 1 or 2;

p is 0, 1 or 2;
e is 0, 1 or 2;
f is 0, 1, or 2;

$R_2$ is a group selected from (a) - (t):

(a)

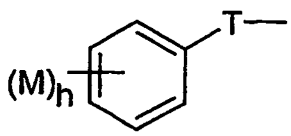

(b)

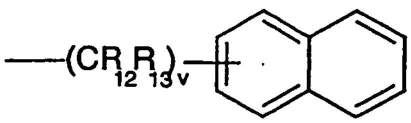

(c)

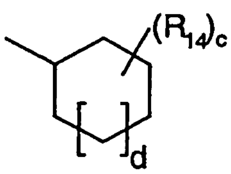

(d)

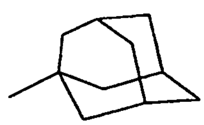

(e)

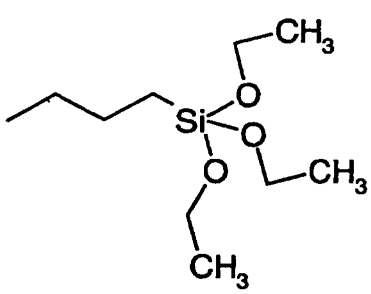

(f) $-C_1-C_{12}alkyl$
(g) $-(CR_{15}R_{16})_sCO_2C_1-C_6alkyl$
(h)

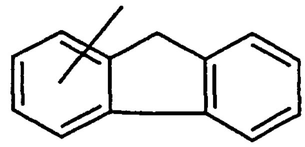

(i)

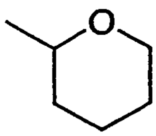

(j)

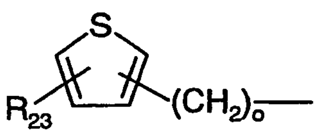

(k)

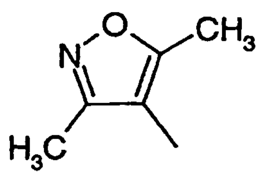

(l) $-CH_2CH_2SO_2CH_3$
(m) $-(CR_{17}R_{18})_rOC_1-C_2alkyl$
(n) $-CH_2CH_2OPh$
(o) $-(CR_{19}R_{20})_qCZ_3$
(p) $-CO_2Ph$
(q) $-COCV_3$
(r)

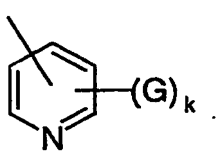

(s)

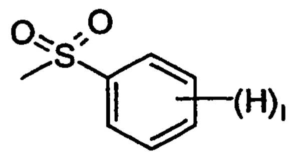

(t)

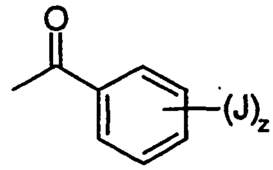

wherein

-T- is selected from (i) or (ii):

(i) $-(CR_{10}R_{11})_u-$
wherein

u is 0, 1 or 2; and each $R_{10}$ and $R_{11}$ is independently hydrogen or $C_1$-$C_6$alkyl;

(ii)

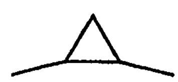

;

each M, G, H and J is independently selected from:

(1) hydrogen;
(2) halogen;
(3) $C_1$-$C_6$alkyl;
(4) $C_1$-$C_6$alkoxy;
(5) phenoxy;
(6) phenyl;
(7) trifluoromethyl;
(8) trifluoromethoxy;
(9) $-CO_2-R_{19}$ wherein $R_{19}$ is hydrogen or $C_1$-$C_6$alkyl;
(10) $-COC_1$-$C_6$alkyl;
(11) hydroxy;
(12) $-(CH_2)-OR_{20}$ wherein $R_{20}$ is hydrogen or $C_1$-$C_6$alkyl;
(13) $-C(CH_2)CH_3$;
(14) $-NO_2$;
(15) $-SCH_3$;
(16) benzyloxy; and
(17) -CN;

each $R_{12}$ and $R_{13}$ is independently hydrogen or $C_1$-$C_6$alkyl;

v is 0 or 1;
$R_{14}$ is hydrogen or $C_1$-$C_4$alkyl;

c is 0, 1 or 2;
d is 0 or 1;
each $R_{15}$ and $R_{16}$ is independently hydrogen or $C_1$-$C_6$alkyl;
s is 0, 1, 2, 3, 4 or 5;
o is 1 or 2;

each $R_{17}$ and $R_{18}$ is independently hydrogen, $C_1$-$C_6$alkyl or

$CO_2C_1$-$C_2$alkyl;
r is 2 or 3;
each $R_{19}$ and $R_{20}$ is independently hydrogen or
$C_1$-$C_2$ alkyl;
$R_{23}$ is hydrogen or $C_1$-$C_4$alkyl;
Z is chlorine or fluorine;
q is 0 or 1;
V is chlorine or fluorine; and
h, k, l, and z are 0, 1, 2, or 3.

The subject invention is directed toward compounds or pharmaceutically acceptable salts of Formula I as depicted above in either racemic or pure stereoisomeric forms.

Terms used herein have the following meanings:

a) "Pharmaceutically acceptable salts" means either an acid addition salt or a basic addition salt which is compatible with the treatment of patients for the intended use.
"Pharmaceutically acceptable acid addition salt" is any non-toxic organic or inorganic acid addition salt of the base compounds represented by Formula I or any of its intermediates. Illustrative inorganic acids which form suitable salts include hydrochloric, hydrobromic, sulfuric and phosphoric acid and acid metal salts such as sodium monohydrogen orthophosphate and potassium hydrogen sulfate. Illustrative organic acids which form suitable salts include the mono-, di- and tri-carboxylic acids. Illustrative of such acids are, for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicyclic, 2-phenoxybenzoic, p-toluenesulfonic acid and sulfonic acids such as methanesulfonic acid and 2-hydroxyethanesulfonic acid. Either the mono- or di-acid salts can be formed, and such salts can exist in either a hydrated, solvated or substantially anhydrous form. In general, the acid addition salts of these compounds are more soluble in water and various hydrophilic organic solvents and which in comparison to their free base forms, generally demonstrate higher melting points.
"Pharmaceutically acceptable basic addition salts" means non-toxic organic or inorganic basic addition salts of the compounds of Formula (I) or any of its intermediates. Examples are alkali metal or alkaline-earth metal hydroxides such as sodium, potassium, calcium, magnesium or barium hydroxides; ammonia, and aliphatic, alicyclic, or aromatic organic amines such as methylamine, trimethylamine and picoline. The selection criteria for the appropriate salt will be known to one skilled in the art.

b) "Stereoisomers" is a general term for all isomers of the individual molecules that differ only in the orientation of their atoms in space. It includes mirror image isomers (enantiomers), geometric (cis/trans) isomers, and isomers of compounds with more than one chiral center that are not mirror images of one another (diastereoisomers).

c) "Alkyl" means a branched or straight chain alkyl or alkylene group, as is appropriate to the formula, specified by the amount of carbons in the alkyl, e.g., $C_1$-$C_6$ alkyl means a one, two, three, four, five or six carbon branched or straight chain alkyl or alkylene, as the case may be, or any ranges thereof, for example, but not limited to, C1-2, C1-3, C1-4, C1-5, C2-3, C2-4, C2-5, C2-C6, C3-C4, C3-5, C3-6, C4-5, C4-6, C5-6, etc.

d) "Patient means a warm blooded animal, such as for example rat, mice, dogs, cats, guinea pigs, and primates such as humans.

e) "Treat" or "treating" means to alleviate symptoms, eliminate the causation of the symptoms either on a temporary or permanent basis, or to prevent or slow the appearance of symptoms of the named disorder or condition.

f) "Therapeutically effective amount" means a quantity of the compound which is effective in treating the named disorder or condition.

g) "Pharmaceutically acceptable carrier" is a non-toxic solvent, dispersant, excipient, adjuvant or other material which is mixed with the active ingredient in order to permit the formation of a pharmaceutical composition, i.e., a dosage form capable of administration to the patient. One example of such a carrier is a pharmaceutically acceptable oil typically used for parenteral administration.

h) "Psychoses" or "Psychotic Disorders" means conditions wherein the patient experiences a major mental disorder of organic and/or emotional origin characterized by derangement of the personality and loss of contact with reality, often with delusions, hallucinations or illusions. Included under the term psychoses are the disorders schizophrenia, schizophreniform disorder, schizoaffective disorder, delusional disorder, brief psychotic disorder, shared psychotic disorder, psychotic disorder not otherwise specified, and substance-induced psychotic disorder, as defined by the Diagnostic and Statistical Manual of Mental Disorders, 4th ed., published 1994 by the American Psychiatric Association, Washington D.C. USA, incorporated herein by reference.

i) "Substance Dependence" means a condition wherein the patient exhibits a maladaptive pattern of substance use, leading to clinically significant impairment or distress. There is a pattern of repeated self-administration that usually results in tolerance, withdrawal, and compulsive drug-taking.

j) "Substance Abuse" means a condition wherein the patient exhibits a maladaptive pattern of substance use manifested by recurrent and significant adverse consequences related to the repeated use of substances. There may be repeated failure to fulfill major role obligations, repeated use in situations in which it is physically hazardous, multiple legal problems, and recurrent social and interpersonal problems. Unlike the criteria for Substance Dependence, the criteria for Substance Abuse do not include tolerance, withdrawal, or a pattern of compulsive use and instead only include the harmful consequences of repeated use.

k) "Parkinson’s Disease" means a slowly progressive neurological condition, characterized by tremor, rigidity, bradykinesia, and postural instability. Other manifestations include depression and dementia.

l) "Parkinsonism" means a condition where the patient exhibits Parkinsonian signs or symptoms (i.e. tremor, muscular rigidity, or akinesia) that develop in association with the use of neuroleptic medication.

m) "Neuroleptic-Induced Tardive Dyskinesia" means a disorder characterized by involuntary movements of the tongue, jaw, trunk, or extremities which have developed in association with the use of neuroleptic medication. The involuntary movements may be choreiform, athetoid or rhythmic.

n) "Gilles de la Tourette Syndrome" means a condition manifested by motor and vocal tics. (A tic is a sudden, rapid, recurrent, nonrhythmic, stereotyped motor movement or vocalization.) The disturbance causes marked distress or significant impairment in social, occupational, or other important areas of functioning. The onset is before age eighteen years and the disturbance is not due to the physiological effects of a substance or general medical condition.

o) "Dementia" means disorders characterized by the development of multiple cognitive deficits that include memory impairment and are due to the direct physiological effects of a general medical condition, to the persisting effects of a substance, or to multiple etiologies (e.g., the combined effects of cerebrovascular disease and Alzheimer’s disease). Memory impairment is required to make the diagnosis of a dementia and is a prominent early symptom. Dementia disorders share a common symptom presentation but are differentiated based on etiology. See Diagnostic and Statistical Manual of Mental Disorders, 4th ed., American Psychiatric Association, for diagnostic criteria.

p) "Anxiety Disorders" means disorders that include Seasonal Affective Disorder, Panic Disorder Without Agoraphobia, Panic Disorder with Agoraphobia, Agoraphobia Without History of Panic Disorder, Specific Phobia, Social Phobia, Obsessive-Compulsive Disorder, Post-traumatic Stress Disorder, Acute Stress Disorder, Generalized Anxiety Disorder, Anxiety Disorder Due to a General Medical Condition, Substance-Induced Anxiety Disorder, and Anxiety Disorder Not Otherwise Specified, as defined by the Diagnostic and Statistical Manual of Mental Disorders, 4th ed.

q) "Sleep Disorders" means disorders that include Primary Sleep Disorders, Sleep Disorder Related to Another Mental Disorder, Sleep Disorder Due to a General Medical Condition, and Substance-Induced Sleep Disorder as defined by the Diagnostic and Statistical Manual of Mental Disorders, 4th ed. Primary Sleep Disorders are those in which none of the etiologies listed below (i.e., another mental disorder, a general medical condition, or a substance) is responsible. Primary Sleep Disorders are presumed to arise from endogenous abnormalities in sleep-wake generating or timing mechanisms, often complicated by conditioning factors. Primary Sleep Disorders in turn are subdivided into Dyssomnias (characterized by abnormalities in the amount, quality, or timing of sleep) and Parasomnias (characterized by abnormal behavioral or physiological events occurring in association with sleep, specific sleep stages, or sleep-wake transitions). A representative example of a Primary Sleep Disorder is Narcolepsy. Narcolepsy is characterized by repeated irresistible attacks of refreshing sleep, cataplexy, and recurrent intrusions of elements of rapid eye movement (REM) sleep into the transition period between sleep and wakefulness.

r) "Mood Disorders" are disorders that have a disturbance in mood as the predominant feature. As defined by the Diagnostic and Statistical Manual of Mental Disorders, 4th ed., Mood Disorders are divided into the Depressive Disorders ("unipolar depression"), the Bipolar Disorders, and two disorders based on etiology - Mood Disorder Due to a General Medical Condition and Substance-Induced Mood Disorder. The Depressive Disorders (i.e., Major Depressive Disorder, Dysthymic Disorder, and Depressive Disorder Not Otherwise Specified) are distinguished from the Bipolar Disorders by the fact that there is no history of ever having had a Manic, Mixed, or Hypomanic Episode. The Bipolar Disorders (i.e., Bipolar I Disorder, Bipolar II Disorder, Cyclothymic Disorder, and Bipolar Disorder Not Otherwise Specified) involve the presence (or history) of Manic Episodes, Mixed Episodes, or Hypomanic Episodes, usually accompanied by the presence (or history) of Major Depressive Episodes.

s) "Circadian Rhythm Disorder" means a persistent or recurrent pattern of sleep disruption leading to excessive sleepiness or insomnia that is due to a mismatch between the sleep-wake schedule required by a person's environment and his or her circadian sleep-wake pattern. The sleep disturbance causes clinically significant distress or impairment in social, occupational, or other important areas of functioning. The disturbance does not occur exclusively during the course of another Sleep Disorder or other mental disorder. The disturbance is not due to the direct physiological effects of a substance (e.g., a drug of abuse, a medication) or a general medical condition.

Preferred compounds are those wherein X is O, Y is NH and -B- is group (a) or group (b). When B is group (a), m is further preferred to be 4. When -B- is group (b), then $R_6$, $R_7$, $R_8$ or $R_9$ are further preferred to be hydrogen. $R_2$ is preferred to be group (a) or group (c). When $R_2$ is group (a), M is further preferred to be hydrogen, $C_1$-$C_6$alkyl, $C_1$-$C_6$alkoxy or phenyl and T is further preferred to be (i) wherein u is 0 or 1. When $R_2$ is group (c), d is further preferred to be 1 and $R_{14}$ is further preferred to be $C_1$-$C_6$alkyl.

Specific embodiments of the invention include the compounds set forth in Table III.

Preferred embodiments of the invention are those compounds of Formula I set forth in Table III that exhibit enhanced D3 potency. Particularly preferred compounds include the following:

1-(4-Methoxy-phenyl)-3-{4-[4-(6-trifluoromethyl-benzo[b]thiophen-3-yl)-piperazin-1-yl]-butyl}-urea

1-p-Tolyl-3-{4-[4-(6-trifluoromethyl-benzo[b]thiophen-3-yl)-piperazin-1-yl]-butyl}-urea

1-{4-[4-(2,6-Difluoro-benzo[b]thiophen-3-yl)-piperazin-1-yl]-butyl}-3-p-tolyl-urea

1-{4-[4-(2-Chloro-6-fluoro-benzo[b]thiophen-3-yl)-piperazin-1-yl]-butyl}-3-(4-chloro-phenyl)-urea

1-(4-Acetyl-phenyl)-3-[4-(4-thieno[2,3-d]isoxazol-3-yl-piperidin-1-yl)-butyl]-urea

1-(4-Ethoxy-phenyl)-3-[4-(4-thieno[2,3-d]isoxazol-3-yl-piperidin-1-yl)-butyl]-urea

1-(4-Ethoxy-phenyl)-3-{4-[4-(6-fluoro-benzo[b]thiophen-3-yl)-[1,4]diazepan-1-yl]-butyl}-urea

1-{4-[4-(6-Fluoro-benzo[b]thiophen-3-yl)-[1,4]diazepan-1-yl]-butyl}-3-(2-fluoro-phenyl)-urea

1-{4-[4-(6-Fluoro-benzo[b]thiophen-3-yl)-[1,4]diazepan-1-yl]-butyl}-3-(3-trifluoromethylphenyl)-urea

1-{4-[4-(6-Fluoro-benzo[b]thiophen-3-yl)-[1,4]diazepan-1-yl]-butyl}-3-(4-methoxy-phenyl)-urea.

1-(3-Imidazol-1-yl-propyl)-3-{(1R, 2R)-2-[4-(6-trifluoromethyl-benzo[b]thiophen-3-yl)-piperazin-1-ylmethyl]-cyclopropylmethyl}-urea (MDL 833306)

The compounds of the present invention may be prepared by various methods. Schemes I through VI show the different ways of preparing the compounds of Formula I.

The compounds of formula (I) of this invention can be synthesized by following or combining one or more of the steps described below, not necessarily in the order presented. Throughout the description of the synthetic steps, the definitions of R, $R_2$, $R_3$, n, j, i, X, Y, B and A are as given above unless otherwise stated or indicated, and other nomenclatures appearing below shall have the same meanings defined in their respective first appearances unless otherwise stated or indicated.

Compounds of formula I wherein Y is N may be prepared according to a process which comprises reacting a compound of formula (II):

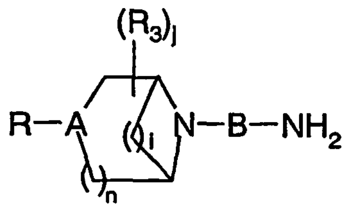

(II)

wherein $R_3$, R, n, i, B, j, A and R as defined in formula I; with a compound of formula (III):

X=C=N-$R_2$ (III)

wherein $R_2$ is as defined in formula I;

Typically, this reaction is carried out in an organic solvent such as, for example, chloroform or tetrahydrofuran at a temperature of about 20 °C to about 25 °C for about 6 to 18 hours.

Compounds of formula I wherein Y is O may be prepared according to a process which comprises reacting a compound of formula (II) with a compound of formula (IV) wherein "LG" is a suitable leaving group selected from bromine, chlorine, iodine or -$OR_2$ in the instance where formula (IV) is available as an anhydride:

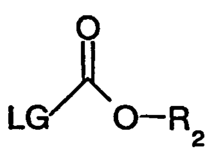

(IV) .

wherein $R_2$ is as defined in formula I;

Typically, this reaction is carried out in an organic solvent such as; for example, chloroform or tetrahydrofuran in the presence of a weak base such as, for example, amberlyte-IRA-67 or triethylamine. The reaction is typically conducted at a temperature of about 20 °C to about 25 °C for about 6 to 18 hours.

Alternatively, compounds of formula I may be prepared according to a process which comprises reacting a compound of formula (V)

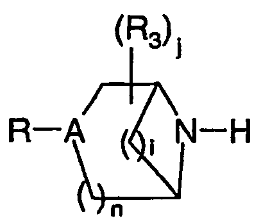

(V)

wherein R, A, n, i, $R_3$ and j are as hereinbefore defined;
with a compound of formula (VI) wherein $R_2$, B and Y are as hereinbefore defined and "LG" is a suitable leaving selected from chlorine, bromine, iodine, mesyl, tosyl, brosyl, triflyl, nosyl, nonaflyl or tresyl:

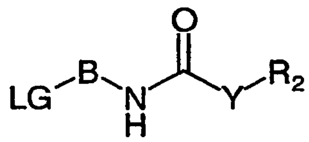

(VI) .

Typically, this reaction is carried out in an aqueous miscible solvent such as, for example, tetrahydrofuran or acetonitrile, in the presence of water and a base such as, for example, potassium carbonate, cesium carbonate, or triethylamine, at a temperature of about 50˚C to about 75˚C for about 12 to 24 hours.

The compound of formula (II) may be prepared via synthetic methods well known in the art. The starting materials are either commercially available or readily synthesized via methods known from the literature. For example, scheme I describes the coupling of an amino-substituted benzthiophene with a commercially-available substituted piperazine. The synthesis is analogous for the un-substituted piperazine analogs. The less sterically hindered piperazine nitrogen is more reactive and cleanly gives a single product in the benzo[b]thiophene coupling. The more sterically hindered nitrogen can then be alkylated with the appropriate alkylating agent.

## SCHEME I

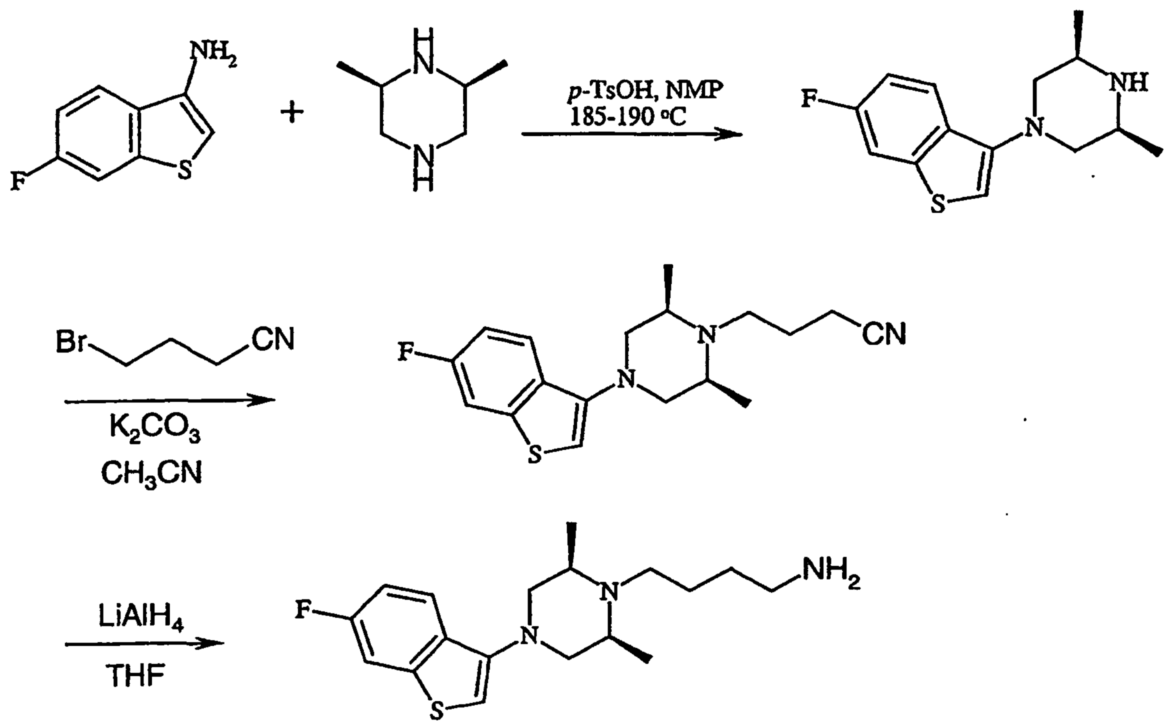


Piperidine-substituted compounds may be prepared via syntheses analogous to those shown in the following reaction schemes II and III.

SCHEME II

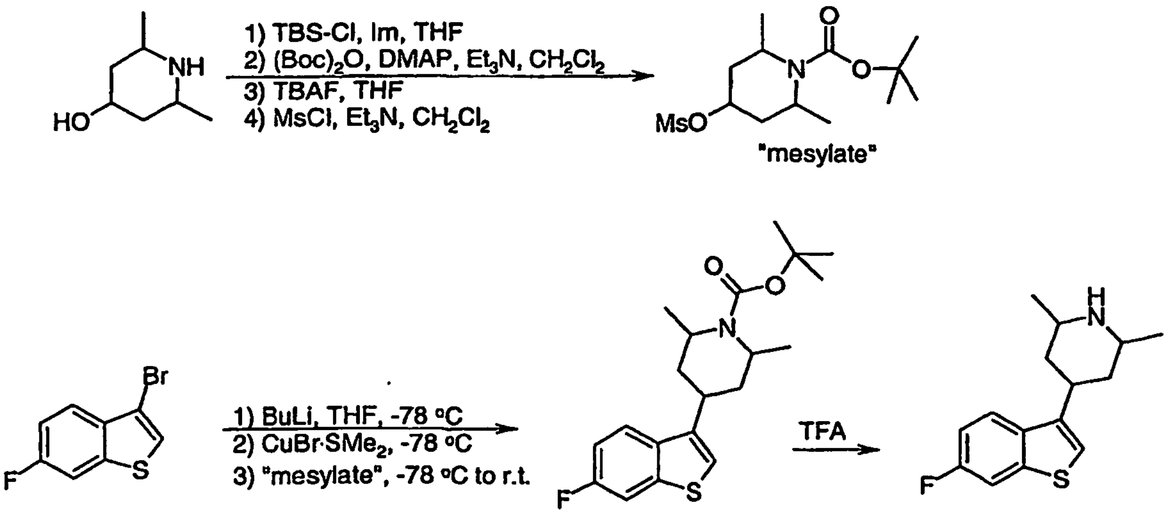


SCHEME III

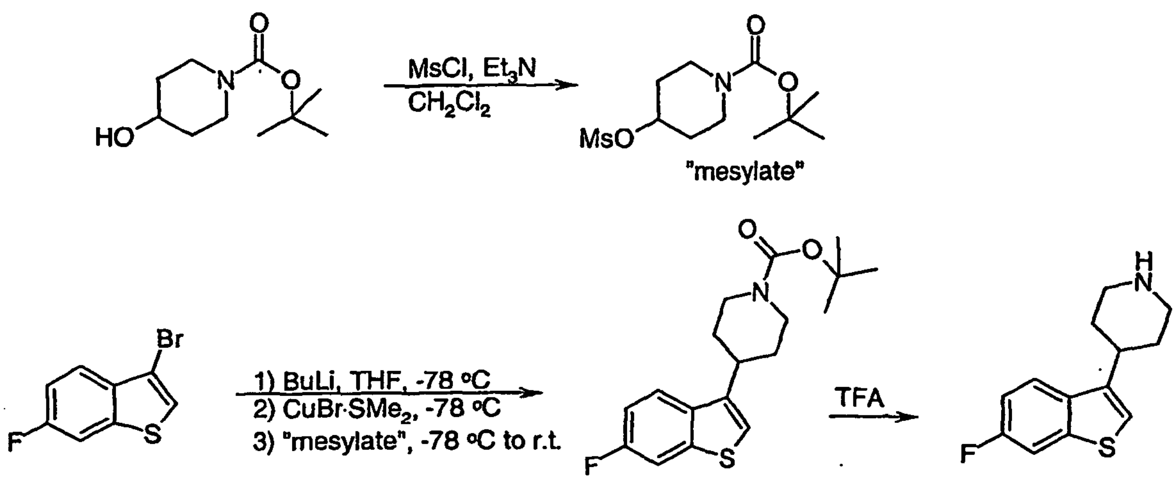


The preparation of various substituted aza- and diazacycloheptanes is described by Treiber et al. in WO 9725324.

Compounds of formula I wherein B contains a carbocycle may be prepared as shown in Scheme IV.

SCHEME IV

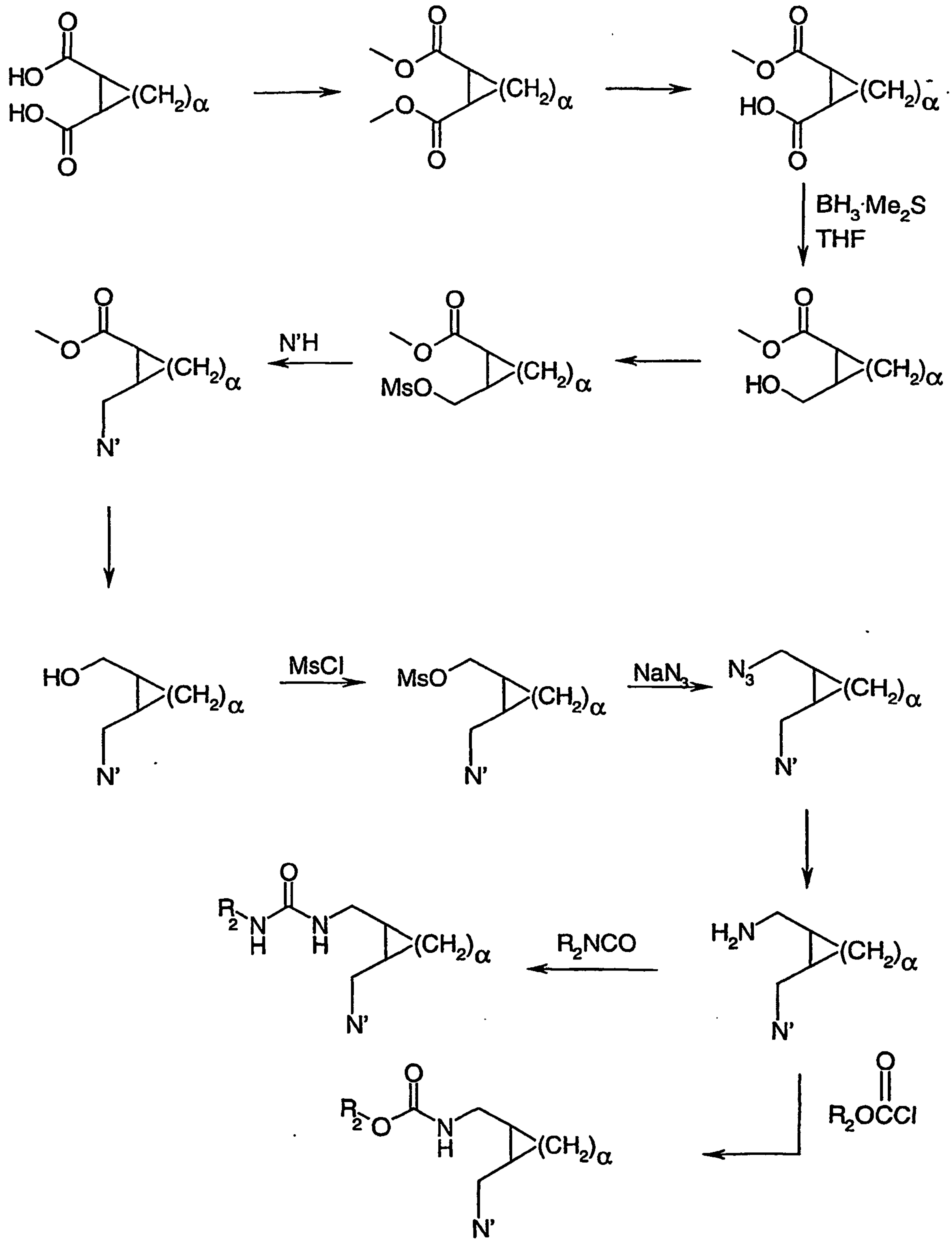


wherein $R_2$ is as hereinbefore defined;
$\alpha$ is 1, 2, 3 or 4; and N'- is

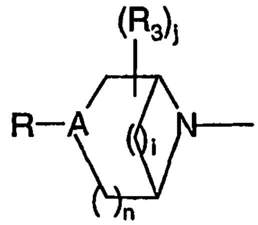

wherein R, A, $R_3$, j, i and n are as hereinbefore defined.

Many of the dicarboxylates or more advanced intermediates that are generically described in Scheme IV are commercially available. Several of these are shown in Table 1. This table is used for illustrative purposes only and is not intended to limit the scope of the present invention in any way.

Table 1 - Starting Materials:

| Structure | Name | CAS # | Supplier |
|---|---|---|---|
| 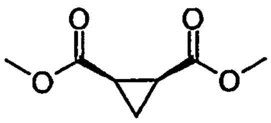 | Dimethyl cis-1,2-cyclopropane dicarboxylate | 826-34-6 | Acros |
| 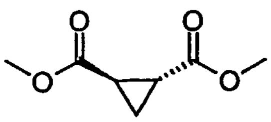 | Dimethyl trans-1,2-cyclopropane dicarboxylate | 826-35-7 | Acros |
| 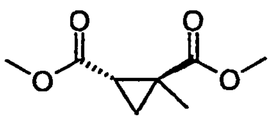 | Dimethyl 1-methyl-trans-1,2-cyclopropane dicarboxylate | 702-92-1 | Acros |
| 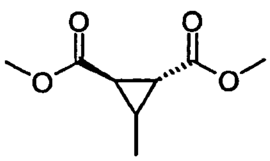 | Dimethyl 3-methyl-trans-1,2-cyclopropane dicarboxylate | 28363-79-3 | Acros |
| 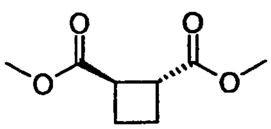 | trans-Cyclobutane-1,2-dicarboxylic acid dimethylester | - | Syntec |
| 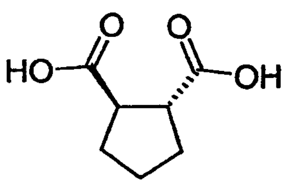 | trans-D, L-1,2-Cyclopentane-dicarboxylic acid | 1461-97-8 | Aldrich |
| 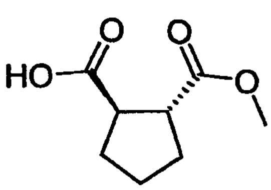 | trans-2-Carbomethoxy cyclopentane-1-carboxylic acid | | Rieke |

(continued)

| Structure | Name | CAS # | Supplier |
|---|---|---|---|
| 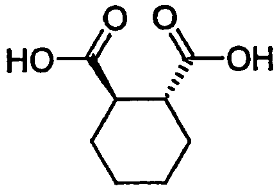 | trans-1,2-Cyclohexane dicarboxylic acid | 2305-32-0 | Aldrich Acros |
| 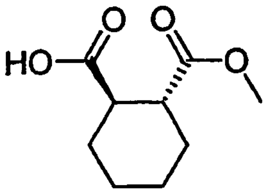 | trans-2-Carbomethoxy cyclohexane-1-carboxylic acid | | Rieke |
| 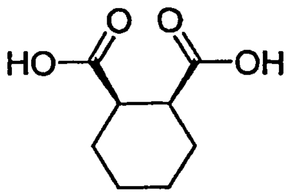 | cis-1,2-Cyclohexane dicarboxylic acid | 610-09-3 | Acros |
| 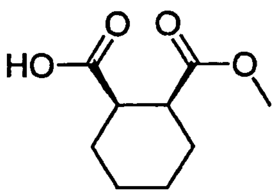 | cis-2-Carbomethoxy cyclohexane-1-carboxylic acid | | Rieke |

When not commercially available, the appropriate starting material may be obtained via standard synthetic methods.

When a compound of formula (I) is obtained as a mixture of enantiomers these may be separated by conventional methods such as crystallization in the presence of a resolving agent, or chromatography, for example using a chiral HPLC column.

Compounds of formula (I) have been found to exhibit affinity for dopamine receptors, in particular $D_3$ receptors, and are expected to be useful in the treatment of disease states which require modulation of such receptors, such as psychotic conditions. Preferred compounds of the present invention are those which have higher affinity for dopamine $D_3$ than dopamine $D_2$ receptors.

A major challenge in antipsychotic research is to produce agents with reduced side effects. Orthostatic hypotension is a common side effect in antipsychotics that is associated with the high potency that these agents have at the alpha-1 adrenergic receptor (hereinafter referred to as "α-1"). A major goal of this work was to find agents with reduced α-1 potency.

The 6-trifluoromethyl benzo[b]thiophenes described herein have a clear and somewhat surprising advantage over the 6-fluoro benzo[b[thiophenes. The 6-fluoro benzo[b]thiophenes are clearly more potent at the alpha-1 adrenergic receptor than are the 6-trifluoromethyl benzo[b]thiophenes. This is shown by comparing pairs of analogs that only differ in substitution at the 6-position of the benzo[b]thiophene (see Table II). In every case, the 6-fluoro benzo[b]thiophene is more potent than the corresponding 6-trifluoromethyl analog. In some cases this small structural difference in substitution at the 6-position produces a dramatic change in α-1 potency. There are several examples wherein 6-fluoro analogs exhibited nanomolar potency while the corresponding 6-trifluoromethyl analog exhibited micromolar potency.

| MDL NUM | MOLSTRUCTURE | halpha 1Ki (nM) | halpha 1 % I |
|---|---|---|---|
| 814949 | 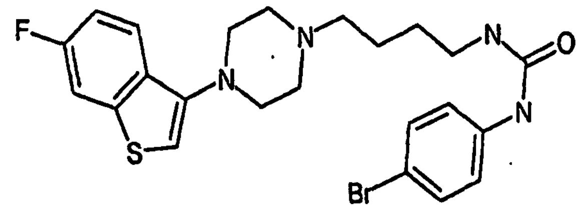 | 18.2 | |
| 816000 | 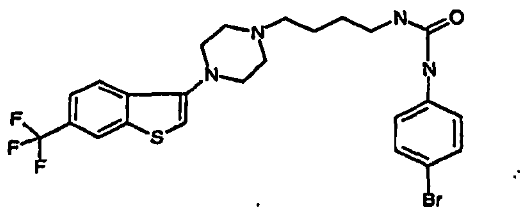 | 234 | |
| 814933 | 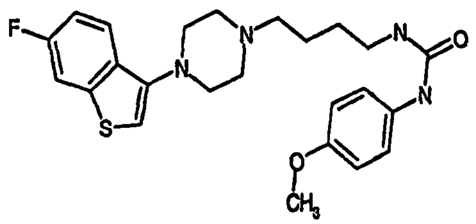 | 1.8 | |
| 815994 | 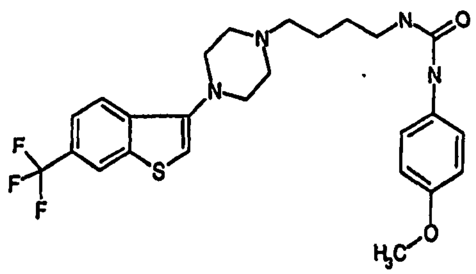 | 1220 | 11.9% Inh @ 1 uM |
| 814937 | 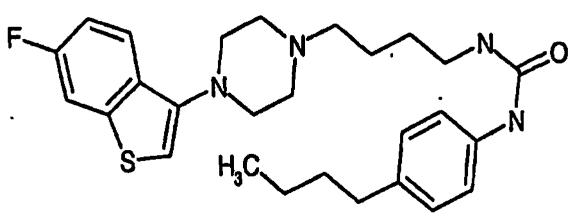 | 532 | |
| 815995 | 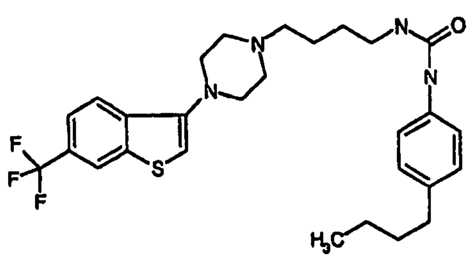 | | 42.4% Inh @ 10 uM |

(continued)

| MDL NUM | MOLSTRUCTURE | halpha 1Ki (nM) | halpha 1 % I |
|---|---|---|---|
| 814941 | 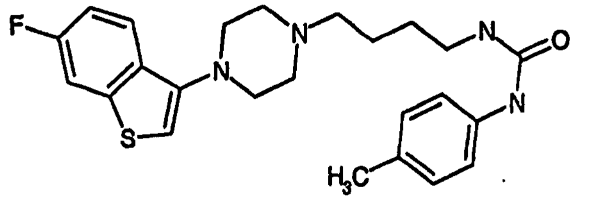 | 13.5 | |
| 815997 | 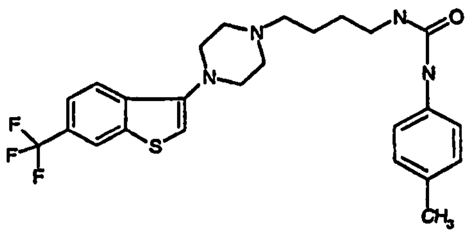 | 936 | 23.5% Inh @ 1 uM |
| 814952 | 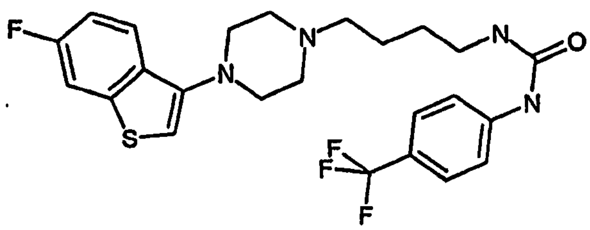 | 44.8 | |
| 816002 | 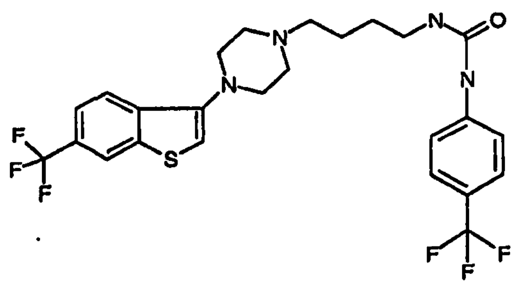 | 435 | 30.2% Inh @ 1 uM |
| 813581 | 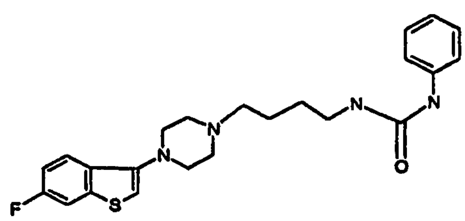 | 1.5 | |
| 816005 | 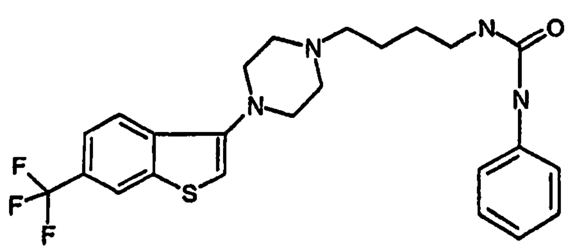 | 56.8 | |

(continued)

| MDL NUM | MOLSTRUCTURE | halpha 1Ki (nM) | halpha 1 % I |
|---|---|---|---|
| 814932 | 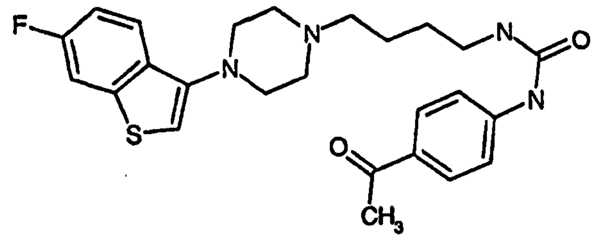 | 2.2 | |
| 815993 | 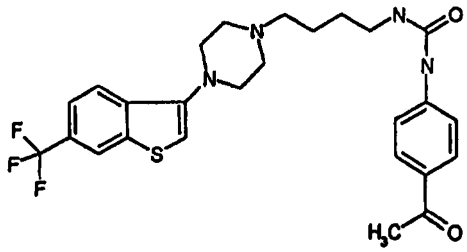 | 74.9 | |
| 814945 | 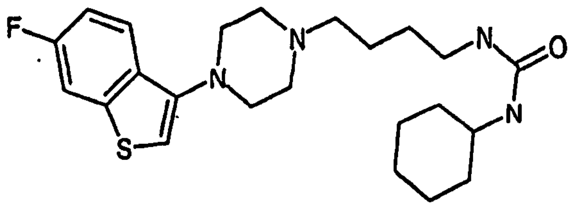 | 4.5 | |
| 816001 | 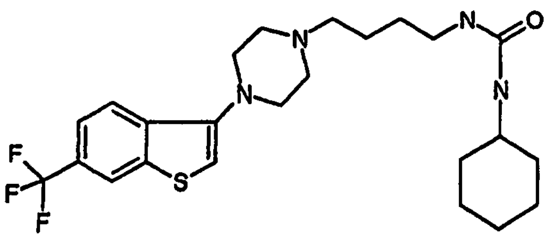 | 83.6 | |
| 814953 | 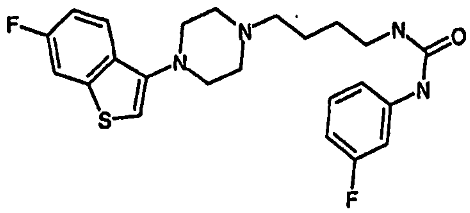 | 3.5 | |
| 816003 | 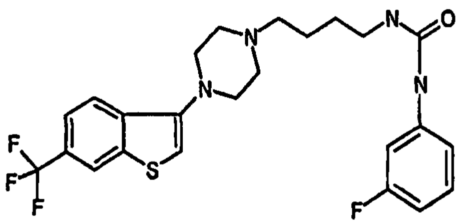 | 56.7 | |

Especially preferred compounds of the instant invention are those with a reduced liability for α-1 receptor

binding while at the same time having a higher affinity for dopamine $D_3$ than dopamine $D_2$ receptors.

Receptor affinity can be measured using standard methodology such as is described below.

### [N-Methyl-$^3$H]Spiroperidol Binding to Cloned Human Dopamine $D_3$ Receptors

**Purpose**

This assay measures the *in vitro* activity of compounds on cloned human dopamine ($D_3$) receptors and predicts the direct dopamine-blocking properties of putative neuropsychiatric agents at human dopamine $D_3$ receptors.

**Methods**

A. Cloning

The $D_3$ gene was isolated from a human striatal cDNA library (Stratagene). The gene was sequenced and subcloned into the expression vector RC/RSV (Invitrogen). CHO (Chinese Hamster Ovary) cells were stably transfected with 10 μg of the $D_3$/RSV plasmid using the DOTAP method from Boehringer Mannheim and 72 clones that were G418 resistant were isolated. Using mRNA and binding displacement data a single high expressing clone was identified. This clone was then grown in large batches for the purpose of developing a 96 well format assay.

B. Cell Culture

1. One plate (10 cm) with approximately 2-3 million $D_3$ cells per plate is incubated with 1 ml of Trypsin-EDTA at room temperature for ~2 min or until cells have lifted off plates. Four ml of Ham's F12 + 10% Fetal Bovine Serum + 1 % Penicillin/Streptomycin + G418 (400 μg/ml) medium are added to resuspend cells and 1 ml of this is added to each large plate (15 cm) containing 19 ml of the same medium as mentioned above.

2. The 5 large plates are incubated at 37˚C + 5% $CO_2$ for - 3 days or until the cells are confluent.

3. After these plates are confluent, they are split into 10 large plates. Medium is aspirated off, 2 ml of Trypsin-EDTA are added to each plate and plates are incubated at RT for 2 min or until cells have lifted off the plate. Eight ml of the F12 medium (same medium as #1 above) are added to each plate (10 ml total) to resuspend the cells and 5 ml are transferred to the 2 new plates containing 15 ml of the F12 media.

4. The 10 large plates are incubated at 37˚C + 5% $CO_2$ for - 2 days or until the cells are confluent.

5. The 10 large plates are split into 60 large plates (using Trypsin-EDTA as #3 except 4 ml of F12 medium are added to resuspend cells and 1 ml is aliquoted to 6 new plates containing 19 ml of F12 medium each).

6. Plates are incubated at 37˚C + 5% $CO_2$ for - 3 days or until cell are confluent.

7. The 60 large plates are then split into 60 roller bottles (100-150 million cells/bottle). Medium is aspirated off , 2 ml of Trypsin-EDTA are added to each plate and incubated at RT for -2 minutes or until cells have lifted off plates. Eight ml of F12 medium are added to each plate to resuspend cells and the entire 10 ml are added to 1 roller bottle containing 90 ml of the F12 medium.

8. The 60 roller bottles are immediately placed on their sides and transferred to the roller bottle incubator. They are incubated at 37˚C + 5% $CO_2$ for - 3-5 days. Cells are spun at 30-40% motor speed in the Forma incubator.

9. Medium is poured off and cells are washed 2X in PBS.

10. Cells are then scraped off in 20 ml of PBS and the bottles are rinsed again with 5 ml of PBS to remove any remaining cells. Cells are stored on ice before membrane prepration.

11. The yield for 60 $D_3$ roller bottles has varied from - 260-500 mg.

Note: All tissue culture reagents are from Gibco-BRL.

C. Membrane Preparation

The cells are harvested into 250 ml centrifuge tubes with 100 volumes of cold phosphate buffered saline (PBS) and spun down (1200xG for 10 min at 4˚C). The medium is removed and 100 ml PBS are added to each centrifuge tube, cells are resuspened and spun down again. The PBS is removed and the final pellet is homogenized in an appropriate volume of 10% DMSO with a polytron on ice at a medium setting.

D. Lowry Protein Assay

A 200 μl sample membrane homogenate is added to 200 μl of 1% SDS, vortexed and allowed to stand for 5 min. Aliquots (25, 50 and 100 μl) of this mixture are assayed in duplicate following the standard Bio-Rad DC protein assay protocol (kit catalog number 500-0112) and using reagent S. Absorbance readings are made at 750 nm (note: the most accurate protein OD readings are between 0.1-0.5 units). The protein concentration is calculated using a standard curve generated concurrently with bovine serum albumin as standard.

E. Storage/Freezing conditions

Following the determination of the protein concentration and Scatchard analysis, the protein is diluted into distilled water with 10% DMSO to the appropriate volume based on expression levels (Bmax). The concentrated protein is then aliquoted into 1.5 ml screw top cap Eppendorf tubes and placed into a -80˚C freezer.

F. Binding Assay Reagents

1. 0.5M Tris Buffer, pH 7.7

a) 44.4 g Tris HCl
26.5 g Tris Base
q.s. to 1 Liter (0.5 M Tris buffer, pH 7.7 at 37˚C)

b) make a 1:10 dilution in distilled $H_2O$ (0.05 M. Tris buffer, pH 7.7)

2. Tris Buffer containing physiological salts
a) Stock buffer

| | |
|---|---|
| NaCl | 7.014 g |
| KCl | 0.372 g |
| $CaCl_2$ | 0.222 g |
| $MgCl_2$ | 0.204 g |

q.s. To 100 ml with 0.5 M. Tris Buffer

b) Dilute 1:10 in distilled $H_2O$
This yields 0.05 M. Tris HCl, pH 7.7, containing NaCl (120 mM), KCl (5 mM), $CaCl_2$ (2 mM) and $MgCl_2$ (1 mM)
Optional: add 0.1 % ascorbic acid and check pH (in assays with compounds that may oxidize.
3. a) 1.0 % polyethyleneimine stock in 0.5M Tris (reagent 1.a)

b) Dilute 1:10 in distilled $H_2O$

4. [N-methyl-$^3$H]-Spiroperidol (60-90 Ci/mmol) is obtained from New England Nuclear; catalog #NET-856.
For $K_i$ determinations: [$^3$H]NMSP is made up to a concentration of 2.7 nM in buffer 2b, such that when 150 μl is added to each tube a final concentration of 0.4 nM is attained in the 1 ml assay. Samples of total CPM added are taken for each experiment to calculate the total ligand concentration.
5. S(-)-Eticlopride is obtained from Research Biochemicals International (RBI catalog number E-101). A refrigerated

stock (good for up to a month) solution of S(-)-eticlopride is made at a concentration of 30 μM in buffer 2b. One hundred microliters are added to 3 wells for the determination of nonspecific binding (this yields a final concentration of 3 μM in the 1 ml assay).

6. Test Compounds

For most assays, a 100 μM stock solution of the test compound is made up in a suitable solvent (usually <0.1% acetic acid) and serially diluted with buffer 2b, such that when 100 μl of drug is combined with the total 1 ml assay, final concentrations ranging from $10^{-5}$ - $10^{-8}$ M are attained. Characteristically eight concentrations are studied for each assay; however, higher or lower concentrations may be used, depending on the potency of the drug.

G. Binding Assay

750 μl Tissue
150 μl [$^3$H]NMSP
100 μl vehicle (for total binding) or 30 μM (-)eticlopride (for nonspecific binding) or appropriate drug concentration.

The 96-Well Packard Unifilters GF/B are incubated for >1 h at 25˚C in 0.1% polyethylamine (from 3,b). The cold tissue is added last and mixed on a orbital shaker for a few seconds and is then incubated at 37˚C for 30 min in a shaking water bath. The assay is stopped by rapid filtration through Packard Unifilter plates. The filter membranes are then washed with 15 ml of ice-cold 0.05 M Tris buffer. The filters are then dried (~15 min under a heat lamp or incubated for 15 min in a 60˚C oven) and a bottom seal is applied. Then 40 μl of Packard Microscint 20 scintillation cocktail is added and a permanent topseal (Type P) is applied and heat sealed. The plates are then shaken on an orbital shaker for 1 h and placed in the Packard Topcount and counted for at least 5 minutes for each point.

Specific binding is defined as the difference between total binding and the binding in the presence of 3 μM S-(-)-eticlopride. Total binding is approximately 10% of the total added ligand. Cheng -Prusoff determination ($K_i$'s) are performed using Prism software using a one-site competition curve analysis where the top and the bottom of the non-linear regression are held constant at 0% and 100% percent inhibition. The percent inhibition at each drug concentration is the mean of duplicate determinations.

**[N-Methyl-$^3$H]Spiroperidol Binding to Cloned Human Dopamine $D_2$Long Receptors**

Purpose:

This assay measures the *in vitro* activity of drugs on cloned human dopamine $D_2$Long ($D_2$L) receptors and predicts the direct dopamine-displacing properties of neuropsychiatric, cardiovascular and renal agents at human dopamine $D_2$ receptors.

Methods:

A. Cloning

The $D_2$L gene was isolated from a human striatal (caudate/putamen) cDNA library. The gene was sequenced and sub-cloned into the expression vector pRC/RSV (Invitrogen). CHO (Chinese Hamster Ovary) cells were stably transfected and 72 clones that were geneticin (G418) resistant were isolated. Using mRNA and binding data a single high expressing cell line was identified (#44). This cell line was then grown in suspension culture for the purpose of developing a 96 well format assay.

B. Cell Culture Conditions

1. Medium for adherent CHO cultures:

**Ham's F12 + 10% fetal bovine serum (FBS) + 400 μg/ml geneticin (G418) + 10 ml/L penicillin-streptomycin (pen-strep)**

2. Cells are transferred to suspension culture when at least 1.5 million cells are available (this allows for 300,000 cells/ml in a 50 ml spinner flask; this is the ideal suspension density). Cell are removed from flasks with trypsin, spun down (1000×G) and resuspended in fresh medium:

**50% CHO-SFM II + 50% Ham's F12 w/ 10% FBS (final FBS conc. 5%)**

**+ 400 μg/ml G418 + pen-strep (10 ml/L)**

3. After the transfer to suspension culture, growth is monitored and cell viability is assessed using trypan blue exclusion. Total and viable cell count on 5 sectors of the hemocytometer are recorded. When the viable cell density reaches 600,000 cell/ml, the volume is doubled.

4. After 1 week of growth in the 50/50 medium, the cells are spun down and transferred to a new spinner flask and replaced with 75% CHO-SFM II / 25% Ham's F12 + 10% FBS plus the pen-strep and G418. Thereafter every 3 days, the medium is replaced with new medium containing a decreasing amount of FBS as follows:

| ml of CHO SFM: ml of Ham'S F12 | Final % FBS conc. |
|---|---|
| 87.50 : 12.5 | 1.25 |
| 93.75 : 6.25 | 0.625 |
| 99.00 : 1.00 | 0.1 |

5. The final maintenance culturing medium is made up as follows:

A stock mixture of 10 ml of pen-strep, 0.5 ml of 400 mg/ml (active; final concentration: 200 mg/ml) G418 and 1ml of FBS are mixed and filtered and refrigerated. A volume (11.5 ml) of this mixture is added to a freshly opened 1 L bottle of CHO-SFM II.

C. Membrane Preparation

The cells are harvested into 250 ml centrifuge tubes with 100 volumes of cold phosphate buffered saline (PBS) and spun down (1200xG for 10 min at 4°C). The medium is removed and 100 ml PBS are added to each centrifuge tube, cells are resuspened and spun down again. The PBS is removed and the final pellet is homogenized in an appropriate volume of PBS with a polytron on ice at a medium setting.

D. Lowry Protein Assay

A 200 μl sample membrane homogenate is added to 200 μl of 1% SDS, vortexed and allowed to stand for 5 min. Aliquots (25, 50 and 100 μl) of this mixture are assayed in duplicate following the standard Bio-Rad *DC* protein assay protocol (kit catalog number 500-0112) and using reagent S. Absorbance readings are made at 750 nm (note: the most accurate protein OD readings are between 0.1-0.5 units). The protein concentration is calculated using a standard curve generated concurrently with bovine serum albumin as standard.

E. Storage/Freezing conditions

Following the determination of the protein concentration, the protein is diluted into distilled water with 10% DMSO to the appropriate volume based on expression levels (Bmax). The concentrated protein is aliquoted into 1.5 ml screw top eppendorf tubes and placed into a -80°C freezer.

F. Binding Assay Reagents

1. 0.5M Tris Buffer, pH 7.7

a) 44.4 g Tris HCl
26.5 g Tris Base
q.s. to 1 Liter (0.5 M Tris buffer, pH 7.7 at 37°C)

b) make a 1:10 dilution in distilled $H_2O$ (0.05 M. Tris buffer, pH 7.7)

2. Tris Buffer containing physiological salts
a) Stock buffer

| | |
|---|---|
| NaCl | 7.014 g |
| KCl | 0.372 g |
| $CaCl_2$ | 0.222 g |
| $MgCl_2$ | 0.204 g |
| q.s. To 100 ml with 0.5 M. Tris Buffer | |

b) Dilute 1:10 in distilled $H_20$
This yields 0.05 M. Tris HCl, pH 7.7, containing NaCl (120 mM), KCl (5 mM), $CaCl_2$ (2 mM) and $MgCl_2$ (1 mM)
Optional: add 0.1 % ascorbic acid and check pH (in assays with compounds that may oxidize.
3. a) 1.0 % polyethyleneimine stock in 0.5M Tris (reagent 1.a)

b) Dilute 1:10 in distilled $H_2O$

4. [N-methyl-$^3$H]-Spiroperidol (60-90 Ci/mmol) is obtained from New England Nuclear; catalog #NET-856.
For $K_i$ determinations: [$^3$H]NMSP is made up to a concentration of 2.7 nM in buffer 2b, such that when 150 μl is added to each tube a final concentration of 0.4 nM is attained in the 1 ml assay. Samples of total CPM added are taken for each experiment to calculate the total ligand concentration.
5. S(-)-Eticlopride is obtained from Research Biochemicals International (RBI catalog number E-101). A refrigerated stock (good for up to a month) solution of S(-)-eticlopride is made at a concentration of 30 μM in buffer 2b. One hundred microliters are added to 3 wells for the determination of nonspecific binding (this yields a final concentration of 3 μM in the 1 ml assay).
6. Test Compounds
For most assays, a 100 μM stock solution of the test compound is made up in a suitable solvent (usually <0.1 % acetic acid) and serially diluted with buffer 2b, such that when 100 μl of drug is combined with the total 1 ml assay, final concentrations ranging from $10^{-5}$ -$10^{-8}$ M are attained. Characteristically eight concentrations are studied for each assay; however, higher or lower concentrations may be used, depending on the potency of the drug.

G. Binding Assay

750 μl Tissue
150 μl [$^3$H]NMSP
100 μl vehicle (for total binding) or 30 μM (-)eticlopride (for nonspecific binding) or appropriate drug concentration.

The 96-Well Packard Unifilters GF/B are incubated for >1 h at 25˚C in 0.1 % polyethylamine (from 3,b). The cold tissue is added last and mixed on a orbital shaker for a few seconds and is then incubated at 37˚C for 30 min in a shaking water bath. The assay is stopped by rapid filtration through Packard Unifilter plates. The filter membranes are then washed with 15 ml of ice-cold 0.05 M Tris buffer. The filters are then dried (~15 min under a heat lamp or incubated for 15 min in a 60˚C oven) and a bottom seal is applied. Then 40 μl of Packard Microscint 20 scintillation cocktail is added and a permanent topseal (Type P) is applied and heat sealed. The plates are then shaken on an orbital shaker for 1 h and placed in the Packard Topcount and counted for at least 5 minutes for each point.
Specific binding is defined as the difference between total binding and the binding in the presence of 3 μM S-(-)-eticlopride. Total binding is approximately 10% of the total added ligand. Cheng -Prusoff determination ($K_i$'s) are performed using Prism software using a one-site competition curve analysis where the top and the bottom of the non-linear regression are held constant at 0% and 100% percent inhibition. The percent inhibition at each drug concentration is the mean of duplicate determinations.

## [$^3$H]PRAZOSIN BINDING TO CLONED HUMAN ALPHA-1A ADRENERGIC RECEPTORS ($\alpha_{1a}$) EXPRESSED IN CHINESE HAMSTER OVARY CELLS (CHO)

**Purpose:** This *in vitro* assay is designed as a screen to identify compounds displaying a affinity for the human $\alpha_{1a}$ adrenoceptor subtype expressed in the membrane fragments of CHO cells. The assay measures the ability of the test compounds to displace [$^3$H] prazosin from $\alpha_{1a}$ sites.

The identification of multiple vascular $\alpha_1$-addrenoceptors ($\alpha_{1a}$, $\alpha_{1b}$, $\alpha_{1d}$) *in vitro* has provided impetus to define the role (s) of these subtypes in cardiovascular regulation *in vivo* (Vargas and Gorman, 1995). Hemodynamic studies in the unanesthetized rat suggest that vascular $\alpha_{1a}$ receptors are the major subtype involved in the sympathetic regulation of peripheral resistance and systemic arterial pressure (Piascik et al., 1989). Additional evidence for an involvement of peripheral $\alpha_{1a}$ receptors in the maintenance of arterial pressure was demonstrated by the findings that the selective $\alpha_{1a}$ antagonist 5-MU dose dependently lowered resting arterial pressure in awake conscious dogs (Piascik et al., 1989). A demonstrated inability of the irreversible antagonist, chloroethylclonidine, to reduce arterial pressure in rats when administered intravenously, is strong evidence against the role of $\alpha_{1b}$ and $\alpha_{1d}$ receptors in the acute regulation of arterial pressure (Vargas et al., 1993).

Therefore, the binding of compounds to $\alpha_{1a}$ adrenergic receptors is believed to be a good measure of a compound's potential to cause orthostatic hypotension and sedation as side effects. Prazosin is a potent antagonist of the human $\alpha_{1a}$-adrenoceptor subtype, which has been cloned and is expressed in the membrane fragments of CHO cells.

**h$\alpha_{1a}$ receptor:** The cloning of the human $\alpha_{1a}$ cDNA was accomplished first by screening a human prostate cDNA library (Clontech), from which a portion of the coding region was obtained. This DNA fragment was then used to screen a human leukocyte genomic library (Clontech), and the rest of the coding sequence was obtained. Later these two fragments were spliced together. The entire coding sequence was then fully sequenced including matching PCR sequence with original genomic coding sequence, thus ensuring splice sites were joined correctly (Schwinn et al., 1995). Once sequenced, the gene was subcloned into the expression vector pcDNA3 (Invitrogen). Plasmid DNA was then used for transfection into CHO cells and G418 resistant clones were isolated. A clone expressing high levels of the h$\alpha_{1a}$ receptor (as determined by mRNA and receptor binding data) was chosen and pharmacologically characterized.

**Culture Media:** Media Ingredients for Adherent $\alpha_{1a}$ expressing CHO Culture:

A. HAM's F-12 (Cellgro)
B. 10% 0.2 micron filtered Fetal Bovine Serum (FBS)(Cellgro)
C. 1% 0.2 micron filtered Penicillin-Streptomycin (Cellgro)
D. G418 0.2 micron filtered (Geneticin 400$\mu$g/ml)(Cellgro)

Cells are cultured using established methods and procedures in either 150x25mm culture plates (scale up to 100 plates) or a combination of these plates and 70 roller bottles. One culturing/harvest cycle typically requires 2 weeks and yields between 100-400mg protein. Plates or bottles are incubated at 37°C + 5% $CO_2$.

**Storage:** Cells are harvested by mechanical scraping, washed using PBS, collected in 250ml Corning polypropylene centrifuge tubes, spun down (1500RPM) and resuspended in d$H_2O$ 10% DMSO (final volume per harvest is approximately 50ml). Protein determination is made using the Biorad DC Assay Kit. Finally, the appropriate volume is aliquoted into a 2ml Corning Cryovial (10mg/1-1.5 ml) which is stored at -80°C.

**Current Lot Data:** $\alpha_{1a}$ (clone # 7)

| | |
|---|---|
| Batch 1/14/98 | |
| Receptor Concentration | 2418 fmoles/mg protein |
| $K_d$ | 0.18nM |
| Volume | 1.5 ml/cryovial |
| Protein Concentration | approx. 10mg/1.5ml |

**Assay Requirement:** 0.5 cryovials per 96 well plate (assay volume = 200$\mu$l/well)

**[$^3$H]-Ligand:** [7-methoxy-$^3$H]-Prazosin: 1.0nM (NEN, NET-823)
70-87 Ci/mmol

**Materials:** Phentolamine mesylate (Research Biochemicals Int. #P-131)
96 well flat bottom plates (Beckman)
Unifilter GF/B Plate (Packard)
Polyethylenimine (Sigma #P-3134)
TomTec or Packard Filtermate 196 Cell Harvesters
Packard TopCount Scintillation Counter

**Buffers:** *A:* 50 mM Tris HCl; 0.1% ascorbate, pH 7.7 (incubation buffer)
***B:*** 50 mM Tris HCl; pH 7.7 (wash buffer)

**Procedure**: Assay additions are as follows (in the order indicated):

Total Binding = 50μl buffer A + 50μl [$^3$H]prazosin + 100μl membrane Nonsp. Bd. = 50μl 10μM phentolamine+50 μl [$^3$H]prazosin+100μl membrane

Test Cpd. = 50μl compound + 50μl [$^3$H]prazosin + 100μl membrane

Compounds to be evaluated are weighed out to yield a 10 mM stock solution in DMSO in a 24 well polystyrene plate. This is diluted to a 0.5 mM stock in $dH_2O$. Serial dilutions in Buffer A are made from which 50μ l additions to the plate are made in duplicate in order to achieve the final concentrations desired. Typically, one 96 well plate is used to evaluate 11 compounds at 4 concentrations ($10^{-6}$-$10^{-9}$ M) in duplicate. Total binding and nonspecific binding are determined in quadruplicate. Usually one standard is run with each assay.

**[$^3$H]Prazosin** is made up in Buffer A such that when 50 μl are added per well the final concentration is 1.0 nM in a final assay volume of 200 μl. The final concentration should be verified by running a sample in a scintillation counter prior to adding the [$^3$H]prazosin to the 96 well plate. Note: The radioactivity should be prepared just before the additions are made so that it is not allowed to sit on the bench for very long.

**Packard GF/B Plate Pretreatment:** The filter plates are presoaked for at least 30 min in ice cold Buffer B containing 0.05% polyethyleneimine (200 μl/200 ml) to maximize filtration efficiency and minimize filter blank binding.

**Incubation & Filtration:** Once buffer, compounds, [$^3$H]prazosin and membrane have been added (and mixed), the 96 well plates are incubated for 40 min at 37˚C and spaced 3-5 min apart. At 40 min, the plates are filtered using a Tomtec Automated Cell Harvester. Filtration is immediately followed by washes of ice cold Buffer B (total vol. ~7 ml).

**Drying and Counting:** Each filter plate is dried under a heat lamp for 15 min. The back of the plate is sealed and 40 μl of Packard microscint fluid are added per well. Using Packard film, each plate is heat sealed prior to being counted in a Packard Topcount Scintillation counter. A program has been written that counts each plate twice sending DPM, CPM and TSIS data to disk and printer.

**Analysis of Results:** Raw DPM and CPM data are captured on disk and are imported into one of several software packages (Graphpad Prism Ver 2.0, Excel) residing on the LAN. Specific binding is defined as the difference between total binding and the binding in the presence of 10 μM phentolamine. Total binding is less than 10% of the total added ligand. Software using one-site competition curve analysis is employed in the calculation of $IC_{50}$ and $K_I$ (Cheng-Prusoff equation, 1973). The top and bottom of the non-linear regression are held constant at 0% and 100% inhibition. The percent inhibition at each drug concentration is the mean of duplicate determinations.

**References:** Vargas, H.M and A.J. Gorman. *Life Sciences.* Vol. 57, No. 25, pp. 2291-2308, 1995.

Morrow, A.L. and I. Creese. *Mol. Pharmacol.* 29: 321-330, 1986.

Piascik, M.T., J.W. Kusiak, and K.W. Barron. *Eur. J. Pharmacol.* 11:101-107, 1989.

Vargas, H.M., D. Cunningham, L. Zhou, H.B. Hartman and A.J. Gorman. *J. Pharmacol. Exp. Ther.* 267:264-272, 1993.

The functional activity of compounds of the invention (i.e. whether they are antagonists, agonists or partial agonists) can readily be determined using the microphysiometer test method that follows:

Chinese Hamster Ovary (CHO) cells, expressing the human dopamine D3 receptor, were grown on the surface of a capsule cup. Cups are assembled and placed on the microphysiometer, and buffer (Dulbecco's Modified Eagle's Medium without sodium bicarbonate and without serum) is perfused through the cup assembly until a stable baseline is achieved (4 hours). Buffer perfusion rate and solution changes were controlled by a computer. Intracellular acidification rate was measured in each of the 8 cup assemblies and recorded by the computer. Buffer containing test compound (10 nM, 100 nM, and 1 uM) was perfused through the cup assembly for 20 min, then buffer containing quinpirole (10 nM) and test compound (same concentrations) was perfused for an additional 1 min. This was followed by a recovery period of 10-60 min where buffer alone was perfused through the cups. Quinpirole increased acidification rate. A $D_3$ antagonist will inhibit this increase in a concentration dependent manner.

$D_3$ antagonists are of potential use as antipsychotic agents for example in the treatment of schizophrenia, schizo-affective disorders, psychotic depression and mania. Conditions which may be treated by $D_3$ agonists include include dyskinetic disorders such as Parkinson's disease, neuroleptic-induced parkinsonism and tardive dyskinesias; depression; anxiety; dementia; circadian rhythm disorders, and drug (e.g. cocaine) dependency.

As employed herein, the phrase "modulating the activity of dopamine $D_3$ receptors" refers to a variety of therapeutic applications. Said therapeutic applications refer to the treatment of conditions or disorders which include dyskinetic disorders, psychoses, anxiety disorders, mood disorders, dementia, sleep disorders, substance dependence, substance abuse and nausea. The compounds are also useful for treating renal dysfunction.

The instant invention also provides a method of treating renal dysfunction comprising administering to a patient in need thereof a therapeutically effective amount of a compound of formula I or a pharmaceutically acceptable salt thereof.

The instant invention further provides a method of treating conditions or disorders of the central nervous system comprising administering to a patient in need thereof a therapeutically effective amount of a compound of formula I, or a pharmaceutically acceptable salt thereof, in conjunction with one or more $D_1$, $D_2$, $D_4$, $D_5$ or 5HT receptor antagonists.

In treating a patient afflicted with a condition or disorder described above, a compound of formula I can be administered in any form or mode which makes the compound bioavailable in therapeutically effective amounts, including orally, sublingually, buccally, subcutaneously, intramuscularly, intravenously, transdermally, intranasally, rectally, topically, and the like. One skilled in the art of preparing formulations can determine the proper form and mode of administration depending upon the particular characteristics of the compound selected for the condition or disease to be treated, the stage of the disease, the condition of the patient and other relevant circumstances. For example, see Remington's Pharmaceutical Sciences, 18th Edition, Mack Publishing Co. (1990), incorporated herein by reference.

The compounds of Formula I can be administered alone or in the form of a pharmaceutical composition in combination with pharmaceutically acceptable carriers, the proportion and nature of which are determined by the solubility and chemical properties of the compound selected, the chosen route of administration, standard pharmaceutical practice and other relevant criteria.

The compounds of formula I may be administered orally, for example, in the form of tablets, troches, capsules, elixirs, suspensions, solutions, syrups, wafers, chewing gums, sprinkles, and the like and may contain one or more of the following adjuvants: binders such as microcrystalline cellulose, gum tragacanth or gelatin; excipients such as starch or lactose, disintegrating agents such as alginic acid, Primogel, corn starch and the like; lubricants such as magnesium stearate or Sterotex; glidants such as colloidal silicon dioxide; and sweetening agents such as sucrose or saccharin may be added or a flavoring agent such as peppermint, methyl salicylate or orange flavoring. When the dosage unit form is a capsule, it may contain, in addition to materials of the above type, a liquid carrier such as polyethylene glycol or a fatty oil. Other dosage unit forms may contain other various materials which modify the physical form of the dosage unit, for example, as coatings. Thus, tablets or pills may be coated with sugar, shellac, or other enteric coating agents. A syrup may contain, in addition to the present compounds, sucrose as a sweetening agent and certain preservatives, dyes and colorings and flavors.

The compounds of Formula I may also be administered topically, and when done so the carrier may suitably comprise a solution, ointment or gel base. The base, for example, may comprise one or more of petrolatum, lanolin, polyethylene glycols, bee wax, mineral oil, diluents such as water and alcohol, and emulsifiers and stabilizers.

The solutions or suspensions may also include one or more of the following adjuvants: sterile diluents such as water for injection, saline solution, fixed oils, polyethylene glycols, glycerine, propylene glycol or other synthetic solvents; antibacterial agents such as benzyl alcohol or methyl paraben; antioxidants such as ascorbic acid or sodium bisulfite; chelating agents such as ethylene diaminetetraacetic acid; buffers such as acetates, citrates or phosphates and agents for the adjustment of tonicity such as sodium chloride or dextrose. The parenteral preparation can be enclosed in ampoules, disposable syringes or multiple dose vials.

The highly lipophilic esters, amides and carbamates of compounds I are capable of sustained release in mammals for a period of several days or from about one to four weeks when formulated and administered as depot preparations, as for example, when injected in a properly selected pharmaceutically acceptable oil. The preferred oils are of vegetable origin such as sesame oil, cottonseed oil, corn oil, coconut oil, soybean oil, olive oil and the like, or they are synthetic esters of fatty acids and polyfunctional alcohols such as glycerol or propyleneglycol.

The depot compositions of formula I are prepared by dissolving a highly lipophilic ester, amide or carbamate of the instant invention in a pharmaceutically acceptable oil under sterile conditions. The oil is selected so as to obtain a release of the active ingredient over a desired period of time. The appropriate oil may easily be determined by consulting the prior art, or without undue experimentation by one skilled in the art.

The dosage range at which the compounds of formula I exhibit their ability to act therapeutically can vary depending upon the particular disease or condition being treated and its severity, the patient, the formulation, other underlying disease states that the patient is suffering from, and other medications that may be concurrently administered to the patient. Generally, the compounds of formula I will exhibit their therapeutic activities at dosages of between about 0.001 mg/kg of patient body weight/day to about 100 mg/kg of patient body weight/day.

In a further aspect, the present invention provides novel radiolabeled imaging agents of formula I, useful, inter alia, for imaging dopamine $D_3$ receptors in the CNS to diagnose CNS abnormalities.

The radiolabeled (tritiated and C-14 labeled) forms compounds of formula I are useful as radioligands to determine the binding of compounds to the dopamine $D_3$ receptor. They are also useful as labeled parent compounds to determine the metabolism of the compound in animals. Preferred for this purpose are compounds of formula I wherein R is group (a) with a radiolabeled $^{14}C$ in the 3-position of the benzo[b]thiophene ring system, L is trifluoromethyl, p is 1, $R_3$ is hydrogen, n is 1, i is 0, and A is N. Particularly preferred for this purpose are compounds of formula IA. As employed herein, a "compound of formula IA" shall refer to the compound of formula I wherein R is group (a) wherein L is trifluoromethyl substituted in the 6-position of the benzthiophene ring system and a radiolabeled $^{14}C$ is in the 3-position of the benzo[b]thiophene ring system, p is 1; A is N, n is 1; i is 0, and $R_3$ is hydrogen. Compounds of formula IA may be prepared in a manner analogous to that set forth in Example 21.

Imbalances in dopamine production have been implicated in a variety of mental and physical disorders, such as Parkinson's disease (PD). It is thus desirable to diagnose and monitor such imbalances and to monitor the effectiveness of drugs and substances that affect brain chemistry. New and powerful imaging methods that enable one to assess the living brain in vivo and thereby monitor brain chemistry and the effectiveness of drugs and substances that affect brain chemistry have been developed. Methods such as positron emission tomography (PET) and single photon emission

computed tomography (SPECT) involve administering to a patient a radioactive tracer substance comprising a ligand that binds to the presynaptic or postsynaptic neuroreceptors in the patient's brain. Emissions (primarily gamma rays are emitted from the positrons or photons from the radioactive tracer) are measured. These emissions are indicative of the number and degree of occupancy of blocking of the neuroreceptors. The number of neuroreceptors and the degree of occupancy or blocking is calculated utilizing a mathematical model, and compared with an intra-person or inter-person control to determine the degree of drug response. Further treatment of the patient with drugs is based on the comparisons made. For these methods to be useful, however, a ligand that has a high specificity and affinity for the desired receptor is required.

It is believed that certain radioactive ligands may be selective for dopamine transporters and are thus potentially useful in evaluating changes in dopamine function in vivo and in vitro, especially for patients with Parkinson's disease (PD), which is characterized by a selective loss of dopamine neurons in the basal ganglia and substantia nigra.

Another aspect of this invention relates to methods for utilizing the compounds of the invention as CNS imaging agents. Imaging techniques are non-invasive diagnostic techniques that generally involve administering a compound with marker atoms that can be detected externally to the mammal. Generally, these methods comprise administering to a mammal a compound of the invention, dissolved or dispersed in a suitable pharmaceutical carrier or diluent. The compound of the invention selectively binds to dopamine $D_3$, thus permitting the imaging of CNS receptors and the ability to, inter alia, evaluate brain chemistry, the effectiveness of drugs, and neuronal functions. Imaging techniques suitable for practicing the present invention include, but are not limited to, single photon emission computed tomography (SPECT) and positron emission tomography (PET).

Radionuclides that are widely used in diagnostic nuclear medicine include technetium [$^{99}$Tc], iodine [$^{123}$I], carbon [$^{11}$C], and fluorine [$^{18}$F]. The radiolabeled imaging agent specifically exemplified herein contains the radionuclide $^{11}$C. It should be noted that the same or similar radiochemistry reactions can be carried out using radionuclides other than $^{11}$C.

The invention is further illustrated by the following non-limiting examples and tabulated information. These examples are understood to be illustrative only and are not intended to limit the scope of the present invention in any way. As used herein, the following terms have the indicated meanings: "g" refers to grams; "mmol" refers to millimoles; "ml" refers to milliliters; "°C" refers to degrees Celsius; "TLC" refers to thin layer chromatography; "LC/MS" refers to liquid chromatography mass spectrometry; "APCI" refers to atmospheric pressure chemical ionization; "mp" refers to melting point.

## EXAMPLES

Example 1

### Synthesis of Intermediate Substituted Piperazines

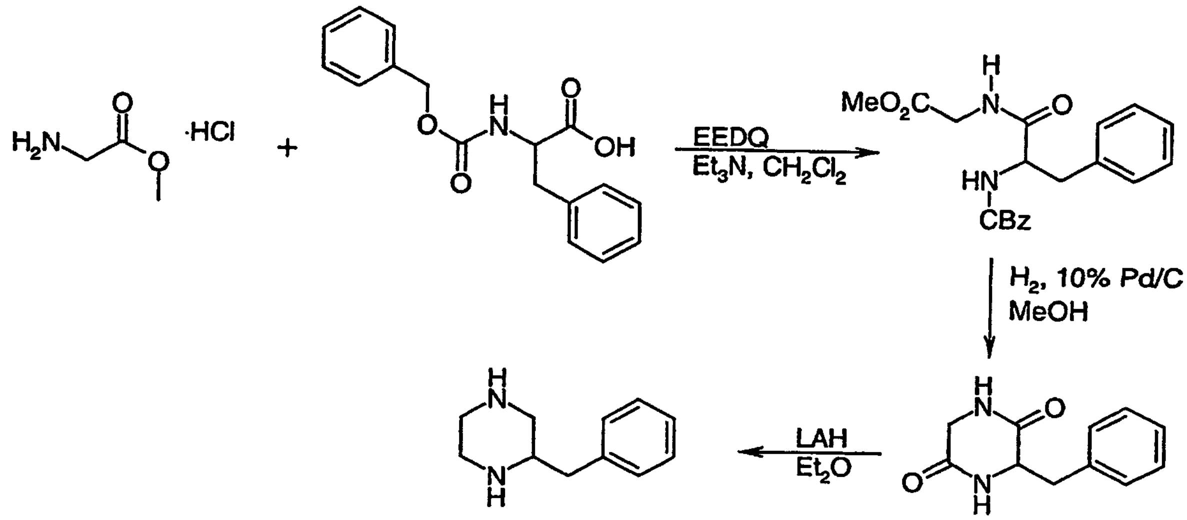

Example 1(a): Preparation of intermediate 3-benzyl-piperazine

To a suspension of 3-benzyl-piperazine-2,5-dione (14.98 g, 73 mmol, prepared following generally the procedure of Halpem and Westley, J. Org. Chem. 1968, 33, 864) in dry diethyl ether (500 mL) is added dropwise to a solution of lithium aluminum hydride (400 mL of a 1 M solution in diethyl ether, 400 mmol, 5.4 eq). The suspension is heated at reflux for 23 hours and then cooled to 0˚C. Water (70 mL) is then cautiously added and the resulting suspension is warmed to room temperature. After 3 hours the suspension is filtered and the solid washed with diethyl ether (1 L). The filtrate is concentrated under vacuum to provide crude title compound (11.40 g, 88%) as a yellow, crystalline solid. A sample (2 g) is recrystallized from cyclohexane and then from toluene to provide the purified title compound (0.83 g) as a fine, white crystals: mp 80-81 ˚C.

| | |
|---|---|
| Anal. Calcd. For $C_{11}H_{16}N_2$: | C, 74.96; H, 9.15; N, 15.89; |
| Found: | C, 74.84; H, 9.01; N, 16.15. |

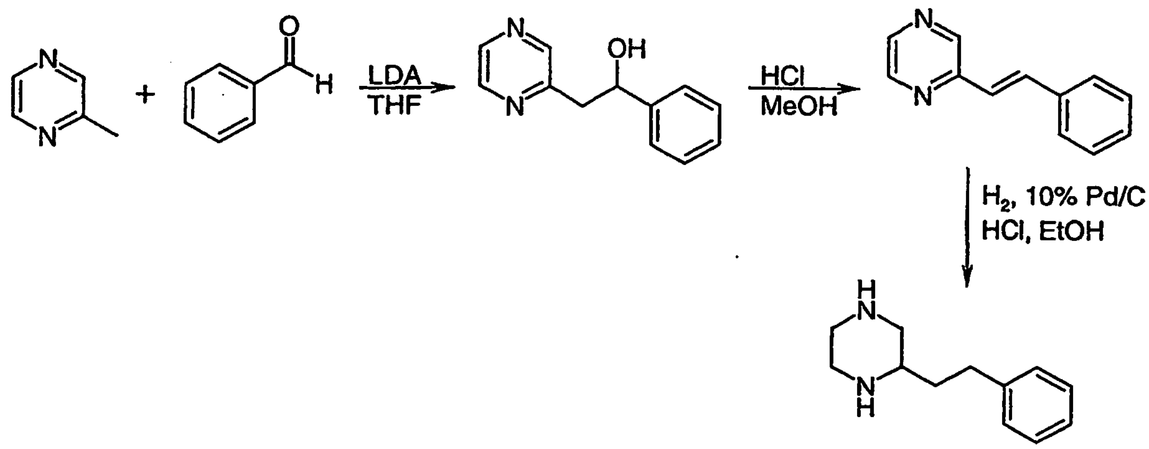


Example 1(b): To a solution of LDA (295 mL, 0.59 mol, 2 M in heptane/ THF/ ethylbenzene) in anhydrous THF (300 mL) cooled to -40 ˚C was added 2-methylpyrazine (48.5 mL, 0.531 mol) dropwise via an addition funnel. The reaction was allowed to warm to -20˚C and was stirred for 90 minutes when a solution of benzaldehyde (54 mL, 0.531 mol) in anhydrous THF (200 mL) was added dropwise via an addition funnel. After complete addition, the reaction was allowed to warm to room temperature and was stirred for 20 hours. The reaction was then cooled in an ice bath and saturated $NH_4Cl$ (500 mL) was added. The resulting mixture was extracted with EtOAc (500 mL, 250 mL). The combined extracts were dried ($Na_2SO_4$), filtered and concentrated to a damp, beige solid. The product was triturated with $Et_2O$ and collected then dried overnight to yield 56.0 g (53%) of a light brown solid, mp 81-84 ˚C.

A solution of the above-obtained solid (56.0 g, 0.28 mol) in MeOH (1.1 L) and conc. HCl (290 mL) was stirred at reflux for 24 hours. The reaction was cooled to room temperature and concentrated to a dark liquid. The dark liquid was cooled in an ice bath and water (1 L) was added. The resulting solution was neutralized with a saturated solution of $Na_2CO_3$ and the product was extracted with EtOAc (1 L, 2 x 500 mL). The combined extracts were dried ($Na_2SO_4$), filtered and concentrated to yield 46 g of a dark brown solid. The solid was purified via flash column chromatography (40% EtOAc in heptane) yielding 22.7 g of the olefin as a brown foam.

A 1 L Parr shaker bottle was flushed with nitrogen and charged with 10% Pd/C (4.5 g, Degussa type) and the above-obtained olefin (20.0 g, 0.110 mol) in EtOH (450 mL). The reaction was hydrogenated at 50 psi for 3.5 hours when the reaction was filtered through a celite plug and rinsed with ethanol. The bottle was recharged with fresh 10% Pd/C (4.5 g, Degussa type), the filtrate and conc. HCl (15 mL). The reaction was hydrogenated at 50 psi for 18 hours when the reaction was diluted with warm MeOH and filtered through a plug of celite. The solid was thoroughly washed with hot MeOH and the filtrate was concentrated to yield 11.2 g (39%) of the final product as the di-HCl salt, mp 297-300.

See: Tetrahedron, 30, 1974 pp667-673 and Tet. Lett. 1979, pp4483-4486

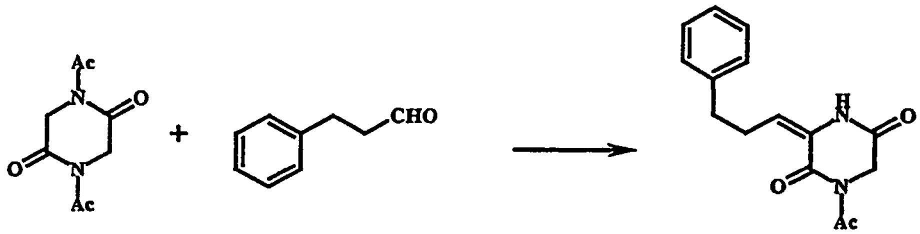

Example 1(c): DBU (14.0 g, 92 mmol) was added to a solution of the piperazine diacetate (18.2 g, 92 mmol) and aldehyde (12.3 g, 92 mmol) in 92 mL of DMF at ambient temperature. The resulting mixture was stirred at room temperature for 5h. The precipitated product was collected by filtration, providing 17.1 g of product.

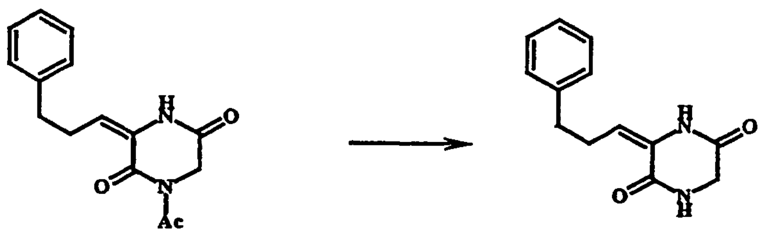

The monoacetate (17.0 g, 62.8 mmol) and hydrazine hydrate (9.4 g, 188.6 mmol) in 125 mL of DMF were stirred at room temperature for 20 h. The precipitated solid was collected by filtration, and washed with water and ethanol, leaving 13.7 g of product.

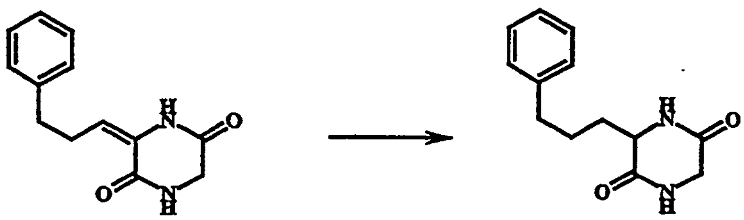

The olefin (13.6 g, 59.1 mmol) and palladium on carbon (2.7 g, 10% Pd/C, Degussa type, 50% $H_2O$) in 1.2 l of methanol were shaken on a Parr hydrogenation apparatus at 40 psi of hydrogen, until hydrogen uptake ceased. The mixture was diluted with dichloromethane and filtered through celite. Concentration of the filtrate provided 12.1 g of product.

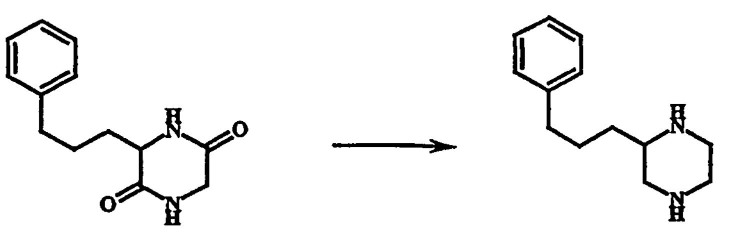

A solution of LAH (156 mL, 156 mmol, 1M in THF) was added dropwise to a 0°C solution of the piperazine dione (12.1 g, 52.1 mmol) in 100 ml of THF. The mixture was heated to reflux and stirred overnight. The mixture was

cooled to 0˚C and 38 mL of water in 200 mL of THF was carefully added. The resulting mixture stirred for 1 h, then it was filtered, the filter cake was washed with THF, and the filtrate was concentrated in vacuo to.give 7.4 g of product.

Example 2

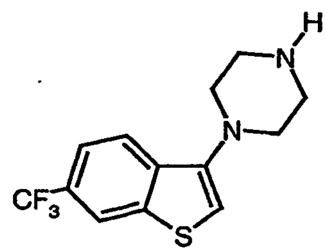

**1-(6-(trifluoromethyl)-benzo[b]thien-3-yl)-piperazine hydrochloride**

2a: 2-Carbomethoxy-3-amino-6-trifluoromethylbenzo[b]thiophene:

Equip a 22-L, 3-necked, round-bottom flask with a mechanical stirrer, nitrogen bubbler, and a thermocouple probe, charge with 1.20 kg (5.55 mole) of 2-nitro-4-trifluoromethylbenzonitrile, 589.3 g (496 mL, 5.55 mole) of methyl thioglycolate, and 4.3 L of NMP. Cool the resulting yellow solution to 2 ˚C, and add slowly, over a period of 78 min a solution prepared from 466.0 g (11.11 mole, 2.0 eq) of lithium hydroxide monohydrate in 3.36 L of water while maintaining the temperature between 2 - 20 ˚C. Allow the brown slurry to warm to 21˚C over a 2 h period, and then dilute with 8.0 L of water (observe exotherm-> 27 ˚C). Stir for 40 min and cool to 18 ˚C, collect the product by filtration, rinsing with 10 L of water, then air-drying at ambient temperature to give 1.295 kg (84.7% yield) of 2-carbomethoxy-3-amino-6-trifluoromethylbenzo[b]thiophene, as a light-yellow solid, 99.8% pure by HPLC assay.

2b: 1-(6-(trifluoromethyl)-benzo[b]thien-3-yl)-piperazine hydrochloride

Equip a 12-L, 3-necked, round-bottom flask with a mechanical stirrer, nitrogen bubbler, and a thermocouple probe, and charge with 1.14 kg (4.14 mole) of 2-carbomethoxy-3-amino-6-trifluoromethylbenzo-[b]thiophene (Example 2a), 196.0 g (2.28 mole, 0.55 eq) of piperazine, 4.0 L of NMP, and 570 mL of xylene. Heat the solution, and hold at 170 - 180 ˚C for 4 h, at which time the reaction is ca. 98% complete as determined by HPLC assay. Cool the brown solution to 168 ˚C, and then add 1.605 kg (18.63 mole, 4.5 eq) of piperazine (temp -> 109 ˚C) following with 1.575 kg (28.28 mole, 2.0 eq) of p-toluenesulfonic acid monohydrate (observe exotherm, 109 -> 130 ˚C). Connect a Dean-Stark trap to the condenser, and heat the reaction to collect an azeotrope. Remove a total of 410 mL of an aqueous distillate, while allowing the pot temperature to increase from 145 to 165 ˚C. Monitor the progress of the reaction by GC/MS and HPLC assays. After 14 h at ca. 165 ˚C (>99% conversion by HPLC and GC/MS assay), cool the reaction to 30-35 ˚C, and then quench into an extractor that contains 5 kg of ice, 12 L of water, and 8.5 L of toluene. Separate the phases, wash the organic extract with 11 L of 0.5 N NaOH, 2 L of saturated aq. NaCl., and then extract with 8 L of 1 N HCl. Dilute the acidic aqueous extract with 1 kg of ice, and basify to pH 11.2 by adding 624 g of 50% NaOH. Extract the resulting mixture with 9.5 L of toluene. Wash the toluene extract with 2 L of saturated aqueous NaCl, dry ($Na_2SO_4$), and filter. Charge the filtrate into a 22 L 3-necked, round-bottomed flask ($N_2$, mechanical stirring, temperature control probe), and add a total of 3.7 L of 1 N ethereal HCl at 20 - 27 ˚C so that the mixture is positive to Congo Red indicator paper. During the HCl addition, add a total of 2.5 L of toluene to improve the stirring of the thick slurry that results. Stir at ambient temperature for 40 min, filter the slurry and wash with 4.5 L of toluene. After air drying, obtain 1.165 kg (87% yield) of 3-piperazinyl-6-trifluoromethyl-benzo[b]thiophene hydrochloride as a light pink-beige solid, 99.1% pure by GC/MS assay.

Example 3

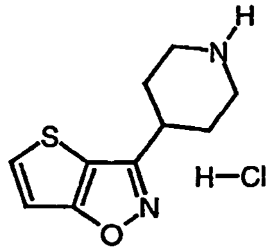

**3-Piperidinyl-4-yl-thieno[2,3-d]isoxazole hydrochloride**

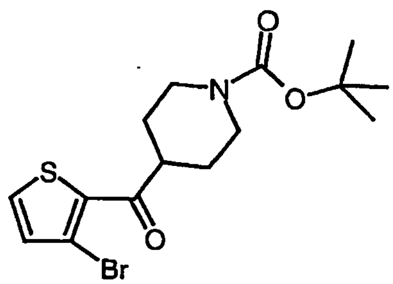

3a: 4-(3-Bromo-thiophene-2-carbonyl)-piperidine-1-carboxylic acid tert-butyl ester

Stir a solution, under nitrogen, of 3-bromothiophene (21.0 mL, 0.224 mol) in tetrahydrofuran (1.0 L) at -78˚C, and add a 2.0M solution of lithium disopropylamide in heptane/tetrahydrofuran/ethylbenzene (112 mL, 0.224 mol) for 45 min. Add, dropwise, over 2 h, a solution of 4-(N-methoxy-N-methylcarboxamido)-1-piperidinecarboxylic acid 1,1-dimethylethyl ester (prepared according to US 5,134,139) (79.4 g, 0.291 mol) in tetrahydrofuran (800 mL). Stir for 2 h, add a saturated ammonium chloride solution, and stir for an additional 0.5 h. Filter the resulting solid, and pour the filtrate into water (800 mL). Extract the aqueous mixture with ether and concentrate to obtain a dark liquid. Pour the liquid into water (400 mL), add NaCl and extract the aqueous mixture with ether. Wash the extract with water, brine, and dry over $Na_2SO_4$. Filter and concentrate to obtain the crude product. Chromatograph the product over silica gel (pet.ether/ether, 4:1) to obtain 41.5 g (50%) of white solid.

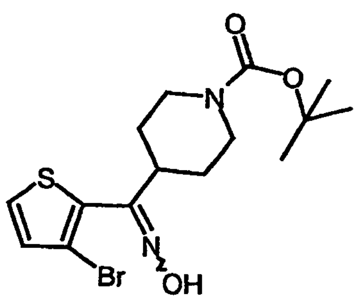

3b: 4-[(3-Bromo-thiophen-2-yl)-hydroxyimino-methyl]-piperidine-1-carboxylic acid tert-butyl ester

Stir a mixture of 4-(3-bromo-thiophene-2-carbonyl)-piperidine-1-carboxylic acid tert-butyl ester (Example 3a) (41.5 g, 0.11 mol), hydroxylamine hydrochloride (15.4 g, 0.23 mol) and pyridine (190 mL) at ambient temperature overnight. Pour the reaction into water (500 mL) and extract with dichloromethane (3x). Wash the combined extracts with saturated $CuSO_4$ solution (2x), dry ($MgSO_4$) and concentrate to a green solid. Dissolve the solid in toluene (175 mL) and let stand at ambient temperature for 3 h. Collect the resulting crystals that form and wash with toluene (60 mL). Concentrate the filtrate and again dissolve the residue in toluene and proceed to collect additional crystals to obtain a

total yield of 25 g (58%) of the title compound as a light, green solid.

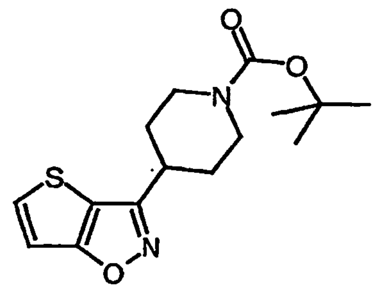

3c: 4-Thieno[2,3-d]isoxazol-3-yl-piperidine-1-carboxylic acid tert-butyl ester

Add to a stirring solution of 4-[(3-bromo-thiophen-2-yl)-hydroxyimino-methyl]piperidine-1-carboxylic acid tert-butyl ester (Example 3b) (25 g., 64.2 mmol) in 2-methoxyethanol (200 mL), a solution of potassium hydroxide (7.2 g, 128.4 mmol) in water (20 mL). Heat the reaction to 60˚C and then add copper powder (1.25 g). Stir at 60-70˚C for 6h and then at ambient temperature overnight. Pour the reaction mixture into water (500 mL) and extract with EtOAc (3x). Concentrate to a dark residue and purify by column chromatography over silica gel (heptane/EtOAc, 4:1) to provide 9.8 g (50%) of a white solid.

3d: 3-Piperidinyl-4-yl-thieno[2.3-d]isoxazole hydrochloride

Add ethereal HCl (10 mL) to 4-thieno[2,3-d]isoxazol-3-yl-piperidine-1-carboxylic acid_tert-butyl ester (Example 3c) (1.0 g, 3.2 mmol) and then methanol (1 mL) to effect solution. Permit to stand at ambient temperature for 1 h and then collect 0.34 g of white solid, mp 240-241 ˚C. From the filtrate collect 0.25 g of additional white solid, mp 263-265˚C. Both samples: MS, m/z= 209 $(M+H)^+$.

Analysis (sample mp 263-265˚C):

| | | | |
|---|---|---|---|
| Calc. For: $C_{10}H_{12}N_2OS{\cdot}HCl$: | 49.08%C | 5.35% H | 11.45%N |
| Found: | 49.03%C | 5.29%H | 11.25%N |

Example 4

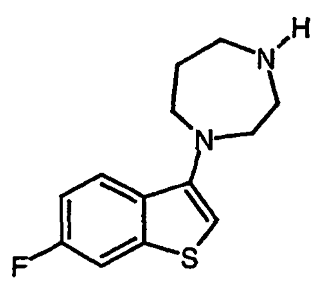

**1-(6-Fluoro-benzo[b]thiophen-3-yl)-[1,4]diazepane**

4a. 3-Amino-6-fluoro-benzo[b]thiophene-2-carboxylic acid

At 50˚C, add to a stirring solution of 2-carbomethoxy-3-amino-6-fluoro-benzo[b]thiophene (prepared according

to US 5,143,923), (90.1 g, 0.4 mol) in $H_2O$ (450 mL), a 50% aqueous solution of NaOH (64 g, 0.8 mol) over 2-3 min. Heat the reaction to 70-73˚C and continue to stir for 3 h. Add 10% aqueous isopropanol (45 mL) and bring to reflux. Remove the isopropanol under $N_2$ and add $H_2O$ (300 mL). Cool the reaction mixture to between 7-10˚C and add concentrated HCl (80 mL). Add $H_2O$ (650 mL), cool to 5-7˚C, filter the resulting solid, and wash the filter cake with $H_2O$ (2x150 mL). Dry the solid under vacuum at 35˚C to obtain 80.6 g (94.7%) of solid mp 160-163˚C, TLC on silica gel (dichloromethane/methanol, 3:1), $R_f$= 0.69.

4b. 1-(6-Fluoro-benzo[b]thiophen-3-yl)-[1,4]diazepane

Heat a solution of 3-amino-6-fluoro-benzo[b]thiophene-2-carboxylic acid (5.0 g, 24 mmol) in 1-methyl-2- pyrrolidinone (5 ml) to 100˚C for 2 h., and then, introduce a stream of nitrogen, to cool the solution to room temperature. Add homopiperazine (9.5 g, 95 mmol) and p-toluene sulfonic acid monohydrate (9.0 g, 47 mmol) and heat the mixture to 145˚C for 4 h. After that time, cool the reaction mixture to room temperature, dilute with ethyl acetate (30 mL) and wash with brine (3x15 mL). Separate the organic layer and dry over Mg $SO_4$. Evaporate the solvent and purify the crude product by column chromatography ($SiO_2$, 100 g $CH_2Cl_2$/MeOH 9:2, then $CH_2Cl_2$/MeOH/$NH_4OH$ 9:2:0.15) to give 3.9 g (65%) of yellowish oil LC/MS
(LiChrospher 5μ, RP-18, 250 mm
$CH_3CN$/ Water-gradient 20% → 100 % (25 min), Flow: 1.5 mL/min)
$t_R$= 10.74 min, m/z= 250.3.

Example 5

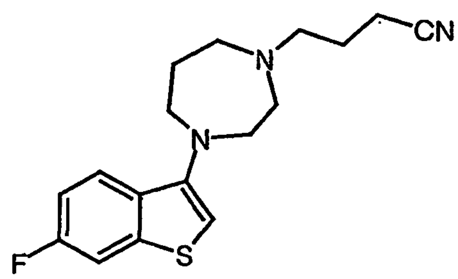

**4-(4-(6-Fluoro-benzo[b]thiophen-3-yl)-[1,4]diazapan-1-yl]butyronitrile**

Add potassium carbonate (39.3 g, 284 mmol) to a solution of 1-(6-fluoro-benzo[b]thiophen-3-yl)-[1,4]diazepane (Example 4) (23.7 g, 95 mmol) and 4-bromobutyronitrile (21.0 g, 142 mmol) in acetonitrile (400 mL) and stir the mixture under reflux for 10 h. Filter the mixture, evaporate the solvent, and dissolve the residue in ethyl acetate (EtOAc). Wash with water and saturated sodium chloride solution, and dry the organic phase over $MgSO_4$. Evaporate the solvent under vacuum, and purify the crude product by column chromatography (EtOAc/MeOH 9:1) to give 12.9 g of a yellow oil LC/MS, (LiChrospher 5 μ, RP-18, 250 mm $CH_3CN$/ Water(0.05% TFA)-gradient 2% → 98 % (20 min), Flow: 0.75 mL/min)
$t_R$= 9.46 min, m/z= 317.3.

Example 6

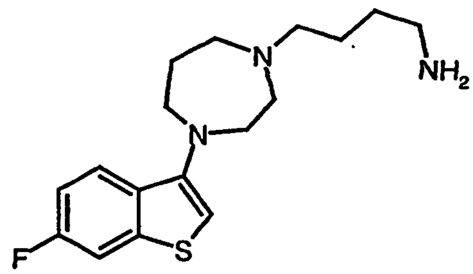

**4-[4-(6-Fluoro-benzo[b]thiophen-3-yl)-[1,4]diazapan-1-yl]butylamine**

Add over 30 min, at room temperature, a solution of $LiAlH_4$ in diethyl ether (1M, 72.5 mL) to a solution of 4-[4-(6-fluoro-benzo[b]thiophen-3-yl)-[1,4]diazapan-1-yl]butyronitrile (Example 5) (11.5 g, 36.2 mmol) in dry diethyl ether (200 mL). Heat the solution to reflux for 5 h. After that time, allow the solution to cool to room temperature and carefully quench the reaction with water and aqueous sodium hydroxide solution. Separate the phases, and re-extract the aqueous phase with EtOAc. Dry the combined organic phases over $MgSO_4$, and remove the solvent under vacuum. Purify the crude product by column chromatography ($CH_2Cl_2$/MeOH/$NH_4OH$ 9:2:0.25) to obtain 8.9 g of a colorless oil LC/MS, (LiChrospher 5μ, RP-18, 250 mm $CH_3CN$/ Water(0.05% TFA)- gradient 2% → 98 % (20 min), Flow: 0.75 mL/min), $t_R$= 7.79 min, m/z= 321.3.

Example 7

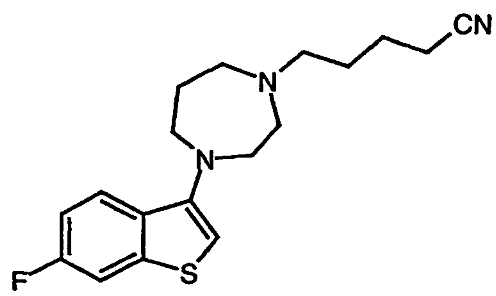

**4-[4-(6-Fluoro-benzo[b]thiophen-3-yl)-[1,4]diazapan-1-yl]pentano-nitrile**

Follow the procedure of Example 5, and substitute pentanonitrile for butyronitrile therein to obtain the title compound. (LiChrospher 5 μ, RP-18, 250 mm $CH_3CN$/ Water(0.05% TFA)-gradient 2% → 98 % (20 min), Flow: 0.75 mL/min) $t_R$=10.4 min, m/z= 331.5

Example 8

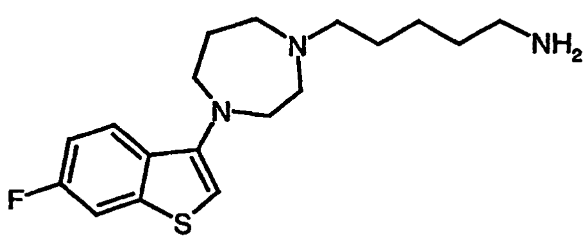

**4-[4-(6-Fluoro-benzo[b]thiophen-3-yl)[1,4]diazapan-1-yl]pentylamine**

Follow the procedure of Example 6, and substitute 4-[4-(6-fluoro-benzo[b]thiophen-3-yl)-[1,4]diazapan-1-yl] pentanonitrile (Example 7) therein to obtain the title compound. LC/MS, (LiChrospher 5 μ, RP-18, 250 mm $CH_3CN$/Water(0.05% TFA)- gradient 2% → 98 % (20 min), Flow: 0.75 mL/min), $t_R$= 8.31 min, m/z= 335.5.

Example 9

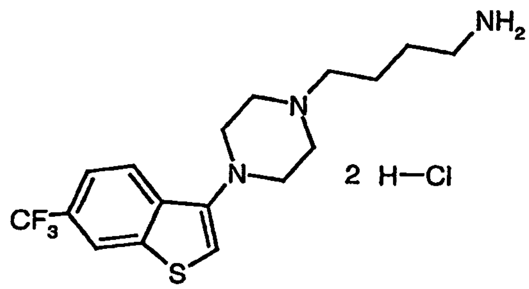

**4-[6-(Trifluoromethyl)-benzo[b]thien-1-piperazinebutanamine dihydrochloride**

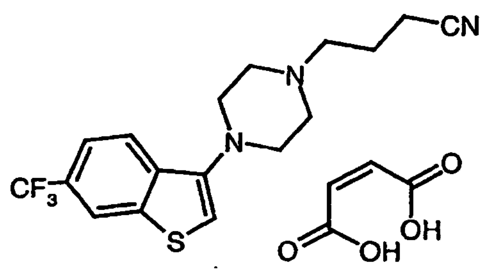

9a: 4-(6-Trifluoromethyl)-benzo[b]thien-3-yl)-1-piperazinebutyl-nitrile (Z)-2-butenedioate

Reflux a mixture of 1-(6-(trifluoromethyl)-benzo[b]thien-3-yl)-piperazine (Example 2) (10.1 g, 35.3 mmol), 4-bromobutyronitrile (6.25 g, 42.3 mmol), anhydrous potassium carbonate (8.00 g, 57.9 mmol), and anhydrous acetonitrile (80 mL) for 18 h. Filter the slurry, wash the insolubles with dichloromethane (2x150 mL), and concentrate the filtrate under vacuum. Take up the residue in dichloromethane (125 mL), wash with 5% aqueous NaOH (75 mL), water (75 mL) and dry ($K_2CO_3$). Concentrate under vacuum and chromatograph the crude product over silica gel (EtOAc) to obtain 10.3 g (82%) of amber oil. Add to an ethanolic solution of the oil (1.2 g, 3.40 mmoL), maleic acid (400 mg, 3.45 mmol) and concentrate the solution under vacuum to receive a gum. Triturate the gum with EtOAc to afford a solid. Recrystallize the solid from methanol/EtOAc to obtain 1.01 g of white crystals, mp 158-159˚C.

| Analysis: | | | |
|---|---|---|---|
| Calc. for: $C_{21}H_{22}F_3N_3O_4S$: | 53.73%C | 4.72%H | 8.95%N |
| Found: | 53.57%C | 4.65%H | 8.86%N |

9b: 4-[6-(Trifluoromethyl)-benzo[b]thien-1-piperazinebutanamine dihydrochloride

Under $N_2$, add, dropwise, a solution of 4-(6-trifluoromethyl)-benzo[b]thien-3-yl)-1-piperazinebutyl-nitrile (free base of Example 9a) (9.00 g, 25.5 mmol) in anhydrous tetrahydrofuran (THF, 70 mL) to a stirred, cooled (3˚C) suspension, of $LiAlH_4$ (1.06 g, 27.9 mmol) in anhydrous THF (120 mL). Maintain the temperature at 3˚C for 5 min and then stir at

ambient temperature for 21 h. Cool the mixture to 0° C and treat sequentially with $H_2O$ (1 mL), 15% aqueous NaOH (1 mL), and $H_2O$ (3 mL). After 20 min at room temperature, filter the mixture, wash the insolubles with dichloromethane (2x50mL), and concentrate the filtrate under vacuum. Take the residue up in dichloromethane (150 mL), wash sequentially with 5% aqueous NaOH (75 mL), $H_2O$ (75 mL) and then dry ($K_2CO_3$). Remove the solvent under vacuum and purify the residue by chromatography over silica gel (ethanol/$NH_4OH$, 95:5) to obtain 5.32 g (58%) of the free base of the title compound. To a solution of the free base (689 mg) in ethanol, add ethanolic HCl until the solution is acidic (pH 2-3). Concentrate under vacuum to a gum, and triturate the gum with ethanol to obtain an off-white solid. Recrystallize the solid from MeOH/$CHCl_3$ to obtain 485 mg of white powder, mp 256-258°C.

| Analysis: | | | |
|---|---|---|---|
| Calculated for $C_{17}H_{22}F_3N_3S$•2HCl: | 47.45%C | 5.62%H | 9.76%N |
| Found: | 47.10% | 5.67%H | 9.62%N |

Example 10

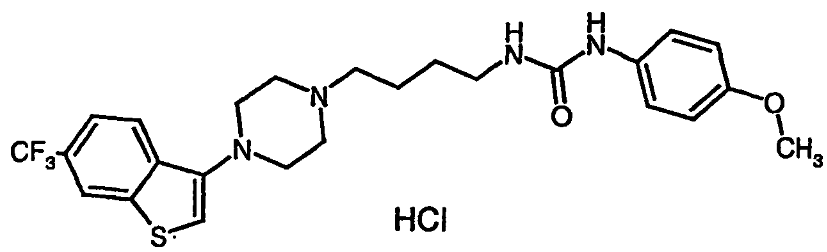

**1-(4-Methoxy-phenyl)-3-{4-[4-(6-trifluoromethyl-benzo[b]thiophen-3-yl)-piperazin-1-yl]-butyl}-urea hydrochloride**

Under nitrogen, add 4-methoxyphenyl isocyanate (0.42 g, 2.8 mmol) in $CHCl_3$ (18 mL) to a stirring solution of 4-[6-(trifluoromethyl)-benzo[b]thien-1-piperazinebutanamine (free base of Example 9b) (1.0 g, 2.8 mmol) in $CHCl_3$ (10 mL). Allow the reaction to stir at ambient temperature for 18 h, filter the resulting solid, wash with $CHCl_3$ and obtain 635 mg of product as a white solid, mp 202-204°C. Concentrate the filtrate and obtain 1.0 g of a sticky residue. Chromatograph the residue over 40 g of silica gel ($CH_2Cl_2$/MeOH, 24:1), and obtain an additional 110 mg of product. Combine the samples, dissolve in hot EtOAc-MeOH (100 mL-2 mL), filter and after cooling add ethereal HCl to precipitate the hydrochloride salt. After 2 h collect 787 mg of the salt as a white solid, mp 228-231 °C. Stir the solid in refluxing acetonitrile for 45 min, allow the mixture to cool and filter the solid to obtain 570 mg of the title compound as a white solid, mp 240-242°C.

| Analysis | | | |
|---|---|---|---|
| Calculated for $C_{25}H_{29}F_3N_4O_2S$•HCl: | 55.29%C | 5.57%H | 10.32%N |
| Found: | 55.21%C | 5.71 %H | 10.22%N |

Example 11

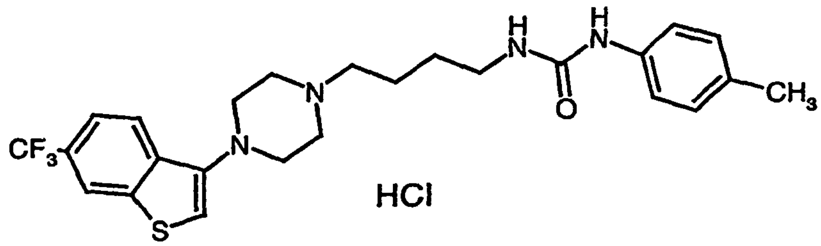

**1-(4-Methyl-phenyl)-3-{4-[4-(6-trifluoromethyl-benzo[b]thiophen-3-yl)-piperazin-1-yl]-butyl}-urea hydrochloride**

Add p-tolyl isocyanate (0.41 g, 3.1 mmol) in $CHCl_3$(8 mL) to a stirring solution of 4-[6-(trifluoromethyl)-benzo [b]thien-1-piperazinebutanamine (free base of Example 9b) (1.1 g, 3.1 mmol) in $CHCl_3$ (8 mL). Allow the reaction to stir at ambient temperature for 63 h, filter the resulting solid, wash with $CHCl_3$ and obtain 677 mg of product as a beige solid. Dissolve the solid in hot EtOAc-MeOH (100 mL-2 mL), filter and after cooling add ethereal HCl to precipitate a hydrochloride salt. Collect 869 mg of the salt as a white solid, mp 231-234˚C. Recrystallize the solid from absolute ethanol to obtain 595 mg (37%) of the title compound as a white solid, mp 240-242˚C

| Analysis | | | |
|---|---|---|---|
| Calculated for $C_{25}H_{29}F_3N_4OS{\bullet}HCl$: | 56.97%C | 5.74%H | 10.63%N |
| Found: | 57.02%C | 5.83%H | 10.68%N |

Example 12

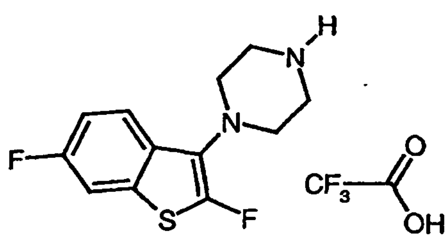

**1-(2,6-Difluoro-benzo[b]thien-3-yl)-piperazine trifluoroacetate**

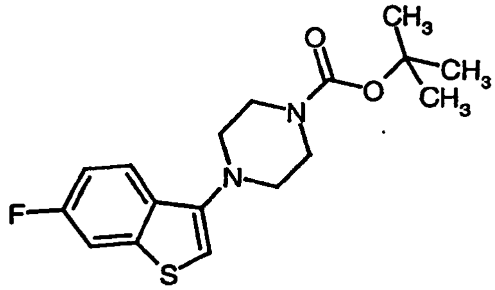

12a: 4-(6-Fluoro-benzo[b]thiophen-3-yl)-piperazine-1-carboxylic acid tert-butyl ester

Add a solution of di-*tert*-butyl dicarbonate (5.15 g, 23.6 mmol) in $CHCl_3$ (15 mL), dropwise, over 45 min to a solution at -65˚C of 1-(6-fluorobenzo[b]thiophen-3-yl)-piperazine (prepared according to US 5,143,923), (2.8 g, 11.8 mmol), 4-(dimethyl -amino)pyridine (0.16, 1.3 mmol), and diisopropylethylamine (4.3 mL, 3.2 g, 24.8 mmol) in $CHCl_3$ (50 mL). Following complete addition, stir the reaction at ambient temperature for 20 h, and then pour the reaction into a mixture of cold (5˚C) 5% aqueous NaOH/EtOAc (150/150 mL). Extract the product into EtOAc, wash the extract with $H_2O$, brine and concentrate to a red oil. Purify the crude oil over silica gel (EtOAc), to obtain 3.6 g, of red oil, LC/MS m/z= 337 $(M+H)^+$.

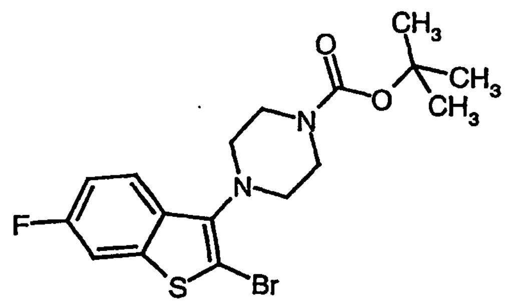

12b: 4-(2-Bromo-6-fluoro-benzo[b]thiophen-3-yl)-piperazine-1-carboxylic acid tert-butyl ester

Add N-bromosuccinimide (0.59 g, 3.3 mmol) to a stirring solution of 4-(6-fluoro-benzo[b]thiophen-3-yl)-piperazine-1-carboxylic acid tert-butyl ester (Example 12a) (1.00 g, 2.97 mmol) in $CHCl_3$ (32.8 mL) and reflux for 30 min. Allow cooling to room temperature and filter. Evaporate the solvent and purify the residue by chromatography over silica gel (EtOAc/heptane, 9:1) to obtain 0.53 g (43%) of oil, MS, m/z= 416 $(M+H)^+$.

In an alternative procedure, add N-bromosuccinimide (1.319 g, 6.62 mmol) to a stirring solution of 4-(6-fluoro-benzo[b]thiophen-3-yl)-piperazine-1-carboxylic acid tert-butyl ester (Example 12a) (2.226 g, 6.62 mmol) in $CCl_4$ and reflux for 2 h. Allow cooling to room temperature and filter. Evaporate the solvent and purify the residue by chromatography over silica gel (EtOAc/heptane, 9:1) to obtain 2.34 g (94%) of oil.

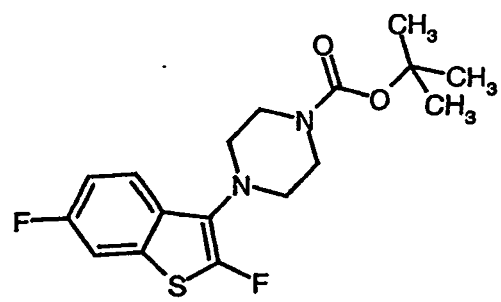

12c: 4-(2-Fluoro-6-fluoro-benzo[b]thiophen-3-yl)-piperazine-1-carboxylic acid tert-butyl ester

At a temperature of -65˚C stir, under nitrogen, a solution of the 4-(2-bromo-6-fluoro-benzo[b]thiophen-3-yl)-piperazine-1-carboxylic acid tert-butyl ester, Example 12b (15.59 g, 37.55 mmol) in anhydrous THF (247 mL) and add, dropwise, n-butyllithium in hexane (2.5M, 19.53 mL , 48.82 mmol). Stir for 30 min and then add, dropwise, N-fluorobenzenesulfonimide (17.76 g, 56.33 mmol) dissolved in anhydrous THF. Stir overnight at ambient temperature, cool the reaction to 0˚C, add saturated NaCl solution and then water. Extract the mixture with EtOAc (3x's), combine the extracts and wash with water and brine. Dry the extract ($MgSO_4$), and concentrate to obtain 11.0 g of oil. Chromatograph the oil over silica gel (ether/pet. ether, 9:1) and obtain 6.28 g (52%) of red oil, MS, m/z, 354 $(M+H)^+$.

12d: 1-(2,6-Difluoro-benzo[b]thien-3-yl)-piperazine trifluoroacetate

Stir a solution of 4-(2-fluoro -6-fluoro-benzo[b]thiophen-3-yl)-piperazine-1-carboxylic acid tert-butyl ester Example 12c (250 mg, 0.70 mmol) in trifluoroacetic acid (2.2 mL) at ambient temperature for 30 min. Evaporate the trifluoroacetic acid and treat the residue with ether. Stir the suspension at ambient temperature for 2 h, and filter the resulting white solid to obtain 191 mg (56%) of the trifluoroacetate salt. MS, m/z= 255 $(M+H)^+$.

Example 13

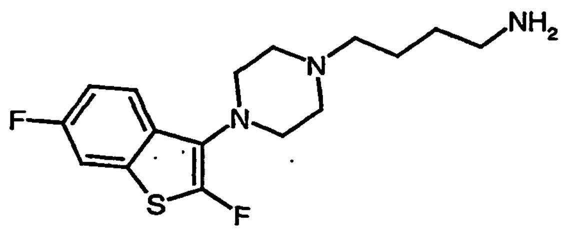

**4-[6-(2,6-Difluoro-benzo[b]thien-1-piperazinebutanamine**

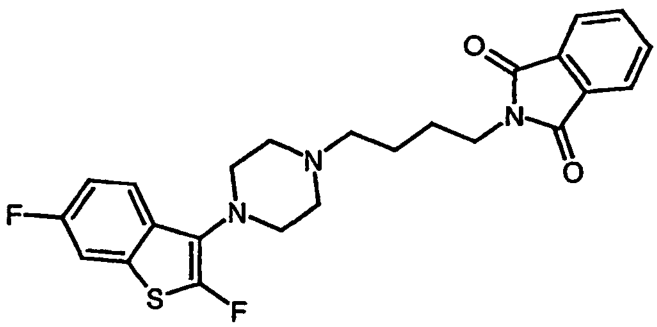

13a: 2-[4-[4-(6-Fluorobenzo[b]thiophen-3-yl)piperazin-1-yl]butylisoindole-1,3-dione

Stir and reflux under argon a mixture of 1-(2,6-difluoro-benzo[b]thien-3-yl)-piperazine (free base of Example 12d) (1.48 g, 5.8 mmol), bromobutylphthalimide (1.65 g, 5.8 mmol), triethylamine (1.2 mL) and acetonitrile (25 mL) for 4 h. Allow the reaction to cool and then dilute with dichloromethane. Wash the organic solution with water, saturated $K_2CO_3$ solution and dry ($K_2CO_3$). Concentrate the solvent and obtain 2.55 g of solid. Chromatograph the solid over silica gel ($CH_2Cl_2$/MeOH, 49:1) to obtain 2.1 g of solid, mp 123-125˚C; MS, m/z= 456 $(M+H)^+$.

13b: 4-[6-(2,6-Difluoro-benzo[b]thien-1-piperazinebutanamine

Stir a suspension, under argon, of 2-[4-[4-(6-fluorobenzo[b]thiophen-3-yl)piperazin-1-yl]butylisoindole-1,3-dione (Example 13a) (2.05 g, 4.5 mmol) in anhydrous MeOH (30 mL) and add hydrazine (0.5 mL, 15.9 mmol). Reflux for 2.5 h and allow cooling to ambient temperature. Cool the reaction in an ice bath and add 1 M HCl to a pH ~1. Filter the mixture, cool the filtrate in an ice bath, and add 50% aqueous NaOH to basify. Extract the aqueous mixture with dichloro -methane, wash the extract with $H_2O$, dry with $K_2CO_3$ and concentrate to obtain 1.4 g of oil, which crystallizes upon standing, LC/MS, m/z= 326 $(M+H)^+$.

Example 14

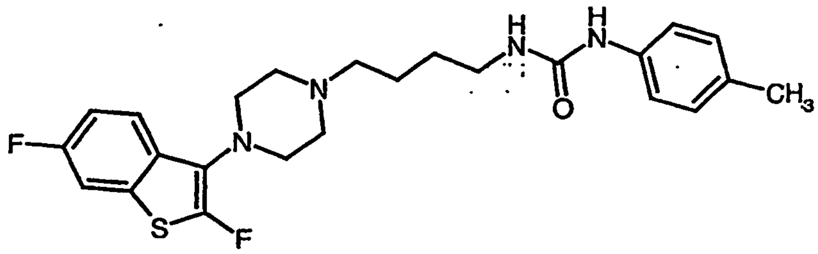

**1-(4-Methyl-phenyl)-3-{4-[4-(2,6-difluoro-benzo[b]thiophen-3-yl)-piperazin-1-yl]-butyl}-urea**

Add a solution of 4-methylphenyl isocyanate (43.3 mg, 0.32 mmol), in $CHCl_3$ (1-2 mL) to a solution of 4-[6-(trifluoromethyl)-benzo[b]thien-1-piperazinebutanamine (Example 13b) (3.5 mL of a 0.086M solution in $CHCl_3$, 0.31 mmol) and shake the reaction for 20 h. Filter the resulting solid or concentrate the reaction and collect the solid after trituration with EtOAc to obtain 102.7 mg of the title compound. LC/MS (Ymc005-AQ, 4x50 mm; water/$CH_3CN$ /acetic acid, 94.5: 5.0:0.5 100% for 1.0 min then water/$CH_3CN$ /acetic acid, 5.0:94:5:0.5 linear gradient → 100 % (2 min, hold 4 min), Flow: 1.0 mL/min) $t_R$= 3.22 min, m/z= 459 $(M=H)^+$.

Examples 15-18

The following HPLC conditions are referred to in Examples 15-18:

HPLC Condition I:

A) 95/5/0.1 % Water/Acetonitrile/Formic Acid,
B) 5/95/0.1 % Water/Acetonitrile/Formic Acid.
Column: YMC ODS-A 4x50 mm, Flow rate: 2 mL/minute.
The initial HPLC conditions consisted of 100% (A) flowing at 2 mL/minute. After the initial injection a linear gradient was performed so that at 2 minutes the HPLC conditions were 100% B. These conditions were then held for 3.4 minutes at which time the system switched back to initial conditions and equilibrated for the next analysis.

HPLC Condition II:

A) 95/5/0.1 % Water/Acetonitrile/Formic Acid,
B) 5/95/0.1 % Water/Acetonitrile/Formic Acid.
Column: YMC ODS-A 2x50 mm, Flow rate=1 mL/minute.
The initial HPLC conditions consisted of 100% (A) flowing at 0.1 mL/minute. After the initial injection a linear gradient was performed so that at 2 minutes the HPLC conditions were 100% B. These conditions were then held for 3.5 minutes at which time the system switched back to initial conditions and equilibrated for the next analysis.

HPLC Condition III:

A) 95/5/0.5% Water/Acetonitrile/Formic Acid,
B) 5/95/0.5% Water/Acetonitrile/Formic Acid.
Column: YMC ODS-A 4x50 mm, Flow rate=2 mL/minute.
The initial HPLC conditions consisted of 100% (A) flowing at 2 mL/minute. After the initial injection a linear gradient was performed so that at 2 minutes the HPLC conditions were 100% B. These conditions were then held for 3.4 minutes at which time the system switched back to initial conditions and equilibrated for the next analysis.

Example 15

**1-{4-[4-(6-Methyl-thieno[2,3-d]isoxazol-3-yl)-piperidin-1-yl]-butyl}-3-(substituted)phenyl-ureas and bis-ureas**

15a: Preparation of 4-[1-(3-bromo-4-methyl-thiophen-2-yl)-methanoyl]-piperidine-1-carboxylic acid tert-butyl ester:

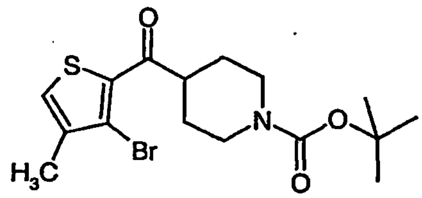

Under inert conditions, add a 2.0 M solution (in tetrahydrofuran/n-heptane) of lithium diisopropylamide (29.65 mmol, 14.83 mL, 1.05 equivalents) to a cold (-78° C) solution of 3-bromo-4-methylthiophene (28.24 mmol, 5.00 g, 1.00 equivalents) in dry tetrahydrofuran (27.33 mL). Stir at -78° C for 1 hour and add a solution of 4-(methoxy-methyl-carbamoyl)-piperidine-1-carboxylic acid *tert*-butyl ester (28.24 mmol, 7.69 g, 1.00 equivalents), dropwise. Continue stirring at -78° C for 3 hours. Quench the reaction mixture with saturated ammonium chloride (aqueous, 55 mL) and allow to warm to room temperature. Extract the reaction mixture with a mixture of ethyl acetate : diethyl ether (1 : 1, 3 x 40 mL). Combine the extracts and dry over magnesium sulfate, filter and evaporate. Purify the residue via flash column chromatography using a mixture of n-heptane : ethyl acetate (4 : 1) to yield a yellow, crystalline solid (9.84 g).
MS (CI, methane) m/e 388 ($MH^+$), LC/MS (APCI), m/e 288 (M-100), retention time 2 min. 43 sec. Condition I.

15b: Preparation of 4-[1-(3-bromo-4-methyl-thiophen-2-yl)-1-hydroxyimino-methyl]-piperidine-1-carboxylic acid tert-butyl ester:

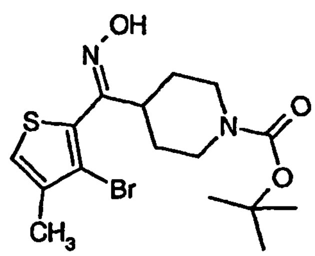

Add ammonium hydroxide hydrochloride (50.68 mmol, 3.52 g, 2.00 equivalents) to a stirred solution of 4-[1-(3-bromo-4-methyl-thiophen-2-yl)-methanoyl]-piperidine-1-carboxylic acid tert-butyl ester (25.54 mol, 9.84 g, 1.00 equivalents) in pyridine (47.5 mL). Stir at room temperature overnight and at 70° C for 4 hours. Cool the reaction mixture and add hydrochloric acid (3 M solution, 115 mL). Extract the reaction mixture with dichloromethane (115 mL), filter the organic layer, wash with water (100 mL), dry over magnesium sulfate, filter and evaporate. Recrystallize the resulting residue from toluene to yield a white solid (4.84 g). LC/MS (APCI), m/e 403 ($MH^+$), retention time 2 min. 32 sec. Condition I.

15c: Preparation of 4-(6-methyl-thieno[2,3-d]isoxazol-3-yl)-piperidine-1-carboxylic acid tert-butyl ester:

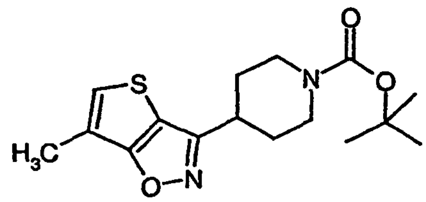

Add cesium carbonate (3.72 mmol, 1.21 g, 1.50 equivalents) and copper iodide (0.25 mmol, 47 mg, 0.10 equivalents) to a stirred solution of 4-[1-(3-bromo-4-methyl-thiophen-2-yl)-1-hydroxyimino-methyl]-piperidine-1-carboxylic acid tert-butyl ester (2.48 mmol, 1.00 g, 1.00 equivalents) in 2-methoxy ethanol (25 mL). Stir the resulting mixture at room temperature overnight and filter to remove the inorganic material. Concentrate the filtrate and partition the resulting oil between ethyl acetate (75 mL) and water (25 mL). Extract the aqueous layer with ethyl acetate (2 x 75 mL) and wash the combined organic layers with saturated sodium chloride (aqueous, 25 mL), dry over magnesium sulfate, filter and evaporate. Purify the residue via flash column chromatography eluting with n-heptane: ethyl acetate (4 : 1) to yield a white solid (588 mg). MS (Cl, methane) m/e 323 ($MH^+$), LC/MS (ESI), m/e 345 ($MNa^+$), retention time 2.05 minutes. Condition II.

15d: Preparation of 6-methyl-3-piperidin-4-yl-thieno[2,3-d]isoxazole hydrochloride:

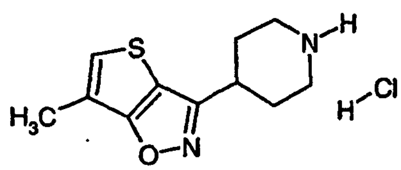

Stir a solution of 4-(6-methyl-thieno[2,3-d]isoxazol-3-yl)-piperidine-1-carboxylic acid tert-butyl ester (8.84 mmol, 2.85 g, 1.00 equivalents) in hydrochloric acid (48.75 mL, 1 M solution in diethyl ether) and methanol (2.00 mL) at room temperature for 3.5 hours. Filter the suspension, collect the white solid and dry to yield the desired product (659 mg). Allow the mother liquor to age overnight, filter, collect the white solid and dry to yield additional desired product (1.252 g). LC/MS (ESI), m/e 223 ($MH^+$), retention time 1.14 minutes. Condition II.

15e: Preparation of 4-[4-(6-methyl-thieno[2,3-d]isoxazol-3-yl)-piperidin-1-yl]-butyronitrile:

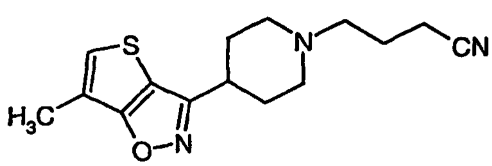

Add potassium carbonate (17.72 mmol, 2.45 g, 2.40 equivalents), potassium iodide (0.73 mmol, 123 mg, 0.10 equivalents), and 4-bromobutyronitrile (8.86 mmol, 0.88 mL, 1.20 equivalents) to a stirred solution of 6-methyl-3-piperidin-4-yl-thieno[2,3-d]isoxazole hydrochloride (7.38 mmol, 1.91 g, 1.00 equivalents) in acetonitrile (10.84 mL) and water (3.60 mL). Stir the resulting mixture at reflux overnight. Cool to room temperature, filter the reaction mixture and wash the solid material collected with dichloromethane and evaporate the filtrate. Take the residue up in dichloromethane (45 mL), wash with sodium hydroxide (aqueous, 18 mL, 2 M), water (18 mL), saturated sodium hydroxide (aqueous, 18 mL), dry over magnesium sulfate, filter and evaporate. Purify the residue via flash column chromatography using a gradient and eluting with a mixture of n-heptane : ethyl acetate (0.5: 9.5) to ethyl acetate (100%) to yield the desired product as a brown oil (663 mg). LC/MS (ESI), m/e 290 ($MH^+$), retention time 1.19 minutes. Condition II.

15f: Preparation of 4-[4-(6-methyl-thieno[2,3-d]isoxazol-3-yl)-piperidin-1-yl]-butylamine:

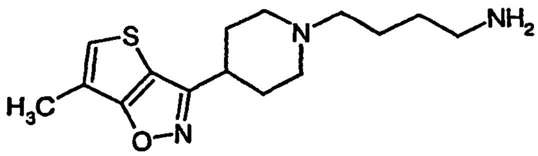

Under inert conditions, add lithium aluminum hydride (3.42 mmol, 3.42 mL, 1.50 equivalents, 1.0 M solution in tetrahydrofuran) to a stirred solution of 4-[4-(6-methyl-thieno[2,3-d]isoxazol-3-yl)-piperidin-1-yl]-butyronitrile (2.28 mmol, 660 mg, 1.00 equivalents) in tetrahydrofuran (dry, 12.86 mL). Stir the resulting solution at room temperature for 2.5 hours. Quench the reaction mixture by adding water (0.16 mL), then sodium hydroxide (aqueous, 0.16 mL, 2 M solution), and then water (0.5 mL). Dilute the resulting suspension with dichloromethane (16 mL) and vigorously stir for 30 minutes. Filter the resulting mixture through a bed of celite®, dry over magnesium sulfate, filter and evaporate to yield the desired product (457 mg) as a brown oil. LC/MS (ESI), m/e 294 ($MH^+$), retention time 0.56 minutes. Condition II.

The following 1-{4-[4-(6-Methyl-thieno[2,3-d]isoxazol-3-yl)-piperidin-1-yl]-butyl}-3-(substituted)phenyl-ureas and bis-ureas were prepared using 4-[4-(6-methyl-thieno[2,3-d]isoxazol-3-yl)-piperidin-1-yl]-butylamine (15f) and carrying out the procedures found subsequent to the table:

| Compound Number | Product | LC/MS (ESI) Condition II |
|---|---|---|
| 15-1 | 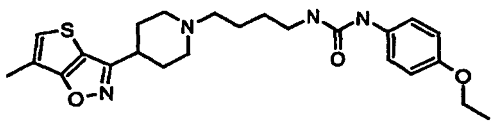 | m/e 457 ($MH^+$), retention time 1.40 minutes |
| 15-2 | 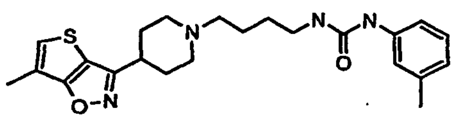 | m/e 427 ($MH^+$), retention time 1.40 minutes |
| 15-3 | 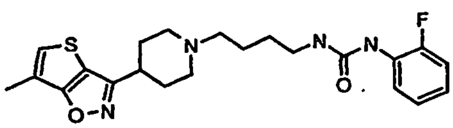 | m/e 431 ($MH^+$), retention time 1.38 minutes |
| 15-4 | 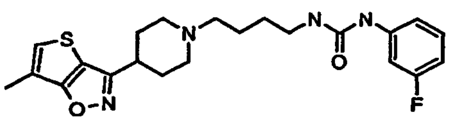 | m/e 431 ($MH^+$), retention time 1.39 minutes |
| 15-5 | 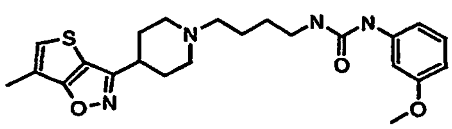 | m/e 443 ($MH^+$), retention time 1.38 minutes |
| 15-6 | 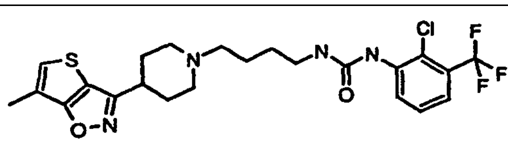 | m/e 515 ($MH^+$), retention time 1.52 minutes |

(continued)

| Compound Number | Product | LC/MS (ESI) Condition II |
|---|---|---|
| 15-7 | 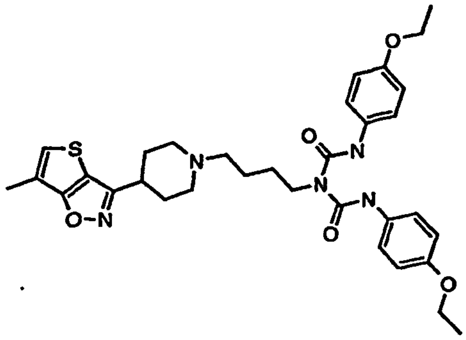 | m/e 620 ($MH^+$), retention time 1.65 minutes |
| 15-8 | 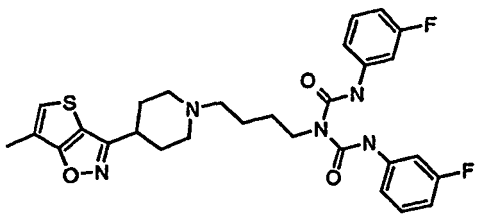 | m/e 567 ($MH^+$), retention time 1.62 minutes |
| 15-9 | 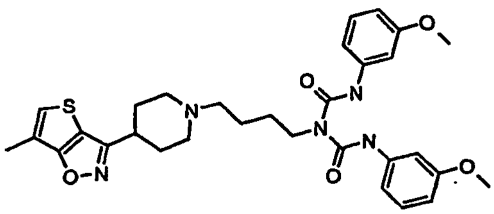 | m/e 592 ($MH^+$), retention time 1.59 minutes |
| 15-10 | 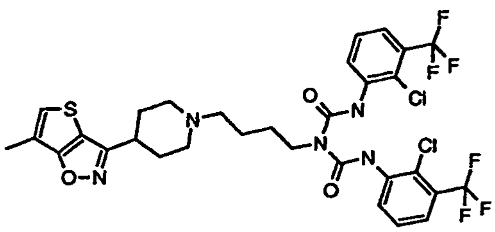 | m/e 736 ($MH^+$), retention time 1.80 minutes |

Add a solution of 4-[4-(6-methyl-thieno[2,3-d]isoxazol-3-yl)-piperidin-1-yl]-butylamine (0.26 mmol, 75 mg, 1.00 equivalents) in dichloromethane (1.00 mL) to the desired isocyanate (0.50 mmol, 1.95 equivalents). Shake each reaction mixture overnight at room temperature evaporate and purify the reaction mixtures via flash column chromatograph (ISCO Combi Flash) using a gradient and eluting with ethyl acetate (100%) to a mixture of ethanol in ethyl acetate (1.5 : 8.5). Concentrate to give each desired product

Example 16

**Preparation of 1-{4-[4-(6-Fluoro-benzo[b]thiophen-3-yl)-[1,4]diazepan-1-yl]-butyl}-3-(substituted)phenyl-ureas**

| Compound Number | Product | LC/MS (APCI) Condition III |
|---|---|---|
| 16-1 | 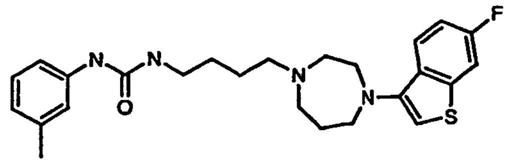 | m/e 455 ($MH^+$), retention time 1 min. 44 sec. |
| 16-2 | 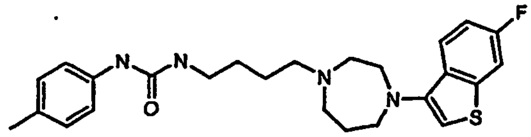 | m/e 455 ($MH^+$), retention time 1 min. 45 sec. |
| 16-3 | 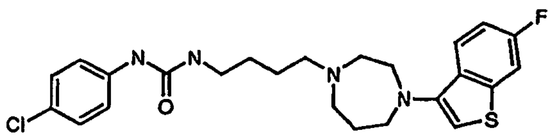 | m/e 475 ($MH^+$), retention time 1 min.46 sec. |
| 16-4 | 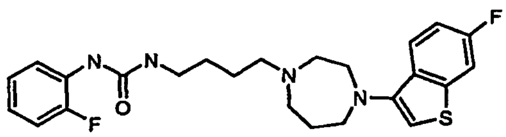 | m/e 459 ($MH^+$), retention time 1 min. 42 sec. |
| 16-5 | 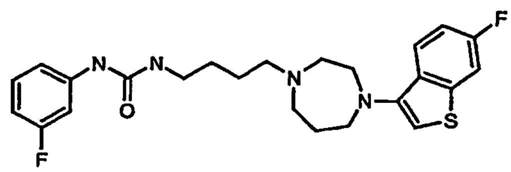 | m/e 459 ($MH^+$), retention time 1 min. 43 sec. |
| 16-6 | 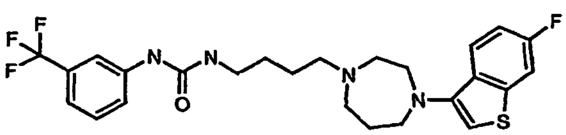 | m/e 509 ($MH^+$), retention time 1 min. 47 sec. |
| 16-7 | 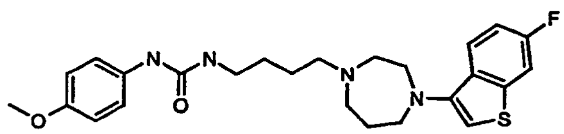 | m/e 471 ($MH^+$), retention time 1 min. 41 sec. |
| 16-8 | 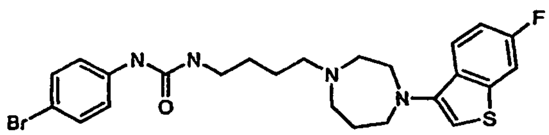 | m/e 519 ($MH^+$), retention time 1 min. 47 sec. |
| 16-9 | 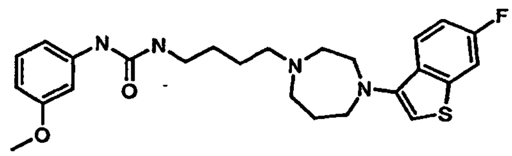 | m/e 471 ($MH^+$), retention time 1 min. 42 sec. |
| 16-10 | 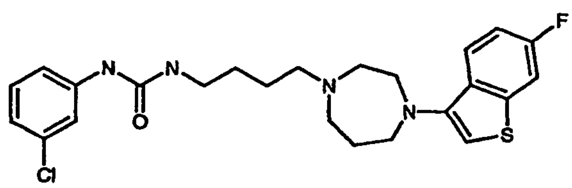 | m/e 475 ($MH^+$), retention time 1 min. 46 sec. |

(continued)

| Compound Number | Product | LC/MS (APCI) Condition III |
|---|---|---|
| 16-11 | 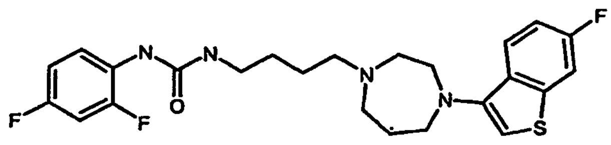 | m/e 477 (MH+), retention time 1 min. 43 sec. |
| 16-12 | 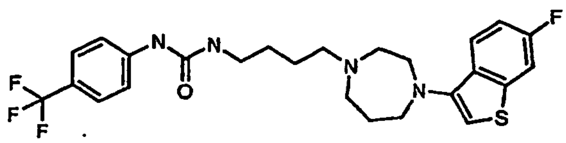 | m/e 509 (MH+), retention time 1 min. 48 sec. |
| 16-13 | 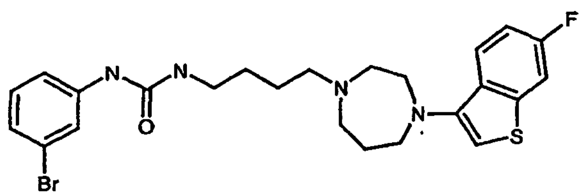 | m/e 519 (MH+), retention time 1 min. 47 sec. |
| 16-14 | 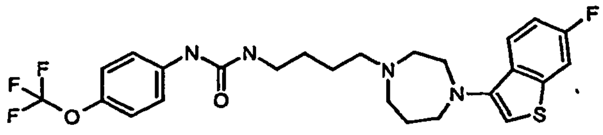 | m/e 525 (MH+), retention time 1 min. 49 sec. |
| 16-15 | 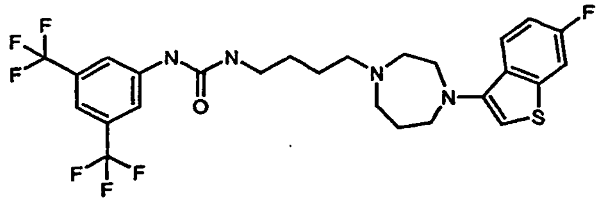 | m/e 577 (MH+), retention time 1 min. 55 sec. |
| 16-16 | 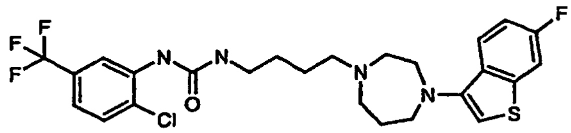 | m/e 543 (MH+), retention time 1 min. 51 sec. |
| 16-17 | 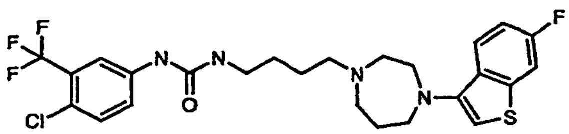 | m/e 543 (MH+), retention time 1 min. 51 sec. |
| 16-18 | 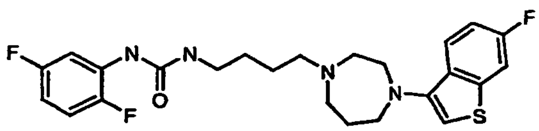 | m/e 477 (MH+), retention time 1 min. 46 sec. |
| 16-19 | 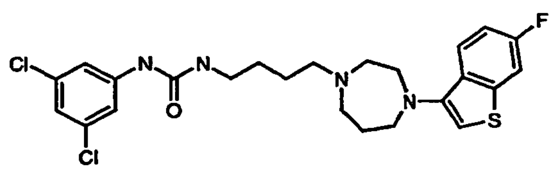 | m/e 509 (MH+), retention time 1 min. 51 sec. |

(continued)

| Compound Number | Product | LC/MS (APCI) Condition III |
|---|---|---|
| 16-20 | 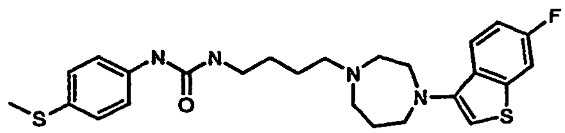 | m/e 487 (MH+), retention time 1 min. 46 sec. |
| 16-21 | 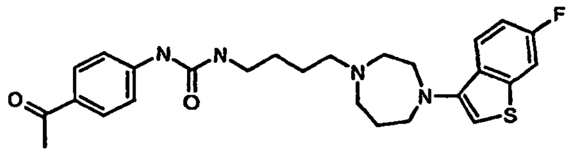 | m/e 483 (MH+), retention time 1 min. 39 sec. |
| 16-22 | 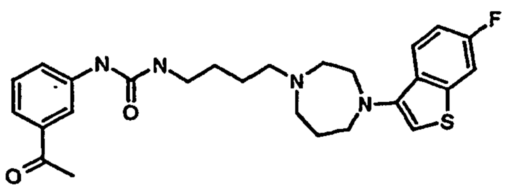 | m/e 483 (MH+), retention time 1 min. 40 sec. |
| 16-23 | 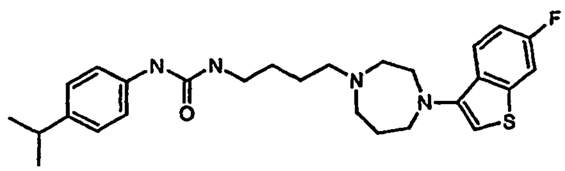 | m/e 483 (MH+), retention time 1 min. 50 sec. |
| 16-24 | 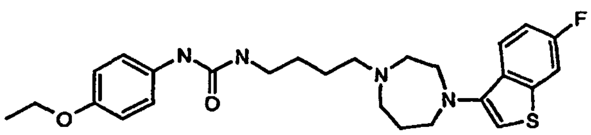 | m/e 485 (MH+), retention time 1 min. 44 sec. |
| 16-25 | 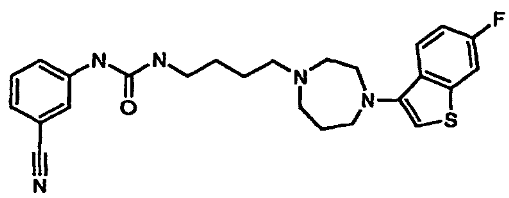 | m/e 466 (MH+), retention time 1 min. 43 sec. |
| 16-26. | 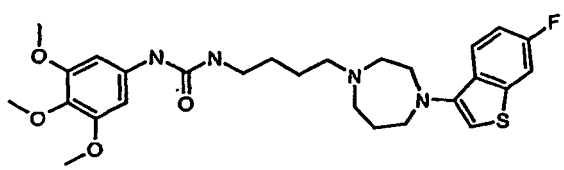 | m/e 531 (MH+), retention time 1 min. 39 sec. |
| 16-27 | 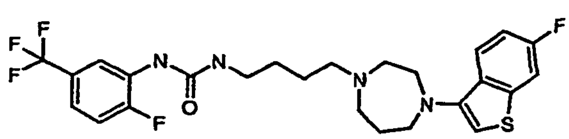 | m/e 527 (MH+), retention time 1 min. 49 sec. |
| 16-28 | 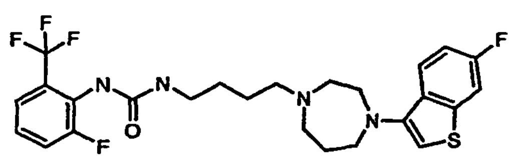 | m/e 527 (MH+), retention time. 1 min. 43 sec. |

(continued)

| Compound Number | Product | LC/MS (APCI) Condition III |
|---|---|---|
| 16-29 | 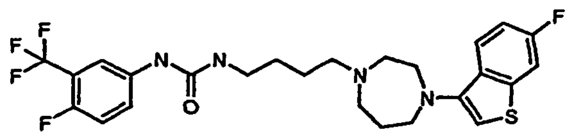 | m/e 527 ($MH^+$), retention time 1 min. 50 sec. |
| 16-30 | 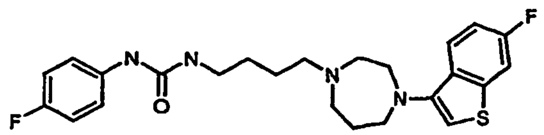 | m/e 459 ($MH^+$), retention time 1 min. 42 sec. |

The above compounds were prepared in a manner analogous to Example 15. Evaporate the pure fractions from a purification performed via flash column chromatograph (ISCO Combi Flash) to yield each compound as an oil. Dry each oil under the vacuum pump to give the final, desired compounds.

Example 17

**4-(5-methyl-thieno[2,3-d]isoxazol-3-yl)-piperidine-1-carboxylic acid**

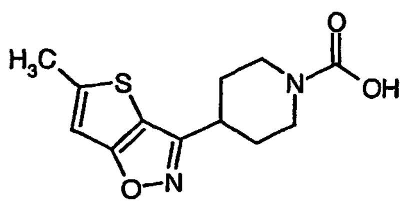

17a: Preparation of 4-[1-(3-bromo-5-methyl-thiophen-2-yl)-methanoyl]-piperidine-1-carboxylic acid tert-butyl ester:

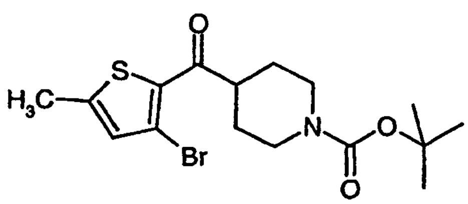

Prepared essentially as in Example 15a except that 2-bromo-5-methyl thiophene is used as the starting material. In addition, 1.20 equivalents of lithium diisopropylamide and 1.24 equivalents of 4-(methoxy-methyl-carbamoyl)-piperidine-1-carboxylic acid *tert*-butyl ester are used for the reaction. Accordingly, stirring time of the reaction mixture may vary. Purification of the residue via flash column chromatography uses a gradient with a mixture of ethyl acetate : n-heptane (1 : 9) to ethyl acetate : n-heptane (2 :8) to yield a yellow oil. LC/MS (ESI), m/e 332 (M-56) and 388 ($MH^+$), retention time 2.15 minutes. Condition II.

17b: Preparation of 4-[1-(3-bromo-5-methyl-thiophen-2-yl)-1-hydroxyimino-methyl]-piperidine-1-carboxylic acid tert-butyl ester:

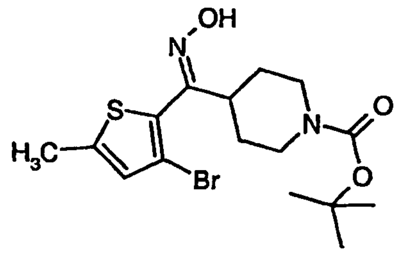

Prepared essentially as Example 15b except that 4-[1-(3-Bromo-5-methyl-thiophen-2-yl)-methanoyl]-piperidine-1-carboxylic acid tert-butyl ester is used as the starting material and the reaction mixture was stirred at 70° C for 6 hours. LC/MS (ESI), m/e 347 (M-56) and 403 ($MH^+$), retention time 2.03 minutes. Condition II.

17c: Preparation of 4-(5-methyl-thieno[2,3-d]isoxazol-3-yl)-piperidine-1-carboxylic acid:

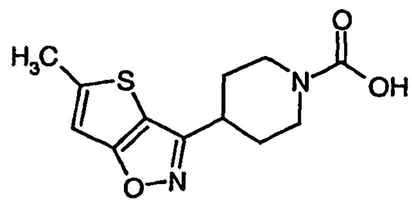

Prepared essentially as Example 15c except that 4-[1-(3-bromo-5-methyl-thiophen-2-yl)-1-hydroxyimino-methyl]-piperidine-1-carboxylic acid tert-butyl ester is used as the starting material. Two other differences are: 1) 0.05 equivalents of copper iodide is used, and 2) no partition between ethyl acetate and water accompanied by subsequent extraction with ethyl acetate is required. Purification of the residue via flash column chromatography uses a mixture of ethyl acetate : n-heptane (1 : 4) to yield a white solid. LC/MS (ESI), m/e 345 ($MNa^+$), retention time 2.12 minutes. Condition II.

Example 18

**5-methoxymethyl-3-piperidin-4-yl-thieno[2,3-d]isoxazole hydrochloride**

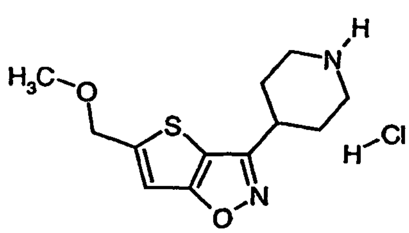

18a: Preparation of (4-bromo-thiophen-2-yl)-methanol:

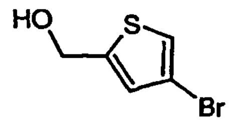

Under inert conditions, add sodium borohydride (13.82 mmol, 0.523 g, 2.08 equivalents) in absolute ethanol (16 mL) dropwise over a period of 15 minutes to a stirred mixture of 4-bromothiophene-2-carboxaldehyde (26.58 mmol, 5.08 g, 1.00 equivalents) in cold (0° C) absolute ethanol (32 mL). Stir the resulting mixture at room temperature for 2.5 hours and add glacial acetic acid dropwise until the effervescence ceases. Evaporate the resulting solution, take the residue up in diethyl ether (75 mL), wash with water (15 mL) and brine (15 mL) and dry over magnesium sulfate. Filter and evaporate to yield the product as a colorless oil (5.13 g).

18b: Preparation of 4-bromo-2-methoxymethyl-thiophene:

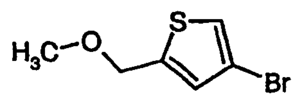

Add sodium hydride (737 mg, 29.23 mmol, 1.10 equivalents, 95%) to a solution containing methyl iodide (1.65 mL, 26.57 mmol, 1.00 equivalents) and (4-bromo-thiophen-2-yl)-methanol (5.13 g, 26.57 mmol, 1.00 equivalents) in tetrahydrofuran (dry, 25 mL). Stir the resulting mixture at room temperature overnight and evaporate. Partition the residue between water (100 mL) and dichloromethane (100 mL). Extract the aqueous layer with dichloromethane (100 mL), combine the organic layers, dry over magnesium sulfate, filter and evaporate to yield the desired product as a yellow oil.

18c: Preparation of 4-[1-(3-bromo-5-methoxymethyl-thiophen-2-yl)-methanoyl]-piperidine-1-carboxylic acid tert-butyl ester:

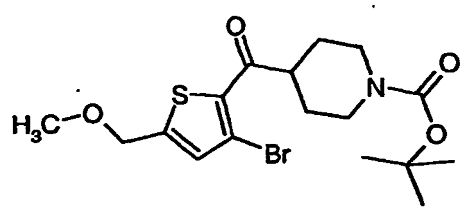

Add lithium diisopropyl amide (13.20 mL, 26.37 mmol, 1.05 equivalents) to a stirred, cold (-78° C) solution of 4-bromo-2-methoxymethyl-thiophene (5.20 g, 25.11 mmol, 1.00 equivalents) in tetrahydrofuran (dry, 24.30 mL). Stir at -78° C for 1 hour and add a solution of 4-(methoxy-methyl-carbamoyl)-piperidine-1-carboxylic acid tert-butyl ester (6.84 g, 25.11 mmol, 1.00 equivalents) in tetrahydrofuran (dry, 16.40 mL), dropwise. Stir the resulting solution at -78° C for 3 hours. Quench the reaction mixture with saturated sodium chloride (aqueous, 50 mL). Allow the resulting mixture to warm to room temperature and extract with a mixture of ethyl acetate : diethyl ether (1 :1,3 x 35 mL). Combine the extracts, dry over magnesium sulfate, filter and evaporate. Purify the residue via flash column chromatography eluting with a mixture of n-heptane : ethyl acetate (4:1) to yield the desired product as a yellow oil (9.47 g). LC/MS (ESI), m/e 362 (M-56) and 418 ($MH^+$), retention time 2.08 minutes. Condition II.

18d: Preparation of 4-[1-(3-bromo-5-methoxymethyl-thiophen-2-yl)-1-hydroxyimino-methyl]-piperidine-1-carboxylic acid tert-butyl ester:

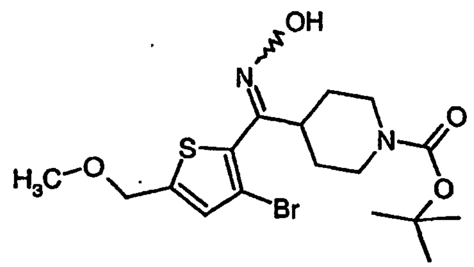

Add hydroxylamine hydrochloride (2.29 g, 45.27 mmol, 2.00 equivalents) to a stirred solution of 4-[1-(3-bromo-5-methoxymethyl-thiophen-2-yl)-methanoyl]-piperidine-1-carboxylic acid tert-butyl ester (9.47 g, 22.64 mmol, 1.00 equivalents) in pyridine (42.40 mL). Stir the resulting solution at room temperature overnight and then at 70° C for 4 hours. Cool the reaction mixture slightly, add hydrochloric acid (3N, 100 mL) and extract the resulting mixture with dichloromethane (100 mL). Wash the extract with water (100 mL), dry over magnesium sulfate, filter and evaporate to yield the desired product as a yellow oil (9.48 g).

18e: <u>Preparation of 4-(5-methoxymethyl-thieno[2,3-d]isoxazol-3-yl)-piperidine-1-carboxylic acid tert-butyl ester:</u>

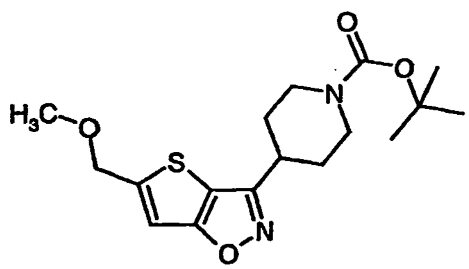

Add cesium carbonate (1.13 g, 3.46 mmol, 1.50 equivalents) and copper iodide (44 mg, 0.23 mmol, 0.10 equivalents) to a stirred solution of 4-[1-(3-bromo-5-methoxymethyl-thiophen-2-yl)-1-hydroxyimino-methyl]-piperidine-1-carboxylic acid tert-butyl ester (1.00 g, 2.31 mmol, 1.00 equivalents) in 2-methoxy ethanol (23.30 mL). Stir the resulting mixture at room temperature overnight or up to 3 days and filter through celite. Evaporate the filtrate, partition the residue between ethyl acetate (70 mL) and water (23 mL) and separate. Extract the aqueous layer with ethyl acetate (3 x 70 mL), combine the organic layers, dry over magnesium sulfate, filter and evaporate. Purify the residue via flash column chromatography eluting with a mixture of hexane : ethyl acetate (4:1) to yield the desired product as a yellow oil. LC/MS (ESI), m/e 375 ($MNa^+$), retention time 1.98 minutes. Condition II.

18f: <u>Preparation of 5-methoxymethyl-3-piperidin-4-yl-thieno[2,3-d]isoxazole hydrochloride:</u>

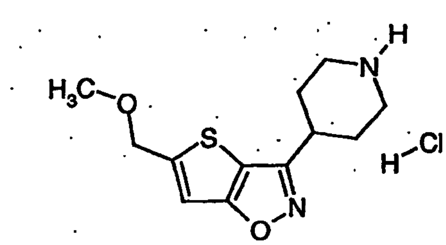

Stir a solution of 4-(5-methoxymethyl-thieno[2,3-d]isoxazol-3-yl)-piperidine-1-carboxylic acid tert-butyl ester (2.21 g, 6.68 mmol, 1.00 equivalents) and hydrochloric acid (1.0 M in diethly ether, 35 mL) overnight to form a suspension. Add additional hydrochloric acid (1.0 M in diethyl ether, 10 mL). Stir the suspension overnight, filter and wash the solid

with ether. Collect the solid and dry to yield the desired product as a dark blue solid. LC/MS (ESI), m/e 253 ($MH^+$), retention time 1.17 minutes. Condition II.

**Example 19**

Scheme V

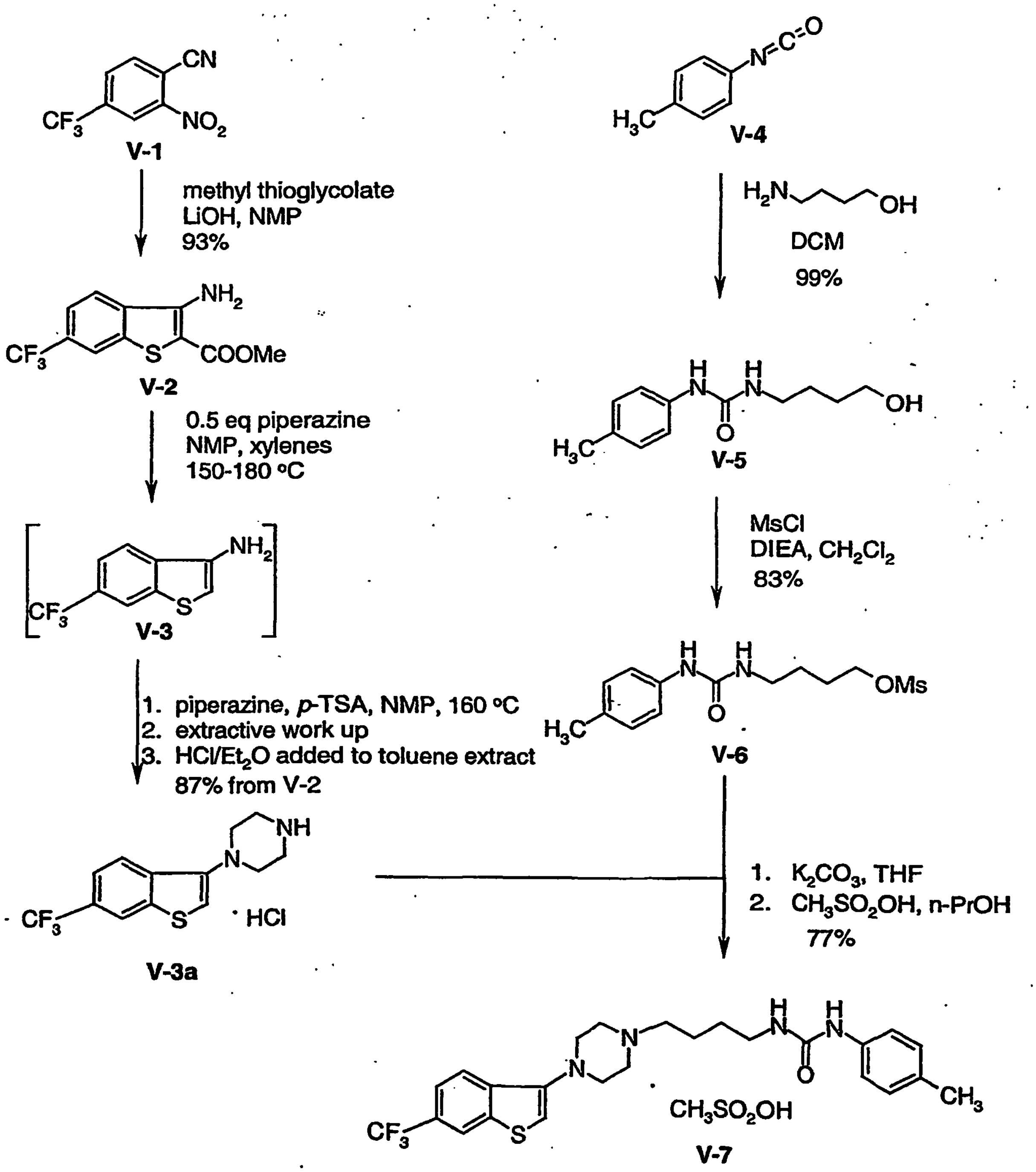


**General:** Gas chromatography/mass spectroscopy was accomplished using a HP Model 5972 system with

the following conditions: 0.25 mm x 30 m, HP 5MS column, cross-linked 5% Ph Me silicone, 0.25 μ film thickness; injector at 250 ˚C; detector at 280 ˚C; 50 ˚C for 1 min, ramp at 20 ˚C/min to 300 ˚C, 300 ˚C for 5 min to 10 min.

**2-Carbomethoxy-3-amino-6-trifluoromethylbenzo[b]thiophene (V-2):**

A 22-L, 3-necked, round-bottom flask equipped with a mechanical stirrer, nitrogen bubbler, and a thermocouple probe, was charged with 1.20 kg (5.55 mole) of 2-nitro-4-trifluoromethylbenzonitrile, 496 mL (589.3 g, 5.55 mole) of methyl thioglycolate, and 4.3 L of NMP. The resulting yellow solution was cooled (-10 ˚C bath) to 1 ˚C, and a solution prepared from 466.0 g (11.11 mole, 2.0 eq) of lithium hydroxide monohydrate in 3.36 L of water was added over a period of 2 h while maintaining a temperature of 2 - 16 ˚C. The tan-orange slurry was stirred at 0 - 8 ˚C for 45 min, then was diluted with 8.0 L of water ($T_{exo}$ -> 21 ˚C). After stirring for 30 min and cooling to 14 ˚C, the product was collected by filtration, rinsing with 15 L of water, then air-drying at ambient temperature to give 1.43 kg (93.5% yield) of 2-carbomethoxy-3-amino-6-trifluoromethylbenzo-[b]thiophene, as a light-yellow solid.

**3-Piperazinyl-6-trifluoromethylbenzo[b]thiophene hydrochloride (V-3a):**

A 22-L, 3-necked, round-bottom flask equipped with a mechanical stirrer, nitrogen bubbler, and a thermocouple probe, was charged with 1.42 kg (5.17 mole) of 2-carbomethoxy-3-amino-6-trifluoromethylbenzo-[b]thiophene (V-2), 245.0 g (2.84 mole, 0.55 eq) of piperazine, 5.0 L of NMP, and 720 mL of xylene. The solution was heated to and held at 165 -176 ˚C for 4.5 h, at which time the reaction was ca. 97% complete as determined by hplc assay. A total of 2.00 kg (23.22 mole, 4.5 eq) of piperazine (T - > 120 ˚C) and 1.97 kg (10.36 mole, 2.0 eq) of *p*-toluenesulfonic acid monohydrate (exotherm observed, 120 > 140 ˚C) were added to the brown solution. A Dean-Stark trap was connected to the condenser, and the reaction was heated to collect an azeotrope. A total of 350 mL of an aqueous distillate was removed, allowing the pot temperature to increase from 140 to 158 ˚C. The Dean-Stark trap was disconnected. The progress of the reaction was monitored by GC/MS assays. After 12 h at ca. 158 - 162 ˚C (>99% conversion by GC/MS assay), the reaction was cooled to 40 ˚C, then quenched into an extractor that contained 6.2 kg of ice, 15 L of water, and 10.6 L of toluene. The phases were separated, and the aqueous layer was extracted with 2 L of toluene. The combined organic extract was washed with 13.7 L of 0.5 N NaOH followed by 2.5 L of saturated aq. NaCl, then was extracted with 10 L of 1 N HCl. The acidic aqueous extract was diluted with 1.2 kg of ice, then was basified to pH 10.7 by adding 770 g of 50% NaOH. The resulting mixture was extracted with 11 L of toluene. The toluene extract was washed with 2.5 L of saturated aq. NaCl, dried ($Na_2SO_4$), and filtered. The filtrate was charged into a 22 L, 3-necked, round-bottomed flask ($N_2$, mechanical stirring, TC probe). A total of 4.5 L of 1 N ethereal HCl was added at 20 - 27 ˚C until the mixture was positive to Congo Red indicator paper. After stirring at ambient temperature for 35 min, the slurry was filtered and washed with 5 L of toluene. After air drying, 1.44 kg (86.5% yield) of 3-piperazinyl-6-trifluoromethylbenzo[b]thiophene hydrochloride **(V-3a)** was obtained as a light pink-beige solid.

***N*-(4-Hydroxybutyl)-*N'*-(4-tolyl) urea (V-5):**

A 12-L, 3-necked, round-bottom flask equipped with a mechanical stirrer, nitrogen bubbler, and a thermocouple probe, was charged with 8 L of methylene chloride and 367.7 g (4.13 moles, 1.10 eq.) of 4-amino-1-butanol, and the resulting solution was cooled to 0 - 5 ˚C. A solution of 499.3 g (3.75 moles, 1.00 eq.) of 4-tolyl isocyanate in 0.5 L of methylene chloride was added over a period of 2.5 h while maintaining a temperature of 0-12 ˚C. The white slurry was stirred for 1 h at 0-10 ˚C, then was filtered, washed with 3 L of methylene chloride, and air dried to afford 0.83 kg (99.9%) of *N*-(4-hydroxybutyl)-*N'*-(4-tolyl) urea **(V-5)** as a white solid.

***N*-(4-Butylmesylate)-*N'*-(4-tolyl) urea (V-6):**

A 22-L, 3-necked, round-bottom flask equipped with a mechanical stirrer, nitrogen bubbler and a thermocouple probe, was charged with 2.057 kg (9.25 moles) of *N*-(4-hydroxybutyl)-*N'*-(4-totyl) urea (V-5), 13 L of DCM and 2.02 L (1.5 kg, 11.57 mole, 1.25 eq) of diisopropylethylamine. The white suspension was cooled to 6 ˚C, and a solution of 1.22 kg (10.64 mole, 1.15 eq) of methanesulfonyl chloride in 0.25 L of DCM was added over a period of ca. 2 h while maintaining a pot temperature of 5 -12 ˚C. After stirring for 10 min at 5 -10 ˚C (100% conversion by hplc assay) the light-yellow solution was quenched into an extractor that contained 9 L of 1 N HCl. The phases were separated. The organic phase was washed with 9 L of 1 N HCl, washed with 9 L of 5% aq. $NaHCO_3$, dried ($MgSO_4$), filtered and then was partially concentrated (30 ˚C, 150 mbar) to a mass of 6.41 kg. After stirring in an ice bath for 30 min, the resulting slurry was filtered, washed with 2 L of cold DCM, and air dried to give 2.32 kg (83.4%) of *N*-(4-butylmesylate)-*N'*-(4-tolyl) urea **(V-6)** as a white solid.

**1-*p*-Tolyl-3-[4-[4-(6-trifluoromethylbenzo[b]thieny-3-yl)piperazin-1-yl]butyl]-urea (V-7, free base):**

A 22-L, 3-necked, round-bottom flask equipped with a mechanical stirrer, nitrogen bubbler, and a thermocouple probe was charged with 1.42 kg (4.40 mole) of V-3a, 1.757 kg (5.85 mole, 1.33 eq) of *N*-(4-butylmesylate)-*N'*-(4-tolyl) urea **(V-6)**, 4.26 L of THF, 1.23 L of water, and 1.52 kg (11.00 mole, 2.50 eq) of $K_2CO_3$. Using a temperature controller, the biphasic mixture was heated at 48 - 51 ˚C for 22 h. The resulting thick slurry was charged to a 20-gal glass-lined steel reactor along with rinses of 9.8 L of THF and 1.05 L water, and heated to reflux (65 ˚C). A total of 13.1 L of water was added to the biphasic mixture over a period of ca. 10 min during which the temperature dropped to ca. 60˚C. The batch was then slowly cooled (crystallization occurred at 43 ˚C) to 0 - 5 ˚C, and aged at this temperature for 30 min, then was filtered, slurry-rinsed in several portions using a cold mixture of 5 L THF/0.42 L water, and given a final rinse with 3 x 12 L of water. After air drying, 1.72 kg (79.7%) of 1-*p*-tolyl-3-[4-[4-(6-trifluoromethylbenzo[b]thieny-3-yl)piperazin-1-yl]butyl]-urea (**V-7**, free base) as a white, fluffy solid was obtained.

**1-*p*-Tolyl-3-[4-[4-(6-trifluoromethylbenzo[b]thieny-3-yl)piperazin-1-yl]butyl]-urea methanesulfonate (V-7):**

A 22-L, 3-necked, round-bottom flask equipped with a mechanical stirrer, nitrogen bubbler, and a thermocouple probe, was charged with 4.045 kg (8.245 mole) of **V-7** (free base) and 11 L of *n*-propanol. A solution of 792.4 g (8.245, 1.00 eq) of methanesulfonic acid in 1 L of *n*-propanol was added in one portion. An exotherm was observed (T -> 39˚C), and the mixture became nearly homogeneous at the end of the addition. The mixture was heated to 92 ˚C, at which temperature all solids had dissolved. The solution was filtered through a coarse frit to remove a trace amount of extraneous matter. The clear, red-brown filtrate was charge into a 20-gal glass-lined steel reactor along with 3.3 L of *n*-PrOH rinse. After slowly cooling, and seeding at 51 ˚C, crystallization commenced at 47 ˚C. Upon further crystallization and cooling to 37 ˚C, the slurry became very thick and was diluted with 12.1 L of *n*-PrOH. After cooling to 0-10 ˚C and stirring for 60 min, product was collected by filtering, rinsing with 5 L of cold *n*-PrOH, and drying (tray oven, 105-115 ˚C, 100 mm, $N_2$ bleed) to give 4.67 kg (96.5) of 1-*p*-tolyl-3-[4-[4-(6-trifluoromethylbenzo[b]thieny-3-yl)piperazin-1-yl]butyl] urea methanesulfonate (**V-7**)), as a white to off-white solid.

**Example 20**

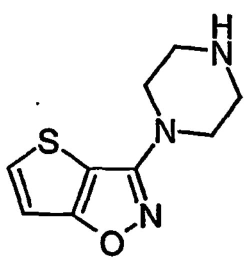

Synthesis of BOC protected piperazine-thienylisoxazole

**3-Bromothiophene-2-carbaldehyde oxime**

3-Bromothiophene-2-carbaldehyde (Maybridge) (28.7gm, 0.15 mol) in ethanol (50ml) was added in one portion to a solution of hydroxylamine hydrochloride (13.8 gm, 0.2 mole), sodium hydroxide (8 gm, 0.2 mol) in water (30ml) and ethanol (100 ml). The mixture was stirred at 0˚C for 2 hours and was kept at 0˚C overnight. The reaction mixture was diluted with cold water (600 ml), and the precipitated solids were collected by filtration to provide 20.5 gm, (67%) of product. The aqueous layer was further extracted with ethyl acetate and, the combined organic layers were washed with brine, dried with magnesium sulfate filtered and concentrated in vacuo to leave an additional 6.9g of product.

**3-Bromothiophene-2-hydroximidoyl chloride:**

To a solution of 3-bromothiophene-2-carbaldehyde oxime (10.8gm, 52.4 mmol), hydrogen chloride (14.5ml, 4M in dioxane) in DMF (100ml) was added *oxone* (16.9gm, 1.05 eqiv) in one portion at room temperature. The mixture was stirred at ambient temperature overnight. At the end of the reaction, DMF solution was poured into water and product was extracted into ethyl acetate. The organic solution was washed with brine, dried over magnesium sulfate, filtered

and concentrated in vacuo to 12.68gm of product which was used in the next reaction without furthur purification.

**(4-t-Butoxycarbonylpiperazinyl)-3-bromo-2-thienyl methanone oxime:**

3-bromothiophene-2-hydroximidoyl chloride (16.4gm, 68 mmol) in tetrahydrofuran (THF, 70ml) was added dropwise to a solution of N-(t-butoxycarbonyl)piperazine (14gm, 1.1 equiv.), DABCO (9.5gm, 1.25eqiv.) in DMF (100ml) at 0˚C over 25 minutes. The mixture was stirred for 3.5 hrs. At the end, the mixture was poured into water and was extracted with ethyl acetate. The organic was washed with brine and dried over magnesium sulfate. The solvent was removed on a rotary evaporator. The crude product (30.5gm) was purified by chromatography on a Biotage cartridge (400gm of silica gel), eluting with methanol in dichloromethane (0-5 % of MeOH). The product thus obtained weighed 24.6 gm (85%).

**(t-BOC-piperazine)-3-thienylbenzisoxazole:**

A mixture of (4-t-Butoxycarbonylpiperazinyl)-3-bromo-2-thienyl methanone oxime (10.3gm, 26.4 mmol), cesium carbonate (10.7gm, 32.7mmol), and copper iodide (500mg) in methoxyethanol (200ml) was stirred at room temperature overnight. The reaction mixture was diluted with ethyl acetate, the washed with water. The aqueous solution was extracted three times with ethyl acetate. The organic solution (total 600ml) was washed with brine and was dried over magnesium sulfate then concentrated to an oil (~10gm). This material was purified by chromatography using a Biotage cartridge (120gm of silica gel, eluting with 0-8% Methanol in dichloromethane). The product thus obtained as light oil (5.1 gm, 62%).

Example 21

The following scheme exemplifies the synthesis of a radiolabeled C14 intermediate useful for the preparation of certain compounds within the scope of the present invention.

**Scheme VI**

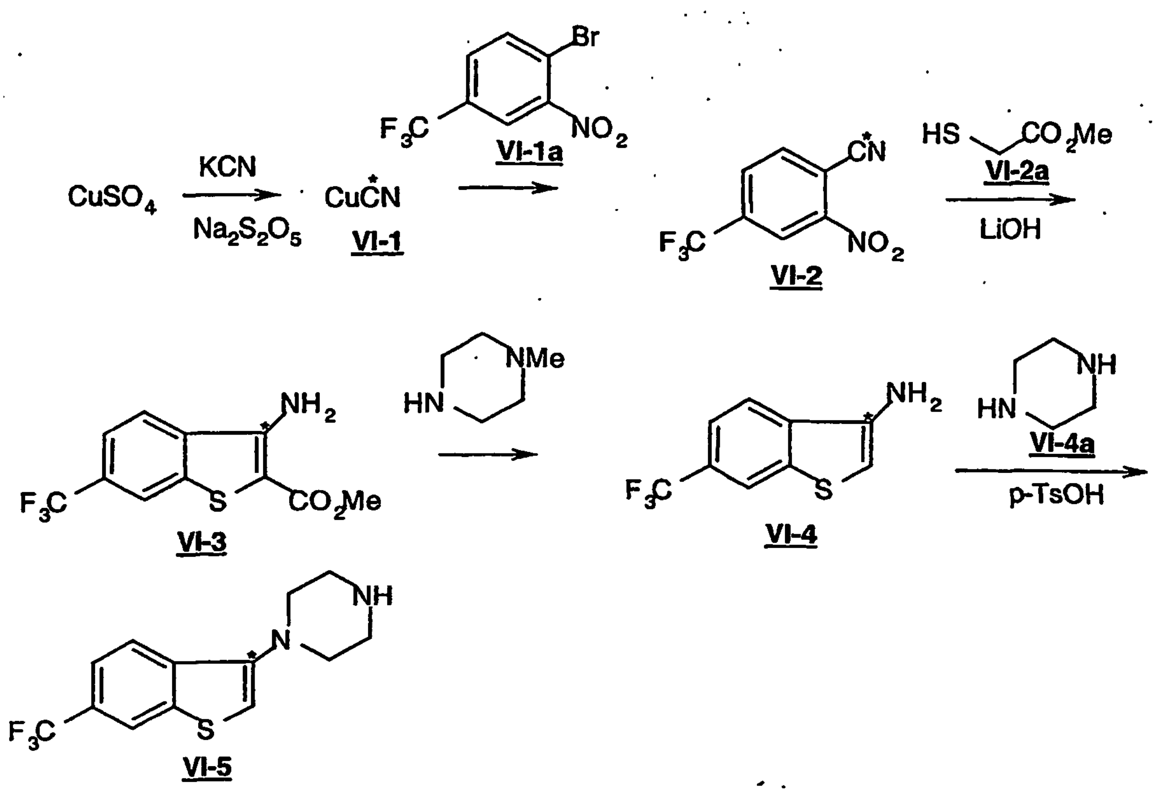

General: Analytical thin layer chromatography (TLC) was performed on E. Merck TLC plates with silica gel 60 $F_{254}$ (0.25 mm). TLC plates used in the analysis of radioactive samples were scanned on a BIOSCAN system 2000 Imaging Scanner using P-10 gas (10% methane, 90% argon). Identity of the intermediates was established by co-migration in radio-TLC and/or radio-HPLC with the standard samples of unlabeled analogues. Flash chromatography was performed using silica gel with a particle size of 40-63 μm. Specific activity was determined on a Packard Minaxi Tri-Carb Liquid Scintillation Analyzer (Model 1600 TR) using Bio-Safe II as scintillation cocktail.

Purification of compounds VI-2, VI-3, VI-4, VI-5, and VI-6 was monitored by HPLC (conditions:A) which was carried out on Waters 600 Controller, Waters 996 Photodiode Array Detector, Millennium Chromatography Manager and Beta-Ram Radioactive Flow Through Monitor System, Model 2 (IN/US Systems Inc.). Final purity determination of VI-7 by HPLC (conditions:B) was performed on Waters Model 510 Pumps, Waters 680 Gradient Controller, Waters 715 Ultra Wisp Autosampler, Waters 484 Tunable Absorbance Detector and Beta-Ram Radioactive Flow-Through Monitor System, Model 2 (IN/US Systems Inc.).

Conditions A: YMC Basic 5 μm, C18, 4.6x250 mm, mobile phase A: (v/v) 50/50 acetonitrile/0.1N ammonium formate, mobile phase B: (v/v) 75/25 acetonitrile/0.1N ammonium formate, flow rate 1.0 mL/min, uv detection at 254 nm.

| Gradient: | Time (minutes) | %MP:A | %MP:B |
|---|---|---|---|
| | 0 | 100 | 0 |
| | 15 | 100 | 0 |
| | 25 | 0 | 100 |
| | 30 | 0 | 100 |
| | 35 | 100 | 0 |

Conditions B: Ultremex 5 μm, C8, 4.6 x 150 mm, mobile phase (v/v/v) 50/50/0.25 - acetonitrile/0.05 M potassium phosphate buffer, pH 3.0/triethylamine, flow rate 1.0 mL/min, uv detection at 210 nm.

**[$^{14}$C] Copper (I) Cyanide (VI-1):**

A solution of copper (II) sulfate pentahydrate

(4.16 g, 16:67 mmol) in water (13.3 mL) was heated to 70 °C and a solution of sodium metabisulfite (1.94 g, 6.28 mmol) in water (3.3 mL) at 70 °C was added in one minute. Immediately a solution of [$^{14}$C] potassium cyanide (245.5 mg, 200 mCi, 3.77 mmol, S.A. 53.0 mCi/mmol) and unlabeled potassium cyanide (0.84g, 12.9 mmol) in water (3.3 mL) at 70 °C. was added in one minute. A white solid precipitated out of solution and blue color of the solution was discharged. After stirring for 10 min at 70 °C, the mixture was filtered hot and the solid was washed with hot water (15mL) and ethanol (15mL). The white solid was dried under vacuum (0.1 mm Hg) for 27 h 45 min to prove **VI-1** (1.393 g, 186.6 mCi) in 93.3% yield.

**2-Nitro-4-(trifluoromethyl)-[7-$^{14}$C]benzonitrile (VI-2):**

To a suspension of [$^{14}$C]copper (I) cyanide (**VI-1)** (1.393 g,15.55 mmol, 186.6 mCi) in 1-methyl-2-pyrrolidinone (NMP, 10mL) was added 4-bromo-3-nitrobenzotrifluoride (6.33 g, 23.45 mmol) and the mixture was heated at 190-195 °C for 1 h. Ethyl acetate (25mL) and water (20 mL) were added at room temperature and the mixture was filtered through celite. To the filtrate more water (20mL) and ethyl acetate (25mL) were added and the aqueous layer was extracted with ethyl acetate (90mL). The organic extract was washed with iron (III) chloride solution (50mL) prepared by dissolving iron (III) chloride (7.468 g, 46.04 mmol) in water (50 mL).The organic extract was further washed with water (30mL), sat. sodium chloride (15mL), dried ($Na_2$ $SO_4$) and the solvent was removed *in vacuo.*
The residue was purified by flash chromatography on silica gel (hexane/ethyl acetate, 9/1-7/3) to provide an oil which was dissolved in hexane (70 mL). The solvent was removed under reduced pressure and residue was dried under vacuum for 15 h 40 min to provide **VI-2** (3.01 g, 167.13 mCi, 89.6% yield) as a yellow solid. Radio-TLC (hexane/ethyl acetate, 9/1), $R_f$=0.21;HPLC (System A), RCP 99.86% (ret. time, 9.2 min).

**[3-$^{14}$C]-3-Amino-2-carbomethoxy-6-trifluoromethylbenzol[b]thiophene (VI-3):**

Nitrile **(VI-2)** (3.01g, 13.9 mmol, 167.13mCi) was dissolved in DMF (14mL) and methyl thioglycolate (1.78g, 15.94 mmol, 95%) was added in one minute. The mixture was cooled to 0-5 °C and a solution of lithium hydroxide (0.689 g, 28.77 mmol) in water (9.2mL) was added dropwise in 12 minutes. After the addition, cooling bath was removed and the mixture was stirred at room temperature for 4 hours. Water (70mL) was added at 0-5 °C and the mixture was stirred

for 15 min at 0-5 °C. the solid was collected on a filter, washed with water (20 mL) and dried under vacuum (0.1 mm Hg) for 40 h 15 min to provide **VI-3** (3.469 g, 151.24 mCi, 90.49% yield). Radio-TLC ($CH_2\ Cl_2$), $R_f$ = 0.372; HPLC (system A), RCP 99.92% (ret. time, 16.722 min).

**[3-$^{14}$C]-3-Amino-6-trifluoromethylbenzo[b]thiophene (VI-4):**

To a solution of benzo[*b*] thiophene **(VI-3)** (3.469 g, 12.6 mmol, 151.2 mCi) in NMP (14mL) was added 1-methylpiperazine (6.69g, 66.79 mmol) and the mixture was heated at 140-145 °C for 5 h. The mixture was allowed to cool to room temperature, poured into water (60mL) and extracted with ethyl acetate (140 mL). The organic extract was washed with water (30 mL), sat. sodium chloride (10mL), dried ($Na_2So_4$) and the solvent was removed in *vacuo.* The residue was purified by flash chromatography on silica gel (hexane/ethyl acetate, 1/1) to yield a greenish solid which was dried under vacuum (0.1 mm HG) for 14 h to provide **VI-4** (w.66g, 146.95 mCi, 97.16% yield). ). Radio-TLC (hexane/ethyl acetate, 1/5), $R_f$= 0.407; HPLC (system A), RCP 99.44 % (ret. time, 10.552 min).

**1-[6-(trifluoromethyl)benzo[b]thien-3-yl-[3-$^{14}$C]piperazine (VI-5):**

To a solution of benzo[b]thiophene **(VI-4)** (2.66 g, 12.24 mmol, 146.95 mCi) in NMP (17mL)was added piperazine (4.309g, 50.02 mmol) and p-toluenesulfonic acid (4.76g, 25.02 mmol) at room temperature. The mixture was heated at 170 °C for 20m h 24 min, allowed to cool to room temperature and poured into a solution of sodium carbonate (4.70 g, 44.3 mmol) in water (60mL). The mixture was extracted with ethyl acetate (20 mL), dried ($Na_2SO_4$) and the solvent was removed in vacuo. The residue was purified by flash chromatography on silica gel ($CH_2Cl_2/MeOH/NH_4OH$, 9/1/0.2) and product was dried under vacuum (0.1 mm Hg) for 11 h 50 min. Ethanol (absolute, 30 mL) was added to the product and solvent was removed under reduced pressure. The residue was dried under vacuum (0.1 mm Hg) for 24 h 55 min to provide **VI-5** (3.44 g, 144.18 mCi, 98.1 % yield) as an oil. Radio-TLC ($CH_2Cl_2/MeOH/NH_4OH$, 9/1/0.2), $R_f$=0.46; HPLC (system A), RCP 99.88% (ret. time, 5.807 min).

**Example 22**

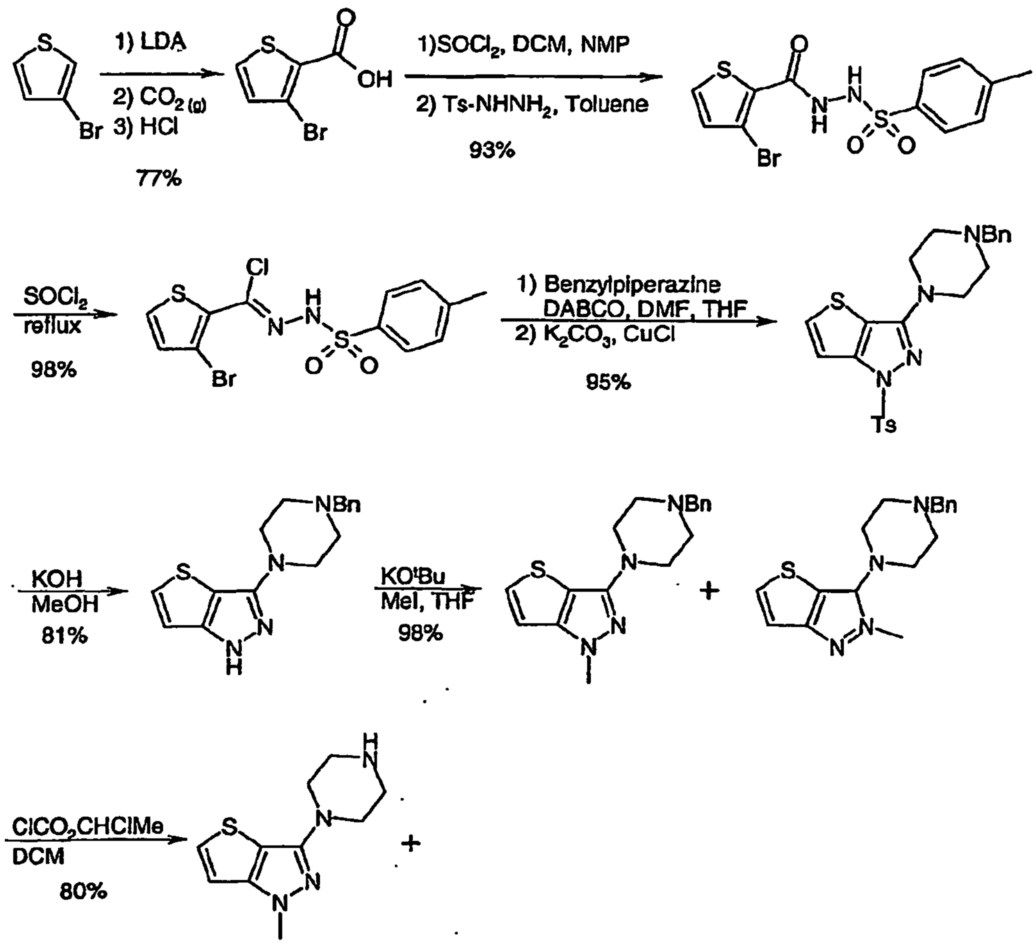


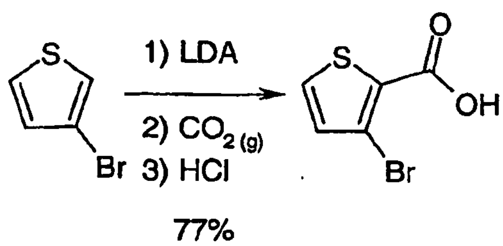


**3-Bromo-thiophene-2-carboxylic acid.** To a solution of 3-bromothiophene (600.0 g, 3.68 mol) in THF (3 L) cooled to -72 °C was added LDA (1.93 L, 3.86 mol, 2 N) slowly over 2 hours. The rate of LDA addition is such that the reaction temperature never exceeded -68 °C. After complete addition, the solution is stirred for an additional 40 minutes. Diethyl ether (3 L) is then added via an addition funnel such that the temperature is maintained below -65 °C. The addition funnel is then replaced with a dispersion tube and $CO_2$ gas is bubbled through the solution for 3 hours. Dry ice (500 g) is then added and the mixture is stirred overnight. The reaction flask is then placed in an ice bath and 6 N HCl is added slowly to prevent excessive bubbling until the pH of the solution is adjusted to 1-2. The resulting mixture is then extracted with EtOAc. The extract is washed with brine then dried over $MgSO_4$, filtered and evaporated. The product is dried under vacuum at room temperature yielding 585.15 g (77%) as an off-white solid.

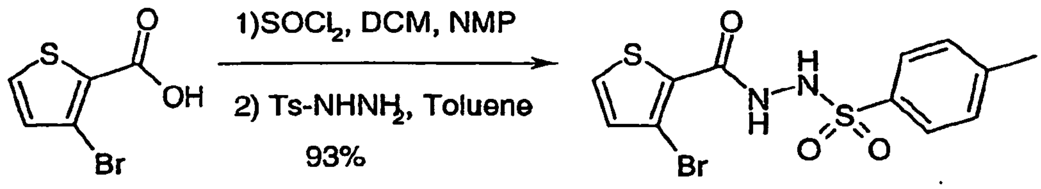


**1-(3-Bromo-thiophene-2-carboxylic acid)-2-(4-toluenesulfonyl)-hydrazine.** To a stirred suspension of the acid (285.53 g, 1.38 mol) in DCM (1.5 L) was added a catalytic amount of NMP (2 mL). Thionyl chloride (105.8 mL, 1.45 mol) is then added and the solution is refluxed until the solids have completely dissolved. The solution is further refluxed for 1 hour, cooled to room temperature and evaporated to afford a light, brown solid. The crude material is dried under vacuum overnight. The brown solid is taken up in toluene (3.5 L) and *p*-toluenesulfonhydrazine (402.25 g, 2.16 mol) is added. The mixture is stirred at 100 ˚C for 8 hours then at room temperature overnight. The resulting mixture was cooled with an ice bath and the resulting solids were collected by filtration and washed with toluene. The solids were then stirred as a slurry in 1 N HCl for 1 hour. The solids were collected by filtration and washed with copious amounts of water. The solid were dried under vacuum at 40 ˚C then recrystallized from toluene / isoproyl alcohol yielding 484.28 g (93%) of the desired product.

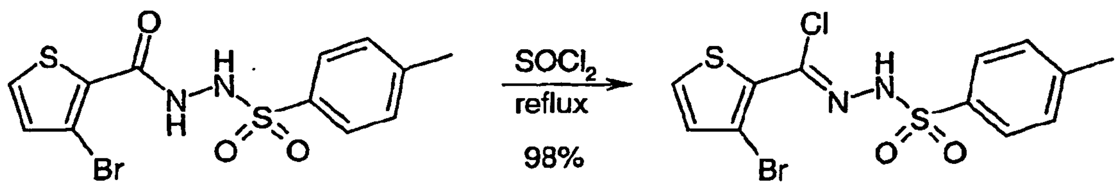


**N-((4-Methylphenyl)-sulfonyl)-3-bromo-thiophene-2-carbohydrazonyl chloride.** 1-(3-Bromo-thiophene-2-carboxylic acid)-2-(4-toluenesulfonyl)-hydrazine (60.80 g, 0.161 mol) was added to thionyl chloride (70.5 mL, 0.966 mol). The resulting mixture was stirred at 80 ˚C until the mixture becomes homogenous. The solution is then stirred at 70 ˚C for 30 minutes and heptane (300 mL) is added over a period of 20 minutes. The solution was cooled slowly to room temperature then cooled further to 5 ˚C. The solids are collected by filtration, washed with heptane (3 x 100 mL) and dried under vacuum yielding 62.1 g (98%) of the desired product as an off-white solid.

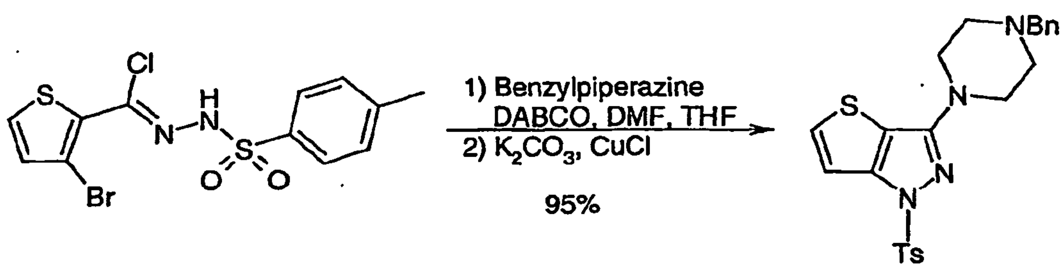


**3-(4-Benzyl-piperazin-1-yl)-1-(toluene-4-sulfonyl)-1*H*-thieno[3,2-*c*]pyrazole.** To a stirred solution of DABCO (14.18 g, 112.18 mol) and benzylpiperazine (35.35 g, 0.200 mol) in DMF (200 mL) cooled to -30 ˚C was added via cannula a solution of N-((4-Methylphenyl)-sulfonyl)-3-bromo-thiophene-2-carbohydrazonyl chloride (62.1 g, 0.158mol) in THF (100 mL). The addition is controlled to prevent the reaction temperature from exceeding -30 ˚C. After complete addition precipitation occurs and the mixture is then allowed to stir at room temperature overnight when $K_2CO_3$ (65.41 g, 0.473 mol) and CuCl (1.0 g, 0.010 mol) was added. The resulting mixture is heated to 110 ˚C and the THF is removed by distillation at this point. The temperature is then increased to 140 ˚C and the mixture is stirred for 6 hours, cooled to room temperature and stirred overnight. The mixture was then poured over water (100 mL) and EtOAc (100 mL). The EtOAC layer is then separated and the aqueous layer is extracted with EtOAC (3 x 500 mL). The combined EtOAC layers were washed with water (500 mL) and then filtered through celite and concentrated. The solids were collected by filtration and washed with cold water then EtOAc / heptane (1:4) and dried under vacuum yielding 66.05 g (95%) of the desired product as an off-white solid.

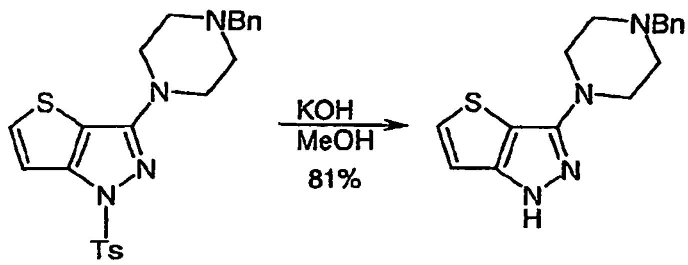


**3-(4-Benzyl-piperazin-1-yl)-1*H*-thieno[3,2-*c*]pyrazole.** To a stirred mixture of $KOH_{(s)}$ (56.09 g, 2.66 mol) in methly alcohol (1.33 L) is added 3-(4-benzyl-piperazin-1-yl)-1-(toluene-4-sulfonyl)-1*H*-thieno[3,2-*c*]pyrazole (241 g, 0.532 mol). The mixture is heated at reflux for 1.25 hours, cooled to room temperature and evaporated. The residue is taked up in EtOAc (1 L) washed with water (2 L), dried ($MgSO_4$) filtered and evaporated. The residue was recrystallized from EtOAc/ Heptane yielding 129 g (81%).

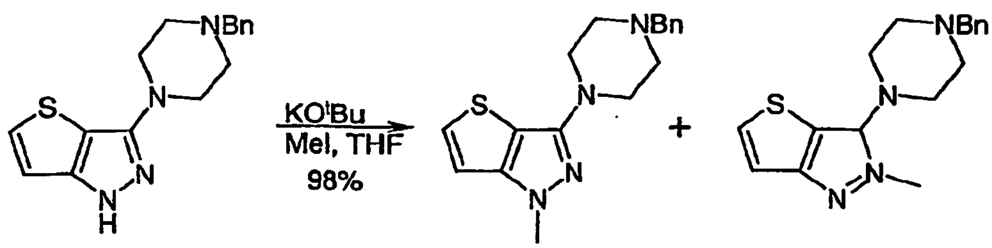


**3-(4-Benzyl-piperazin-1-yl)-1-methyl-1*H*-thieno[3,2-*c*]pyrazole. To a stirred** solution of 3-(4-benzyl-piperazin-1-yl)-1*H*-thieno[3,2-*c*]pyrazole (318.0 g, 1.07 mol) in THF (2.5 L) was added a mixture of potassium t-butoxide (134.4 g, 1.2 mol) in THF (1.5 L) dropwise over a period of 1 hour while keeping the reaction temperature below 25 ˚C. After complete addition, the mixture was cooled to -30 ˚C and MeI (65.4 mL, 1.05 mol) was added dropwise over a period of 30 minutes. The mixture is then slowly warmed to room temperature overnight. To the reaction mixture is slowly added saturated $NaHCO_3$ (1 L). The solution is then evaporated to remove the THF and the resulting aqueous mixture is taken up in EtOAc and washed with water and brine. The EtOAc extract is dried ($Na_2SO_4$), filtered and evaporated. The viscous concentrate is filtered through a silica gel plug with 1:1 EtOAc/heptane and evaporated yielding a viscous oil that is then dried under vacuum where it solidifies and yields 326.03 g (98%) as a 12:1 ratio of regioisomers in favor of the desired product.

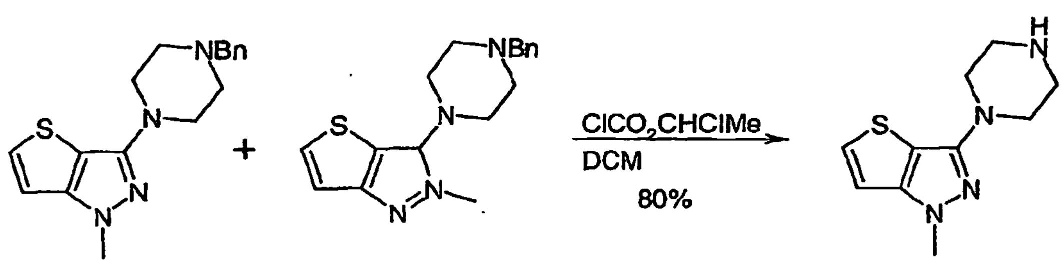


**1-Methyl-3-piperazin-1-yl-1*H*-thieno[3,2-*c*]pyrazole.** To a solution of a mixture of 3-(4-Benzyl-piperazin-1-yl)-1-methyl-1*H*-thieno[3,2-*c*]pyrazole and the 2-methyl analog (189.0 g, 0.60 mol) is dissolved in DCM (1.25 L) is added 1-chloroethylchloroformate (78.6 mL, 0.72 mol). The solution is heated at reflux for 1 hour when the mixture is cooled and the solvent is removed by evaporation. The residue is taken up in methanol (1L) and heated at reflux for 30 minutes. After cooling, the solution is treated with 1 N HCl in ether (200 mL) and an additional 1 L of ether to afford the precipitation of the product. The solid is collected via filtration and washed with cold ether. The solid is recrystallized from methanol (1 L) and the HCl salt is collected by filtration, washed with ether and dried under vacuum yielding 123.04 g (80%) of the desired product as an 80:1 mixture of regioisomers in favor of the desired regioisomer as seen by NMR.

Example 23

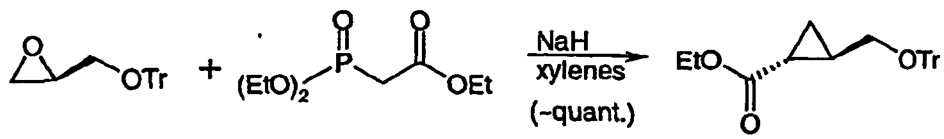


**Trityloxymethyl-(1R, 2R)-cyclopropanecarboxylic acid ethyl ester.** To a suspension of sodium hydride (15.20 g, 380 mmol, 60% oil dispersion) in xylenes (300 mL) was added triethylphosphonoacetate (85.07 g, 379 mmol) in a controlled manner to avoid the excessive evolution of gas and to maintain the internal temperature less than 55 ˚C. After the complete addition, the mixture was stirred for 20 minutes when the yellow solution was added via cannula to a solution of (R)-trityl glycidyl ether (100.0 g, 316 mmol) in xylenes (300 mL). The resulting solution was heated to 125 ˚C for 2 hours. The resulting solution was cooled to room temperature, acidified with the addition of 10% HCl (320 mL) and extracted with EtOAc (2 x 300 mL). The combined extracts were washed with brine (100 mL), dried ($MgSO_4$), filtered and evaporated yielding a 175 g of a crude product as an oil. The material was carried on crude.

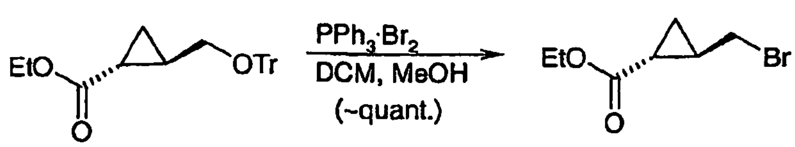


**2R-bromomethyl-cyclopropane-1R-carboxylic acid methyl ester.** A solution of triphenylphosphine (124.7 g, 1.34 mol) in $CH_2Cl_2$ (260 mL) was cooled to 5 ˚C when a solution of bromine (24.4 mL, 1.34 mol) in $CH_2Cl_2$ (65 mL) was added over 20 minutes while the temperature was maintained below 12 ˚C. The mixture was stirred at 5 ˚C for 1 hour when 2 M HCl/$Et_2O$ (16 mL, 32 mmol) was added followed by the addition of crude trityloxymethyl-(1R , 2R)-cyclopropane carboxylic acid ethyl ester (124 g, 0.32 mol). The resulting mixture was stirred at room temperature overnight when saturated $NaHCO_3$ (600 mL) was added. The mixture was separated and the aqueous layer was extracted with $CH_2Cl_2$ (200 mL). The combined organic layers were washed with water (400 mL), dried ($MgSO_4$), filtered and evaporated. The residue was diluted with heptane (200 mL) and evaporated two times to remove excess $CH_2Cl_2$. The residue was allowed to stand for 30 minutes when the solid: Impurities were removed by filtration. The filter cake was washed with heptane (2 x 400 mL). The combined organic layers were evaporated to provide 92.68 g of a crude yellow liquid. The crude liquid was distilled (BP = 80-85 ˚C/1.5 torr) to provide 55.19 g (84% yield for the two steps) of a colorless liquid.

Example 24

**7-Trifluoromethyl benzo[b]thienyl piperidine**

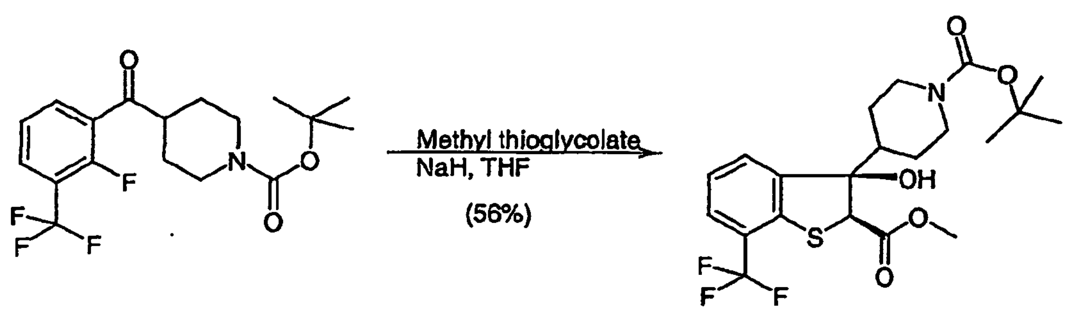


**4-(3-Hydroxy-2-methoxycarbonyl-7-trifluoromethyl-2,3-dihydrobenzo[*b*]thiophen-3-yl)-piperidine-1-**

**carboxylic acid *tert*-butyl ester (MDL 832712).** To a room temperature solution of 4-(2-fluoro-3-trifluromethyl-benzoyl)-piperidine-1-carboxylic acid tert-butyl ester (9.00 g, 24.0 mmol), methyl thioglycolate (2.40 mL, 26.8 mmol), and anhydrous THF (200 mL) under nitrogen was added NaH (1.15 g of a 60% oil dispersion, 28.7 mmol) in one portion. After the gas evolution ceased, the reaction was stirred at 55 ˚C. After 100 min, the reaction was cooled to room temperature and diluted with ethyl acetate (500 mL). The mixture was washed with water (300 mL) and brine (300 mL) successively; dried over magnesium sulfate, filtered, and the solvent removed to afford a sticky white solid. Trituration with 20% ethyl acetate/heptane afforded 6.20 g (56%) of the desired product as a white powder.

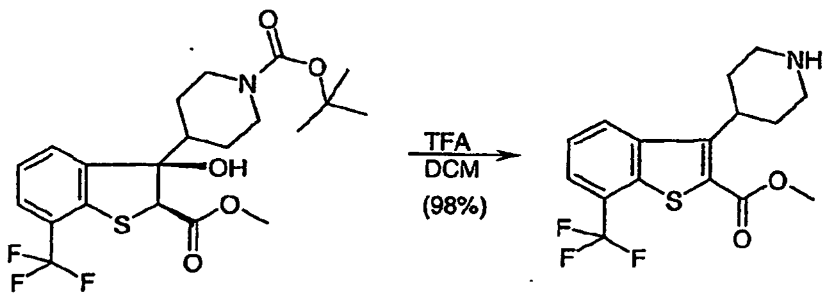


**3-Piperidin-4-yl-7-trifluoromethyl-benzo[*b*]thiophene-2-carboxylic acid methyl ester.** To a room temperature solution of 4-(3-hydroxy-2-methoxycarbonyl-7-trifluoromethyl-2,3-dihydro-benzo[*b*]thiophen-3-yl)-piperidine-1-carboxylic acid *tert*-butyl ester (6:00 g, 13.0 mmol) in DCM:(30 mL) was added TFA (30 mL) causing rapid gas evolution. After 5 min, the reaction was stirred at 40 ˚C for 5.5 h. After cooling to room temperature, the reaction was poured into 20% aqueous potassium carbonate (400 mL) and extracted with DCM (2 x 200 mL). The combined extracts were dried over magnesium sulfate, filtered, and the solvent removed to give a thick oil. After drying under high vacuum 4.37 g (98%) of the desired product was obtained as a white foam.

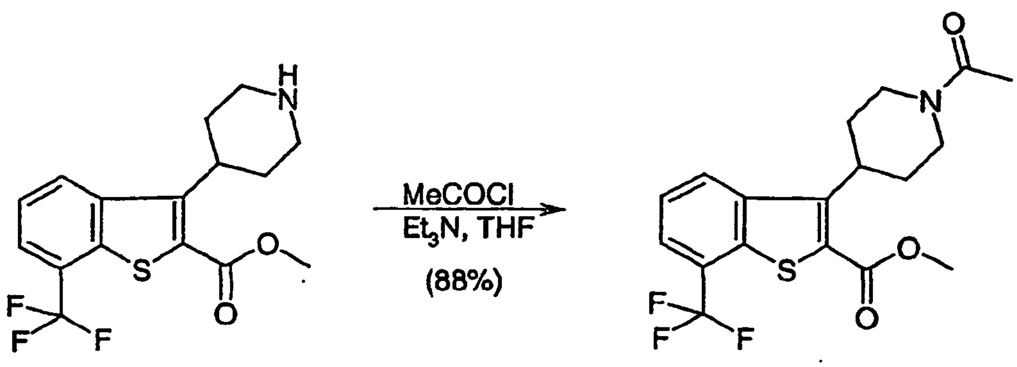


**3-(1-Acetyl-piperidin-4-yl)-7-trifluoromethyl-benzo[*b*]thiophene-2-carboxylic acid methyl ester.** To a room temperature solution of 3-piperidin-4-yl-7-trifluoromethyl-benzo[*b*]thiophene-2-carboxylic acid methyl ester (4.37 g, 12.7 mmol), triethylamine (2.70 mL.19.4 mmol), and anhydrous THF (80 mL) under nitrogen was added acetyl chloride (1.10 mL, 15.5 mmol) in one portion and the reaction stirred at room temperature overnight. The reaction was diluted with ethyl acetate (300 mL) and washed with water (150 mL) and brine (150 mL) successively. The organic layer was dried over magnesium sulfate, filtered, and the solvent removed. The residue was chromatographed on silica, eluting with 10% methanol/ethyl acetate, to afford 4.28 g (88%) of the desired product as a white solid, mp: 155.2 ˚C.

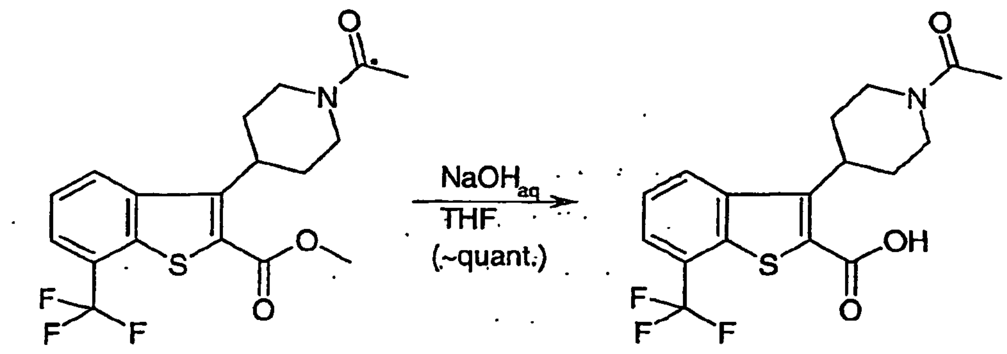


**3-(1-Acetyl-piperidin-4-yl)-7-trifluoromethyl-benzo[*b*]thiophene-2-carboxylic acid.** To a solution of 3-(1-acetyl-piperidin-4-yl)-7-trifluoromethyl-benzo[*b*]thiophene-2-carboxylic acid methyl ester (4.10 g, 10.6 mmol) in THF (25 mL) was added 0.5 N aqueous sodium hydroxide (23.4 mL, 11.7 mmol) and the reaction stirred at room temperature. After 18 h, the reaction was acidified with 1 N HCl (200mL) and the mixture extracted with DCM (2 x 100 mL). The organics were washed with water (100 mL), dried over magnesium sulfate, filtered, and concentrated to give 4.13 g of the desired product as a white foam.

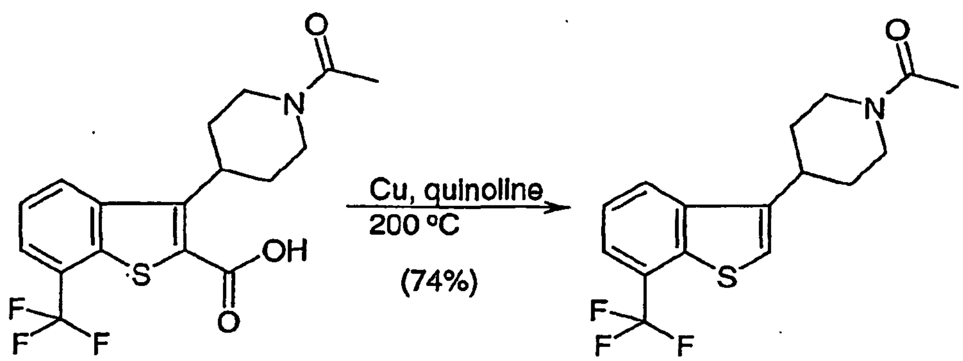


**1-[4-(7-Trifluoromethyl-benzo[*b*]thiophen-3-yl)-piperidin-1-yl]-ethanone (MDL 832823).** A mixture of 3-(1-acetyl-piperidin-4-yl)-7-trifluoromethyl-benzo[*b*]thiophene-2-carboxylic acid (4.13 g, 11.1 mmol), Cu powder (0.706 g, 11.1 mmol), and quinoline (20 mL) was heated to 200 °C under nitrogen. After 10 min, no gas evolution was observed and the reaction cooled at room temperature. The mixture was diluted with ethyl acetate (100 mL), filtered through a Celite bed and the filtrate washed with 1 N HCl (2 x 100 mL), 5% aqueous potassium carbonate (100 mL), water (100 mL), and brine (100 mL) successively. The organics were dried over magnesium sulfate, filtered, and concentrated to give an amber oil. The oil was chromatographed on silica, eluting with 10% methanol/ethyl acetate to afford 2.69 g (74%) of the desired product as a tan solid.

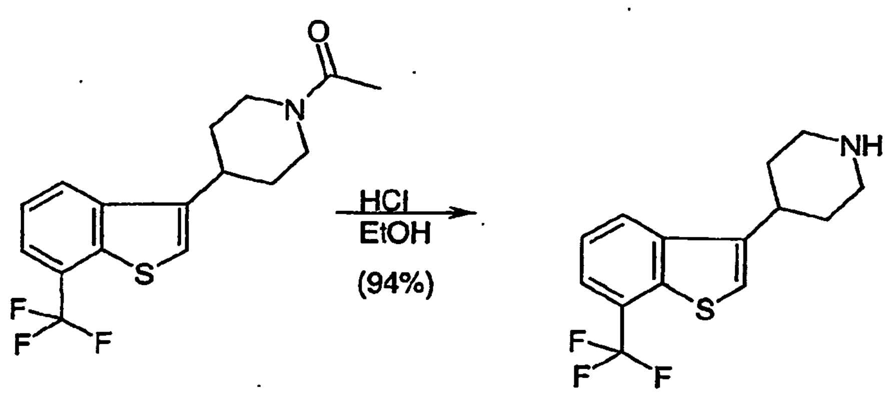

**4-(7-Trifluoromethyl-benzo[*b*]thiophen-3-yl)-piperidine.** A mixture of 1-[4-(7-trifluoromethyl-benzo[*b*]thiophen-3-yl)-piperidin-1-yl]-ethanone (2.95 g, 9.01 mmol), concentrated HCl (30 mL), and ethanol was heated at 80 ˚C for 18 h. After cooling to room temperature, the reaction was basified with 20% aqueous potassium carbonate (150 mL) and the mixture extracted with DCM (2 x 100 mL). The organics were washed with water (100 mL), dried over potassium carbonate, filtered, and concentrated to give 2.42 g (94%) the desired product as an amber waxy solid.

Example 25

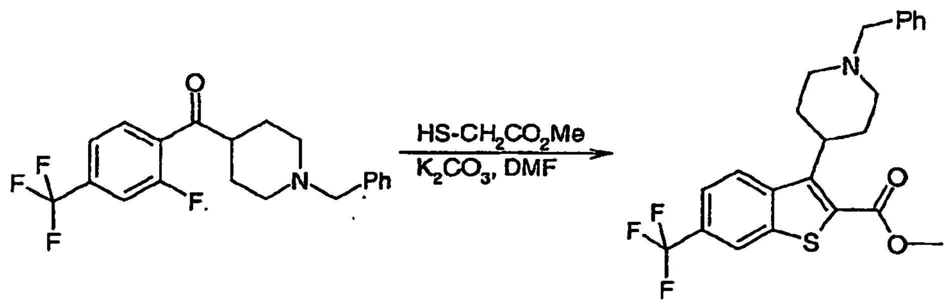


**3-(1-Benzyl-piperidin-4-yl)-6-trifluoromethyl-benzo[*b*]thiophene-2-carboxylic acid methyl ester (MDL 833803).** To a room temperature solution of (1-benzyl-piperidin-4-yl)-(2-fluoro-4-trifluoromethyl-phenyl)-methanone (7.5 g, 20.5 mmol), methyl thioglycolate (2.0 mL, 22.5 mmol), and DMF (100 mL) was added $K_2CO_3$ (5.65 g, 41.0 mmol). The reaction was stirred at 60 ˚C for 24 hours, cooled to room temperature and diluted with ethyl acetate (500 mL). The mixture was washed with water (2 x 300 mL) and brine (300 mL) successively, dried over magnesium sulfate, filtered, and the solvent removed to afford an oil. The oil was purified via chromatography (30% EtOAc in heptane) yielding 5.91 g (67%) as a solid.

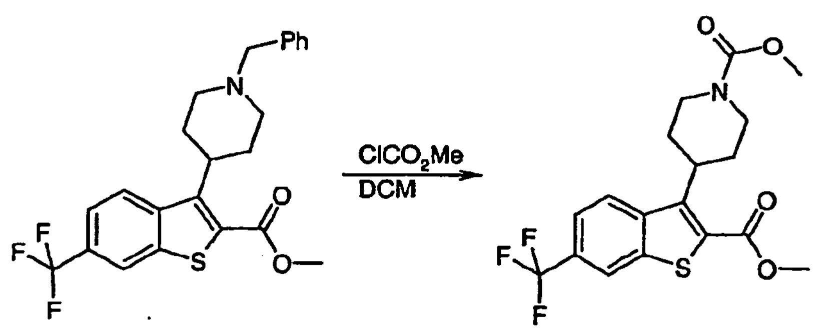


**4-(2-Methoxycarbonyl-6-trifluoromethyl-benzo[b]thiophen-3-yl)-piperidine-1-carboxylic acid methyl ester.** To a solution of 3-(1-benzyl-piperidin-4-yl)-6-trifluoromethyl-benzo[*b*]thiophene-2-carboxylic acid methyl ester (5.9 g, 13.6 mmol) in DCM (50 mL) was added methyl chloroformate (1.26 mL, 16.3 mmol) drop-wise. The resulting solution was stirred overnight when the volatiles were removed *in vacuo.* The residue was washed with heptane to yield 4.2 g (77%) of the desired product as a white solid.

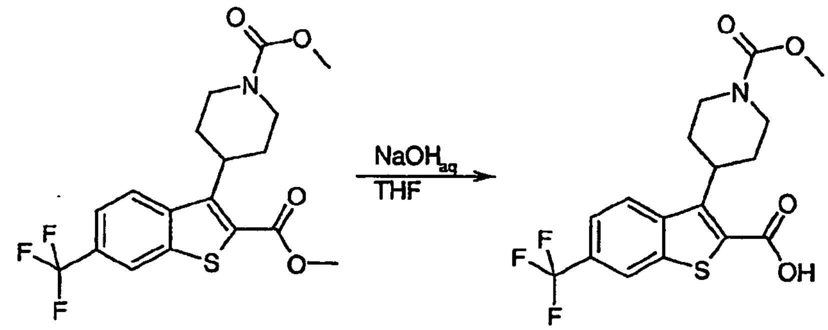


**4-(2-Carboxy-6-trifluoromethyl-benzo[b]thiophen-3-yl)-piperidine-1-carboxylic acid methyl ester.** To a stirred solution of 4-(2-Methoxycarbonyl-6-trifluoromethyl-benzo[b]thiophen-3-yl)-piperidine-1-carboxylic acid methyl ester (1.1 g, 2.7 mmol) in THF (7.0 mL) was added 1 N NaOH (2.97 mL). The resulting mixture was stirred at room temperature overnight when the mixture was diluted with water (50 mL) and washed with ether (100 mL). The aqueous layer was acidified with the addition of 3 N HCl and the product was extracted with EtOAc (2 x 150 mL). The combined organic layers were washed with brine (50 mL), dried ($MgSO_4$), filtered and evaporated yielding 960 mg (92%) of the desired product as a white solid.

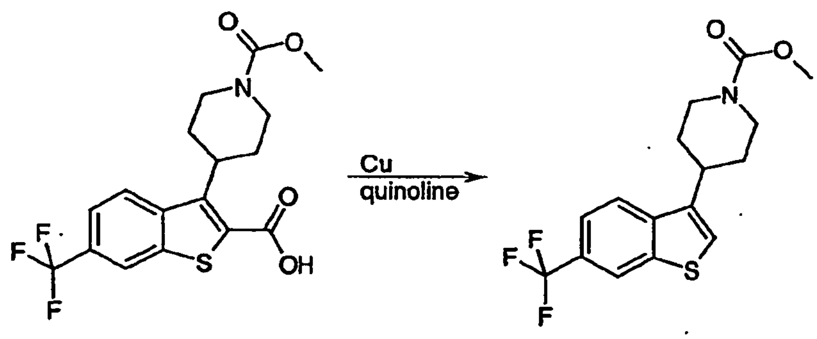


**4-(6-Trifluoromethyl-benzo[b]thiophen-3-yl)-piperidine-1-carboxylic acid methyl ester.** A mixture of 4-(2-carboxy-6-trifluoromethyl-benzo[b]thiophen-3-yl)-piperidine-1-carboxylic acid methyl ester (4.3 g, 11.1 mmol) and copper (705 mg, 11.1 mmol) in quinoline (28 mL) was heated at 200 ˚C for 45 minutes. Upon cooling to room temperature the mixture was diluted with EtOAc (50 mL) and filtered. The filtrate was washed with 5% HCl (2 x 20 mL), water (20 mL) and brine (20 mL), dried ($MgSO_4$), filtered and evaporated. The residue was separated via chromatography (30% EtOAc in heptane) yielding 3.14 g (82%) of the desired product as a white solid.

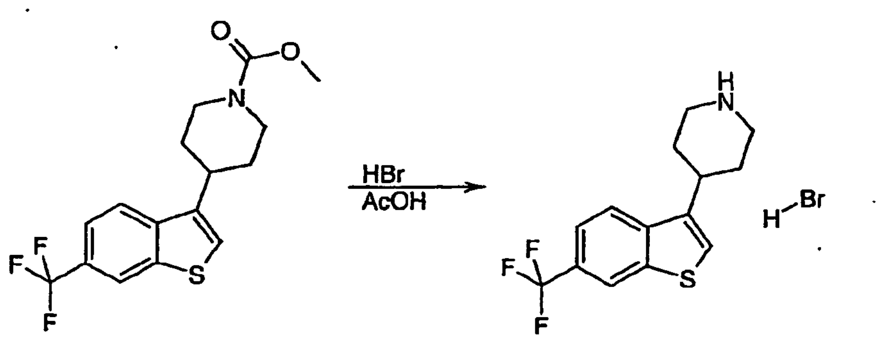


**4-(6-Trifluoromethyl-benzo[*b*]thiophen-3-yl)-piperidine hydrobromide.** A mixture of 4-(6-trifluoromethyl-benzo[b]thiophen-3-yl)-piperidine-1-carboxylic acid methyl ester (3.1 g, 9.0 mmol) in HBr (45 mL, 30% in acetic acid) was stirred at room temperature for 20 hours when the volatiles were removed *in vacuo.* The residue was washed with EtOAc and the product was collected by filtration yielding 3.09 g (94%) of the desired product as a white solid.

Example 26

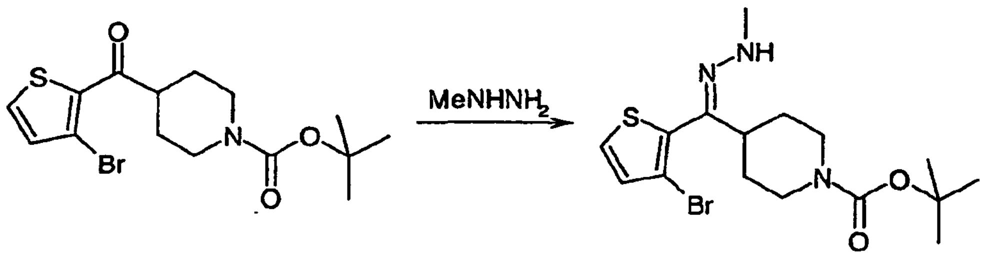


**4-[(3-Bromo-thiophen-2-yl)-(methyl-hydrazono)- methyl]-piperidine-1-carboxylic acid *tert*-butyl ester.** A mixture of 4-(thiophene-2-carbonyl)-piperidine-1-carboxylic acid *tert*-butyl ester (1.96g, 5.2 mmol) in methylhydrazine (2 mL) was heated at 75 ˚C overnight. The excess methyl hydrazine was then removed with a vacuum pump. The residue was purified by chromatography (eluted with 0-8% of MeOH in DCM) yielding 0.95 g (45%) of the desired product.

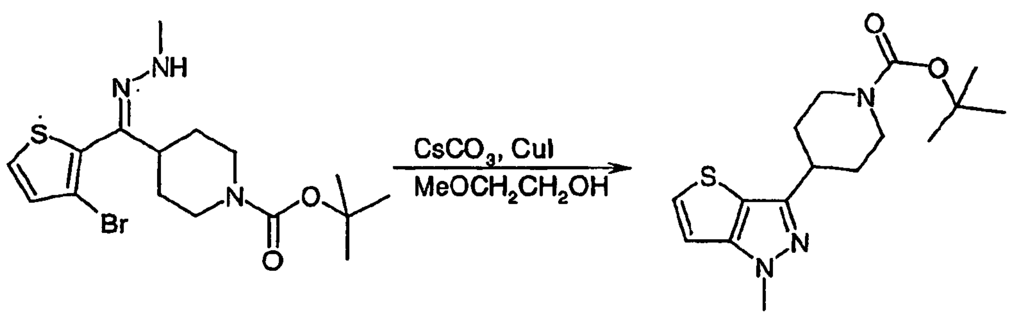


**4-(1-Methyl-1H-thieno[3,2-c]pyrazol-3-yl)-piperidine-1-carboxylic acid *tert*-butyl ester.** 4-[(3-Bromo-thiophen-2-yl)-(methyl-hydrazono)-methyl]-piperidine-1-carboxylic acid *tert*-butyl ester (700 mg, 1.74 mmol) was mixed with CuI (20 mg), $CsCO_3$ (650 mg, 1.15 eq) in methoxyethanol (10 mL). The mixture was heated to 70 ˚C for 2 hr. then stirred overnight at room temperature. The solvent was stripped on rotary evaporator. The residue was extracted into EtOAc then washed with brine and concentrated down to an oil. This oil was purified via chromatography (eluted with 0-10% MeOH in DCM) yielding 520 mg (68%) of the desired product.

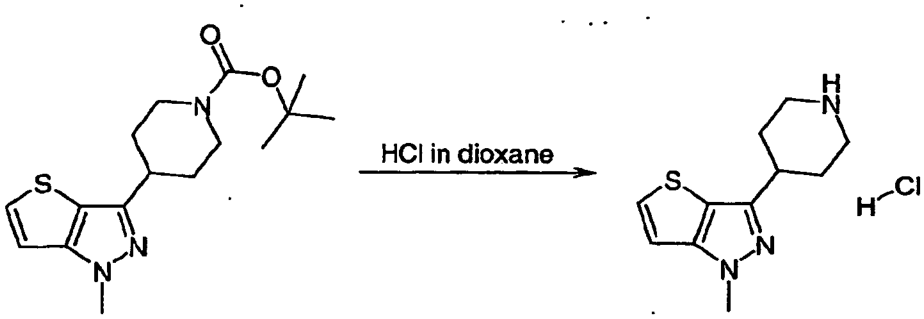


**1-Methyl-3-piperidin-4-yl-1*H*-thieno[3,2-*c*]pyrazole hydrochloride (A002436287A).** 4-(1-Methyl-1H-thieno[3,2-c]pyrazol-3-yl)-piperidine-1-carboxylic acid *tert*-butyl ester (520 mg, 1.6 mmol) was stirred at room temperature in a solution of HCl (5 mL, 4N HCl in dioxane) for 4hours. The volatiles were removed *in vacuo* and the residue was triturated with ether (twice) to yield off white solids 304 mg (74%) as the desired hydrochloride salt.

**Example 27**

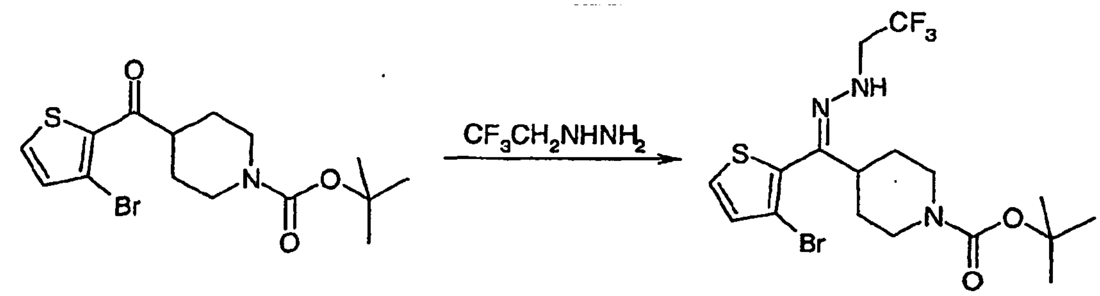


**4-{(3-Bromo-thiophen-2-yl)-[(2,2,2-trifluoro-ethyl)-hydrazono]-methyl}-piperidine-1-carboxylic acid *tert*-butyl ester.** To a mixture of 4-(thiophene-2-carbonyl)-piperidine-1-carboxylic acid *tert*-butyl ester (2.34 g, 6.24 mmol) in n-butanol (20 mL) was added trifluoroethylhydrazine (2.43 g, 12.4 mmol). The resulting mixture was heated at 110 ˚C overnight. The volatiles were then removed *in vacuo.* The residue was purified by chromatography (eluted with 0-10% MeOH in DCM) yielding 2.41 g (92%) of the desired product.

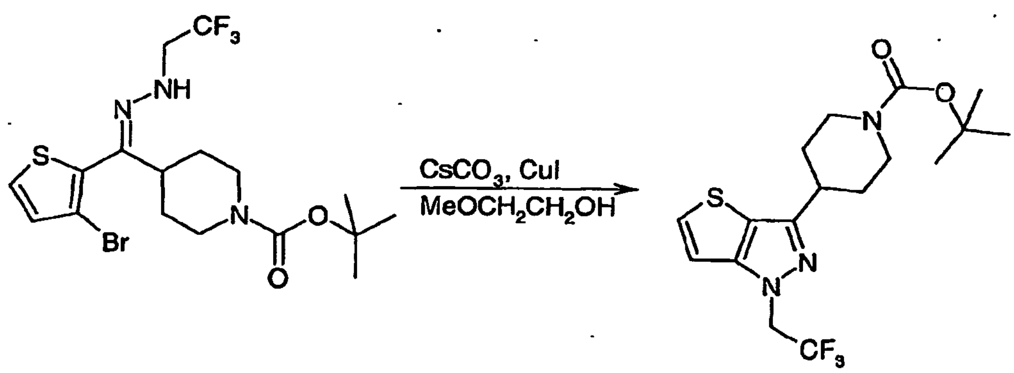


**4-[1-(2,2,2-Trifluoro-ethyl)-1*H*-thieno[3,2-c]pyrazol-yl]-piperidine-1-carboxylic acid *tert*-butyl ester.** 4-{(3-Bromo-thiophen-2-yl)-[(2,2,2-trifluoro-ethyl)-hydrazono]-methyl}-piperidine-1-carboxylic acid *tert*-butyl ester (2.34 g, 4.98 mmol) was mixed with CuI (50 mg), $CsCO_3$ (1.9 g, 1.2 eq) in methoxyethanol (25 mL). The mixture was heated to 75˚C for 1 hour. The mixture was then diluted with EtOAc and filtered. The filtrate was evaporated and the residue was purified via chromatography (eluted with 0-10% MeOH in DCM) yielding 2.03 g (>95%) of the desired product.

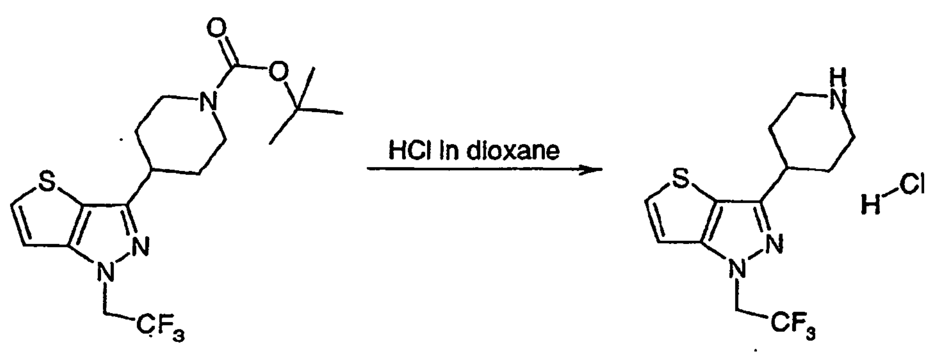


**3-Piperidin-4-yl-1-(2,2,2-trifluoro-ethyl)-1*H*-thieno[3,2-*c*]pyrazole hydrochloride (833906).** 4-[1-(2,2,2-Trifluoro-ethyl)-1*H*-thieno[3,2-*c*]pyrazol-3-yl]-piperidine-1-carboxylic acid *tert*-butyl ester (1.9 g, 4.87 mmol) was stirred at room temperature in a solution of HCl (6 mL, 4N HCl in dioxane) for 4hours. The volatiles were removed *in vacuo* and the residue was triturated with ether (twice) to yield off white solids 2.1 g (74%) as the desired hydrochloride salt.

Example 28

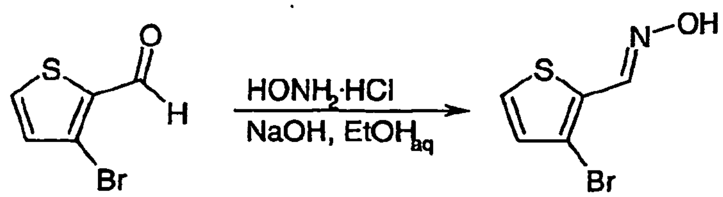


**3-Bromo-thiophene-2-carbaldehyde oxime**. 3-Bromothiophene-2-carbaldehyde (28.7 g, 0.15 mol) in ethanol (50 mL) was added in one portion to a solution of hydroxylamine hydrochloride (13.8 g, 0.2 mol), sodium hydroxide (8 g, 0.2 mol) in water (30 mL) and ethanol (100 mL). The mixture was stirred at 0 °C for 2 hours and was kept at 0 °C overnight when a precipitate formed. The mixture was diluted with cold water (600 ml) and the solid was collected by filtration yielding 20.5 g, (67%). The aqueous solution was further extracted with ethyl acetate. The organic solution was washed with brine, dried with magnesium sulfate, filtered and evaporated yielding 6.9 g of additional product as a light yellow solid. The total yield was 27.4 g (89%).

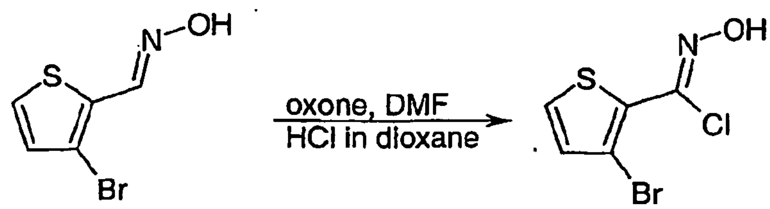


**3-Bromo-thiophene-2-(chloro-carbaldehyde) oxime.** To the solution of 3-bromothiophene-2-carbaldehyde oxime (10.8 g, 52.4 mmol), hydrogen chloride (14.5 mL, 4M in dioxane) in DMF (100 mL) was charged with *oxone* (16.9 g, 1.05 eqiv) in one portion at room temperature. The mixture was stirred at room temp overnight when the solution was poured in to water and extracted with ethyl acetate. The organic solution was washed with brine and dried over magnesium sulfate, filtered and evaporated to dryness to give a yellow solid (12.68 g, quantitative by weight) which was used in the next reaction without further purification.

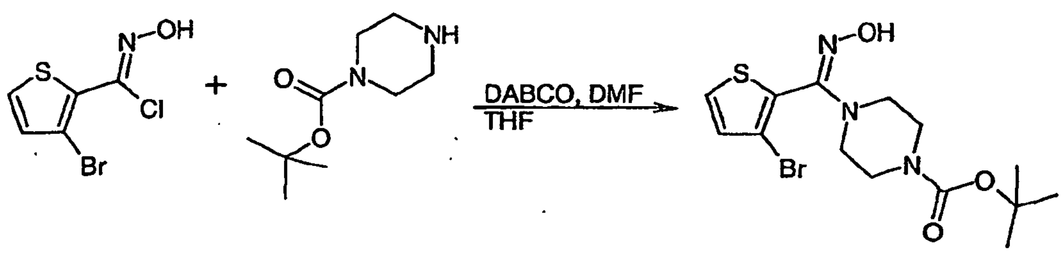


**4-[(3-Bromo-thiophen-2-yl)-hydroxyimino-methyl]-piperazine)-1-carboxylic acid *tert*-butyl ester.** A solution of 3-bromo-thiophene-2-(chloro-carbaldehyde) oxime (16.4 g, 68 mmol) in THF (70 mL) was added drop-wise to a solution of N-(t-butoxycarbonyl)piperazine (14 g, 1.1 equiv.), DABCO (9.5 g, 1.25 eqiv.) in DMF (100 mL) at 0 °C over 25 minutes. The mixture was stirred at 0 °C for 3.5 hours when the mixture was poured into water and was extracted with ethyl acetate. The organic layer was washed with brine and dried over magnesium sulfate, filtered and evaporated. The crude product (30.5 g) was purified via chromatography (eluted with 0-5 % of MeOH in DCM) yielding 24.6 g (85%) of the desired product.

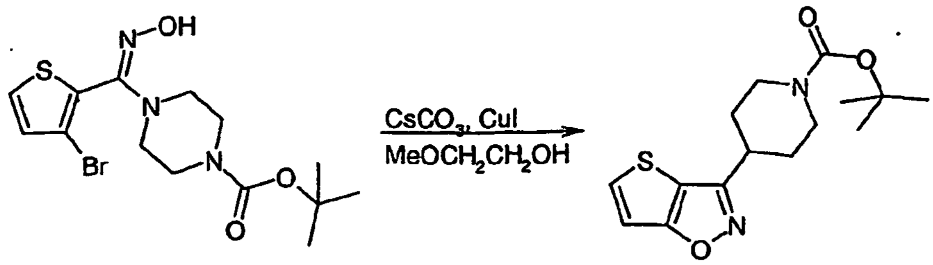


**4-Thieno[2,3-*d*]isoxazol-3-yl-piperazine-1-carboxylic acid *tert*-butyl ester.** A mixture of 4-[(3-bromo-thiophen-2-yl)-hydroxyimino-methyl]-piperazine)-1-carboxylic acid *tert*-butyl ester (10.3 g, 26.4 mmol), cesium carbonate (10.7 g, 32.7 mmol), copper iodide (500 mg) in methoxyethanol (200 mL) was stirred at room temperature overnight. The reaction mixture was diluted with ethyl acetate and washed with water. The aqueous solution was extracted three times with ethyl acetate. The combined organic layers (total 600ml) were washed with brine, dried over magnesium sulfate, filtered and evaporated. The residue was purified via chromatography (120gm of silica gel, eluted with 0-8% Methanol in dichloromethane) yielding 5.1 g (62%) of the desired product as light oil.

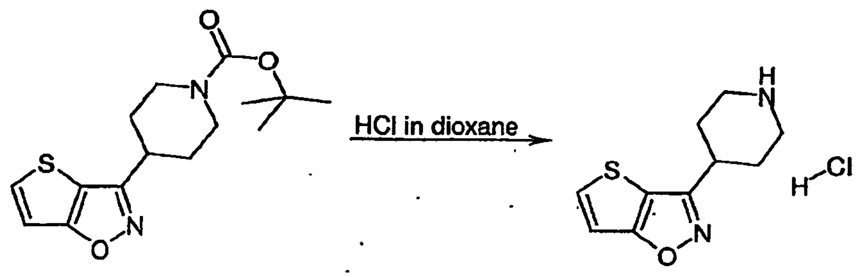


**3-Piperazin-1-yl-thieno[2,3-*d*]isoxazole.** 4-Thieno[2,3-*d*]isoxazol-3-yl-piperazine-1-carboxylic acid *tert*-butyl ester (5.0 g, 16.2 mmol) was stirred at room temperature in a solution of HCl (25 mL, 4N HCl in dioxane) for 4 hours. The volatiles were removed *in vacuo* and the residue was triturated with ether (twice) to yield off white solids 3.3 g (84%) as the desired hydrochloride salt.

<u>Example 29</u>

**Synthesis of 1-(3-Imidazol-1-yl-propyl)-3-{(1R, 2R)-2-[4-(6-trifluoromethyl-benzo[*b*]thiophen-3-yl)-piperazin-1-ylmethyl]-cyclopropylmethyl}-urea (MDL 833306).**

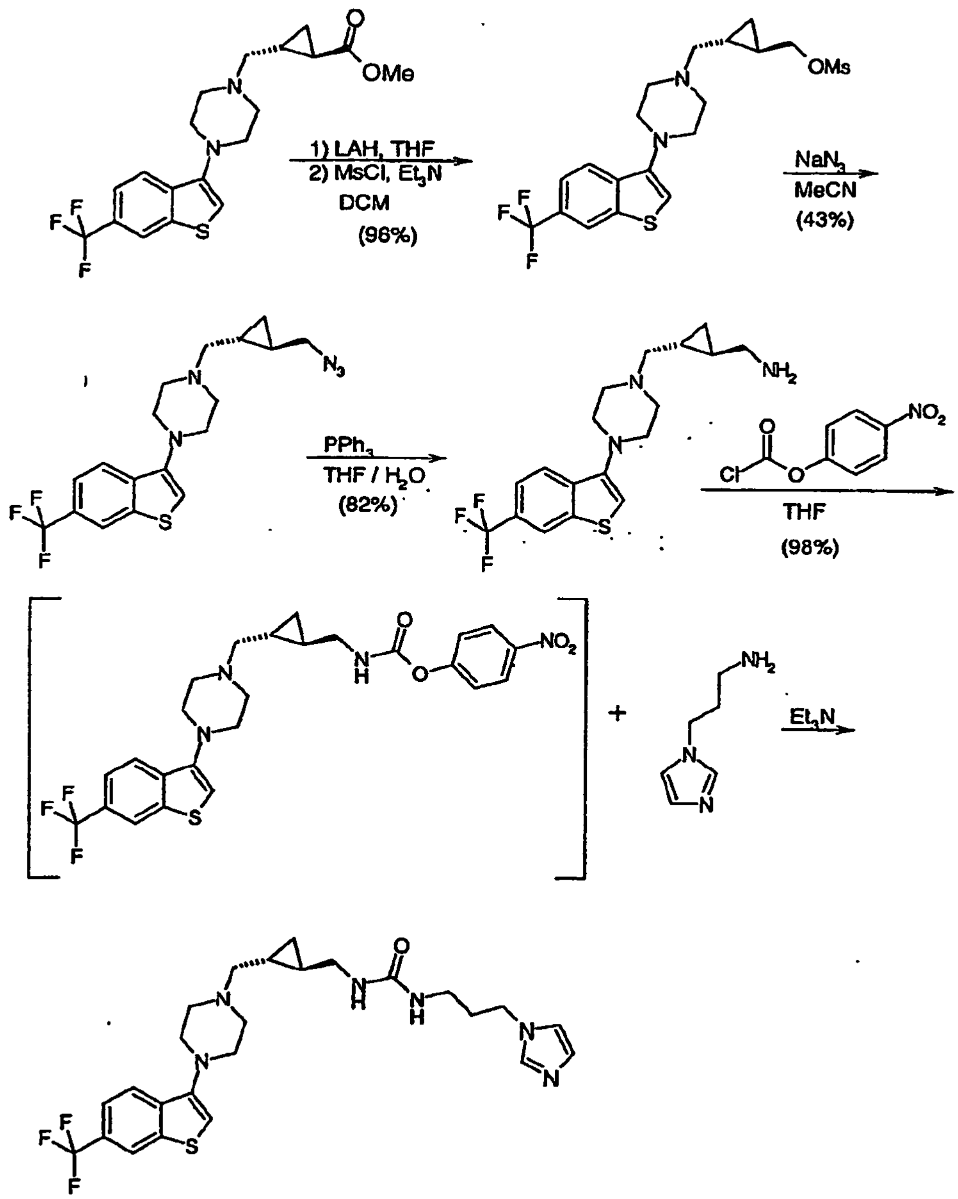


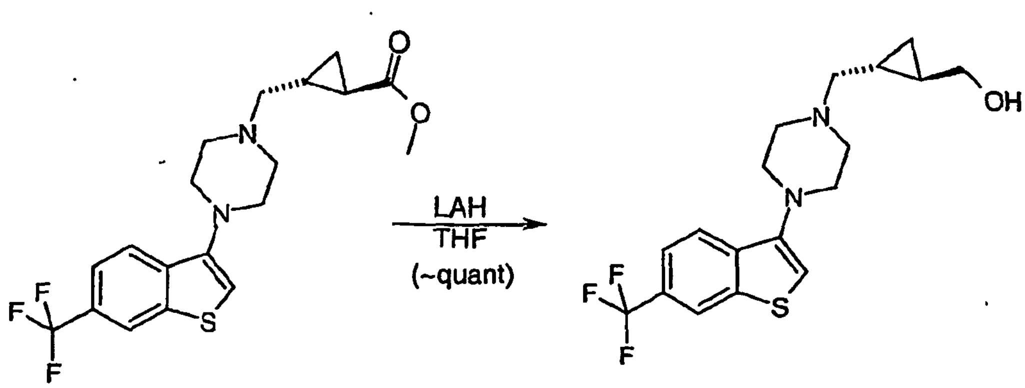


{(1R, 2R)-2-[4-(6-Trifluoromethyl-benzo[b]thiophen-3-yl)-piperazin-1-ylmethyl]-cyclopropyl}-metha-

**nol.** To a stirred solution of 2R-[4-(6-trifluoromethyl-benzo[*b*]thiophen-3-yl)-piperazin-1-ylmethyl]-1R-cyclopropanecarboxylic acid methyl ester (5.0 g, 12.5 mmol) in THF (75 mL) cooled to 0 °C was added lithium aluminum hydride (18.75 mL, 18.75 mmol, 1.0 M in THF) drop-wise. The resulting mixture was stirred at 0 °C for 2 hours when water (1 mL), 2 N NaOH (1 mL) and water (3 mL) was added sequentially. The resulting mixture was diluted with DCM (90 mL) and filtered through a celite plug. The aluminum salts were thoroughly washed with DCM and the filtrate was dried ($MgSO_4$), filtered and evaporated yielding 4.6 g of the desired product.

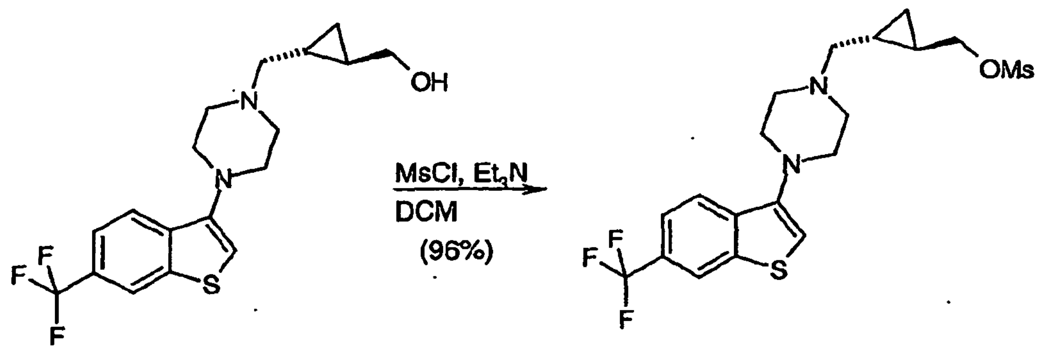


**Methanesulfonic acid (1R, 2R)-2-[4-(6-triftuoromethyl-benzo[b]thiophen-3-yl)-piperazin-1-ylmethyl]-cyclopropylmethyl ester.** To a stirred solution of {(1R, 2R)-2-[4-(6-Trifluoromethyl-benzo[b]thiophen-3-yl)-piperazin-1-ylmethyl]-cyclopropyl}-methanol (3.712 g, 10.03 mmol) in $Et_3N$ (8.5 mL, 61.1 mmol) and anhydrous $CH_2Cl_2$ (100 mL) at 0 °C under $N_2$ was added dropwise $CH_3SO_2Cl$ (930 uL, 12.02 mmol). Stirring was continued at 0 °C for 2.5 h. The reaction was quenched with $H_2O$ (20 mL). A solution of $K_2CO_3$ (4.28 g, 31.01 mmol) in $H_2O$ (60 mL) was then added. The resulting mixture was stirred at rt for 15 min, then extracted with CH2Cl2, washed with brine, dried over $Na_2SO_4$. Filtration, concentration and drying afforded the desired product (4.301 g, 96%).

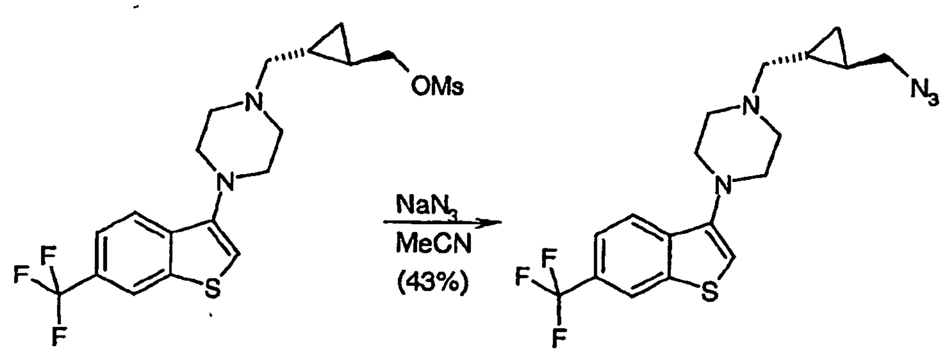


**1-[(1R, 2R)-2-Azidomethyl-cyclopropylmethyl]-4-(6-trifluoromethyl-benzo [b] thiophen-3-yl)-piperazine.** A mixture of Methanesulfonic acid (1R, 2R)-2-[4-(6-trifluoromethyl-benzo[b]thiophen-3-yl)-piperazin-1-ylmethyl]-cyclopropylmethyl ester (3.995 g , 8.92 mmol), $NaN_3$ (1.16 g , 17.85 mmol) and anhydrous $CH_3CN$ (60 mL) was stirred at 47 °C under $N_2$ for 4 h, then an additional quantity of $NaN_3$ (580 mg, 8.92 mmol) was added. Stirring was continued at 47°C for a further 4 h. After cooling to rt, the mixture was filtered through Celite 545, washed with $CH_3CN$. The combined filtrate and washings were concentrated and then separated by Prep LC (heptane/EtOAc- 70:30, 60:40, 50:50, 40:60, 30:70, 20:80, 100% EtOAc) to give the desired product (1.5 g, 43 %).

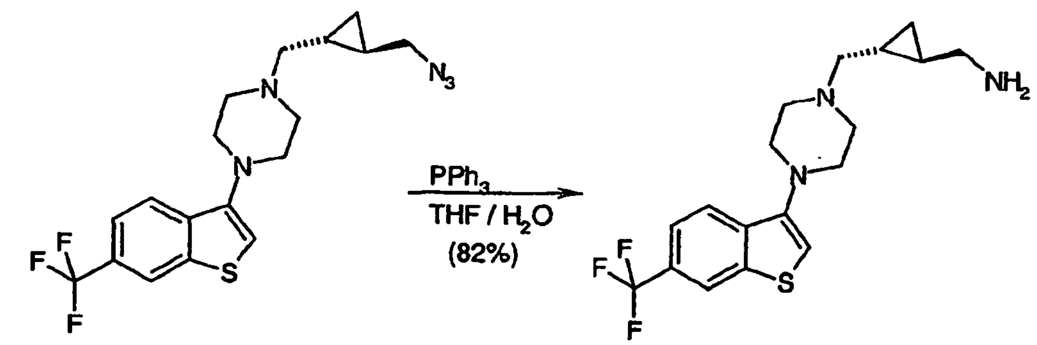


***C*-{(1R,2R)-2-[4-(6-Trifluoromethyl-benzo[*b*]thiophen-3-yl)-piperazin-1-ylmethyl]-cyclopropyl}methylamine.** A solution of 1-[(1R,2R)-2-Azidomethyl-cyclopropylmethyl]-4-(6-trifluoromethyl-benzo[*b*]thiophen-3-yl)-piperazine (1.495 g , 3.78 mmol), $PPh_3$ (3.97 g, 15.15 mmol) and $H_2O$ (273 uL, 15.17 mmol) in THF (30 mL) was stirred at 40 ˚C under $N_2$ for 18 h, then at 55 ˚C for 23 h. After cooling to rt, the mixture was concentrated, and then flash chromatographed (100% EtOAc, then MeOH / $CH_2Cl_2$ /$Et_3N$--60:40:10) to provide the desired product (1.14 g , 82 %).

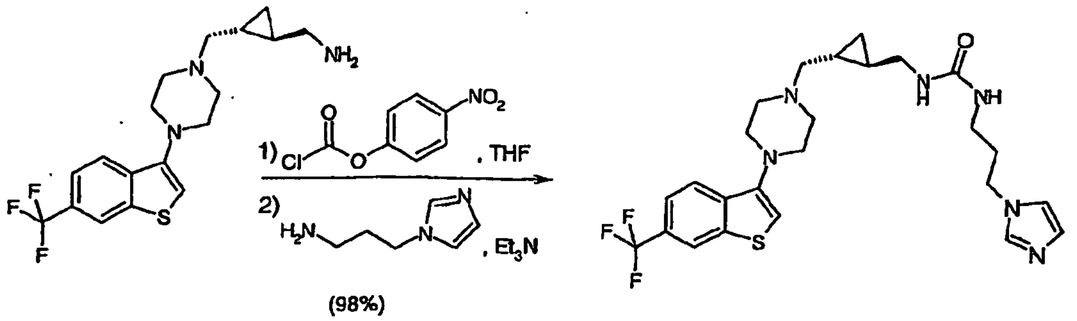


1-(3-Imidazol-1-yl-propyl)-3-{(1R, 2R)-2-[4-(6-trifluoromethyl-benzo[*b*]thiophen-3-yl)-piperazin-1-ylmethyl]-cyclopropylmethyl}-urea (MDL 833306). A solution of *C*-{(1R,2R)-2-[4-(6-Trifluoromethyl-benzo[b]thiophen-3-yl)-piperazin-1-ylmethyl]-cyclopropyl}methylamine (30 mg , 0.081 mmol) and 4-nitrophenyl chloroformate (18 mg , 0.089 mmol) in anhydrous THF (3 mL) was stirred at rt under $N_2$ for 1 h. Then $Et_3N$ (113 uL, 0.81 mmol) was added, followed by 3-imidazol-1-yl-propylamine (39 uL , 0.33 mmol). Stirring was continued at 35 ˚C for 2 h. The reaction mixture was filtered. The filtrate was concentrated, then separated by Prep LC (MeOH / $CH_2Cl_2$-5:95, 10:90, 15:85, 20:80, 25:75, 30:70) to give the target compound (41 mg, 98 %).

TABLE III

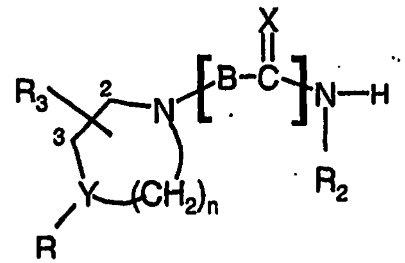

| COMPOUND NUMBER | R | n | 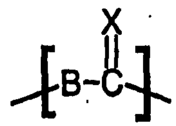 | $R_2$ | $R_3$ | Y | $d3K_i$ |
|---|---|---|---|---|---|---|---|
| 815383 | 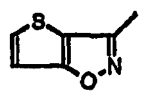 | 2 | 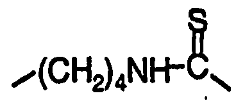 | 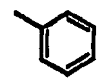 | H | CH | 190 |
| 815384 | 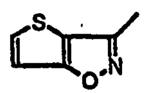 | 2 | 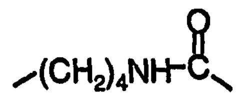 | 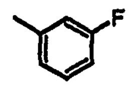 | H | CH | 9 |
| 815385 | 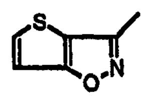 | 2 | 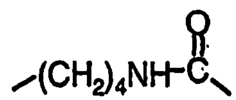 | 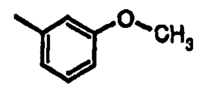 | H | CH | 4 |
| 815386 | 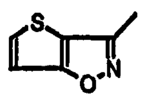 | 2 | 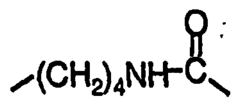 | 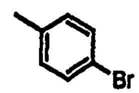 | H | CH | 18 |
| 815387 | 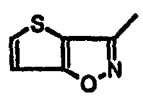 | 2 | 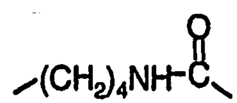 | 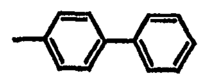 | H | CH | 28 |

(continued)

| COMPOUND NUMBER | R | n | 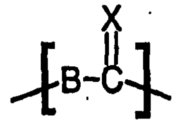 | $R_2$ | $R_3$ | Y | $d3K_i$ |
|---|---|---|---|---|---|---|---|
| 815388 | 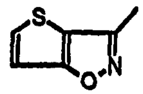 | 2 | 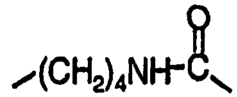 | 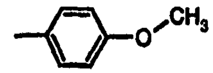 | H | CH | 12 |
| 815389 | 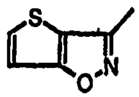 | 2 | 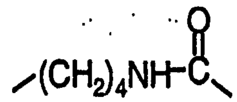 | 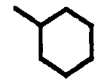 | H | CH | 30 |
| 815390 | 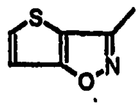 | 2 | 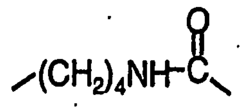 | 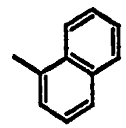 | H | CH | 52 |
| 815391 | 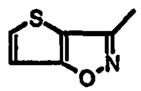 | 2 | 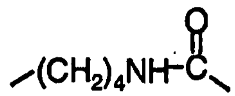 | 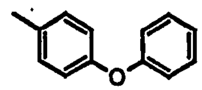 | H | CH | 16 |
| 815392 | 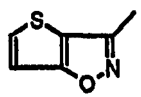 | 2 | 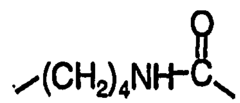 | 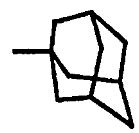 | H | CH | 24 |
| 815393 | 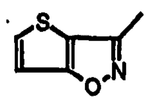 | 2 | 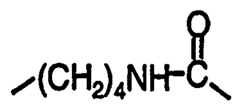 | 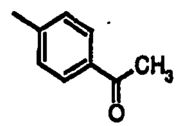 | H | CH | 3.7 |
| 815394 | 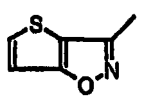 | 2 | 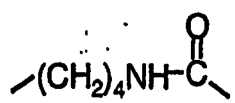 | 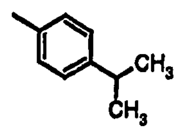 | H | CH | 13 |

(continued)

| COMPOUND NUMBER | R | n | 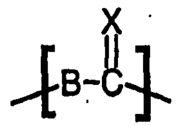 | $R_2$ | $R_3$ | Y | $d3K_i$ |
|---|---|---|---|---|---|---|---|
| 815395 | 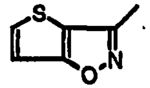 | 2 | 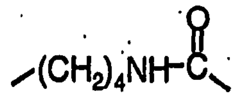 | 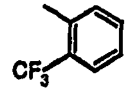 | H | CH | 110 |
| 815934 | 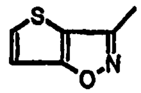 | 2 | 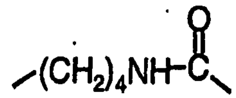 | 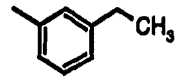 | H | CH | 4.19 |
| 815935 | 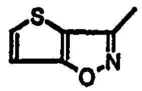 | 2 | 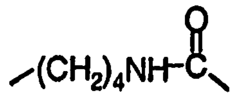 | 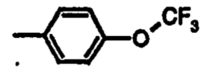 | H | CH | 53.8 |
| 815936 | 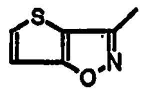 | 2 | 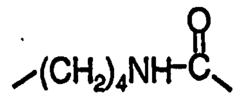 | 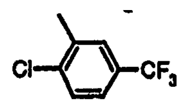 | H | CH | 3.91 |
| 815937 | 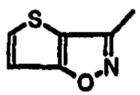 | 2 | 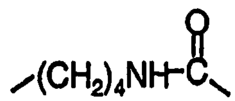 | 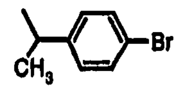 | H | CH | 66.3 |
| 815938 | 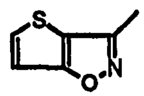 | 2 | 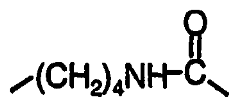 | 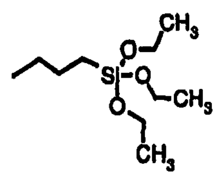 | H | CH | 153 |
| 815939 | 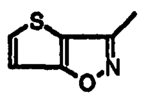 | 2 | 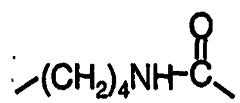 | 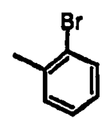 | H | CH | 19.1 |

(continued)

| COMPOUND NUMBER | R | n | 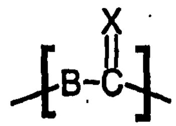 | $R_2$ | $R_3$ | Y | $d3K_i$ |
|---|---|---|---|---|---|---|---|
| 815940 | 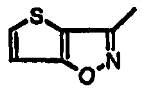 | 2 | 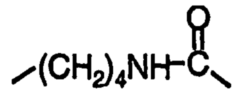 | 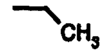 | H | CH | 140 |
| 815941 | 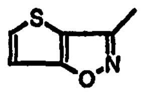 | 2 | 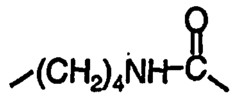 | 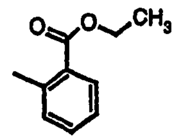 | H | CH | 128 |
| 815942 | 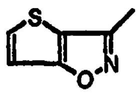 | 2 | 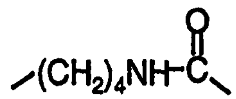 | 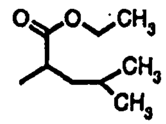 | H | CH | 41.7 |
| 815943 | 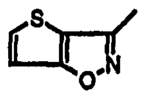 | 2 | 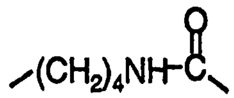 | 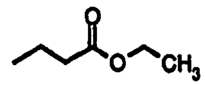 | H | CH | 198 |
| 815944 | 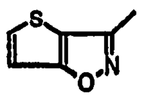 | 2 | 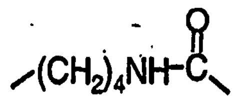 | 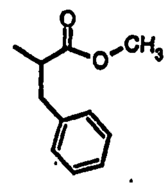 | H | CH | 297 |
| 815945 | 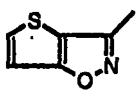 | 2 | 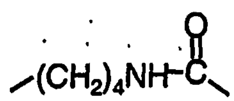 | 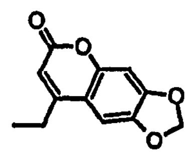 | H | CH | 116 |
| 815946 | 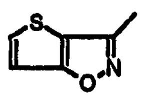 | 2 | 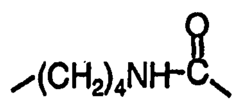 | $-(CH_2)_7CH_3$ | H | CH | 28.3 |

(continued)

| COMPOUND NUMBER | R | n | 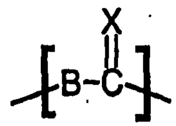 | $R_2$ | $R_3$ | Y | $d3K_i$ |
|---|---|---|---|---|---|---|---|
| 815947 | 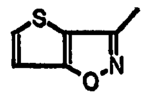 | 2 | 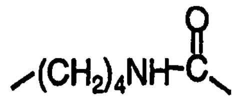 | 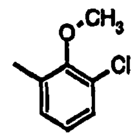 | H | CH | 26 |
| 815948 | 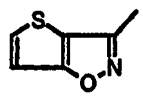 | 2 | 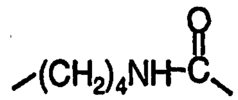 | 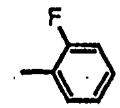 | H | CH | 6.04 |
| 815949 | 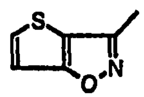 | 2 | 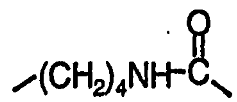 | 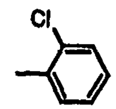 | H | CH | 13.6 |
| 815950 | 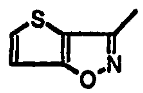 | 2 | 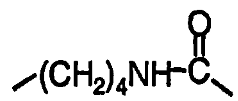 | 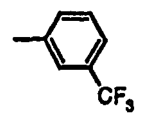 | H | CH | 4.59 |
| 815951 | 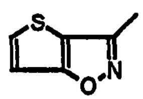 | 2 | 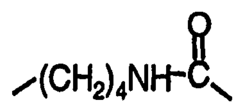 | 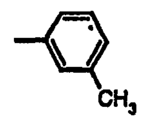 | H | CH | 2.23 |
| 815952 | 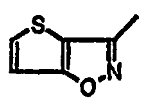 | 2 | 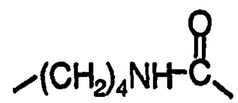 | 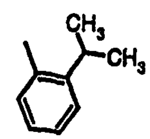 | H | CH | 27 |
| 815953 | 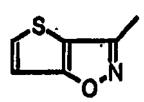 | 2 | 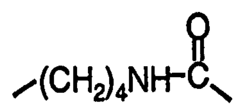 | 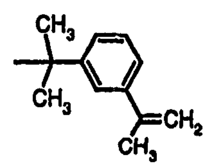 | H | CH | 67.9 |

(continued)

| COMPOUND NUMBER | R | n | 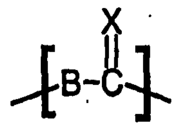 | $R_2$ | $R_3$ | Y | $d3K_i$ |
|---|---|---|---|---|---|---|---|
| 815954 | 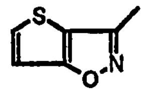 | 2 | 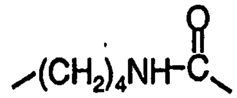 | 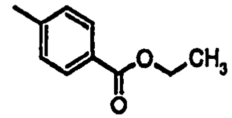 | H | CH | 14.9 |
| 81595 | 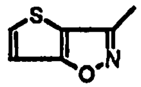 | 2 | 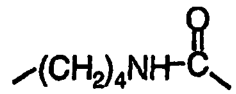 | 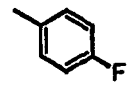 | H | CH | 23.5 |
| 81595 | 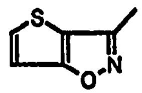 | 2 | 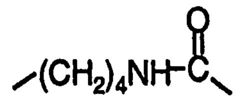 | 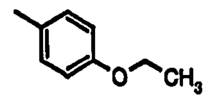 | H | CH | 5.24 |
| 816210 | 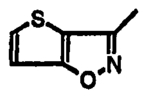 | 2 | 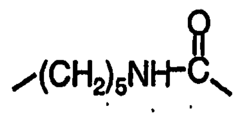 | 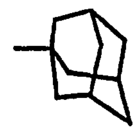 | H | CH | 187 |
| 816557 | 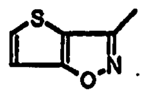 | 2 | 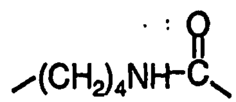 | 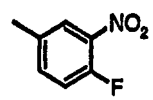 | H | CH | 20.7 |
| 816558 | 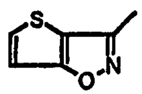 | 2 | 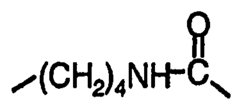 | 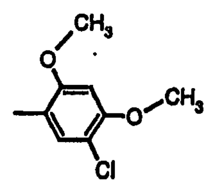 | H | CH | 33.3 |
| 816559 | 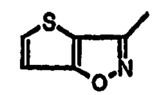 | 2 | 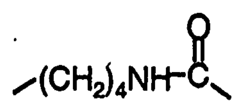 | 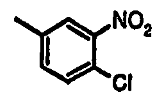 | H | CH | 13.1 |

(continued)

| COMPOUND NUMBER | R | n | 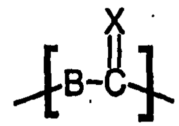 | $R_2$ | $R_3$ | Y | $d3K_i$ |
|---|---|---|---|---|---|---|---|
| 816560 | 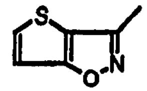 | 2 | 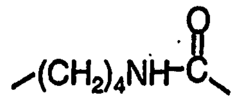 | 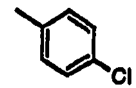 | H | CH | 10.9 |
| 816561 | 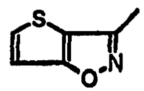 | 2 | 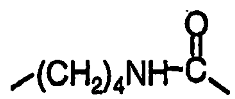 | 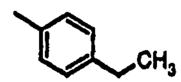 | H | CH | 24.2 |
| 816562 | 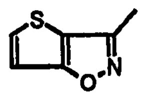 | 2 | 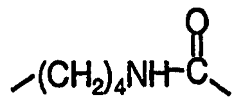 | 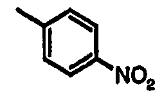 | H | CH | 6.99 |
| 816563 | 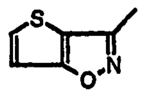 | 2 | 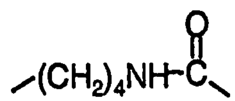 | 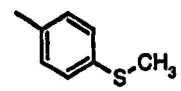 | H | CH | 21.5 |
| 816564 | 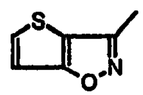 | 2 | 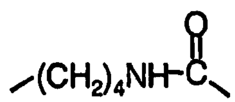 | 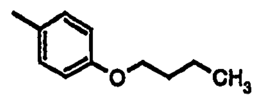 | H | CH | 55.9 |
| 816565 | 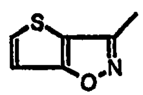 | 2 | 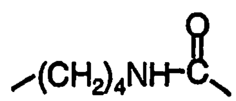 | 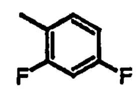 | H | CH | 55.5 |
| 816566 | 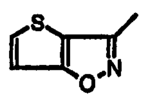 | 2 | 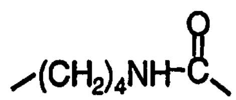 | 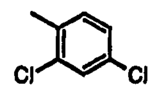 | H | CH | 95.1 |
| 816567 | 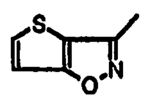 | 2 | 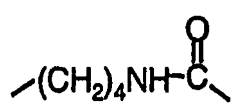 | 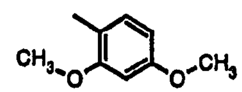 | H | CH | 73.8 |

(continued)

| COMPOUND NUMBER | R | n | 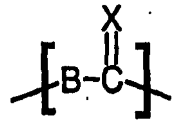 | $R_2$ | $R_3$ | Y | $d3K_i$ |
|---|---|---|---|---|---|---|---|
| 816568 | 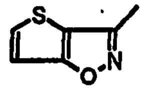 | 2 | 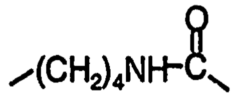 | 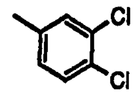 | H | CH | 11.2 |
| 816748 | 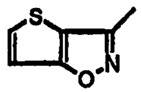 | 2 | 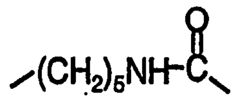 | 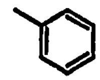 | H | CH | 97.2 |
| 816749 | 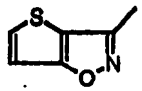 | 2 | 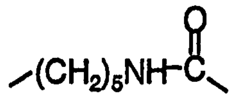 | 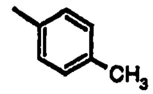 | H | CH | 115 |
| 816750 | 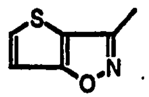 | 2 | 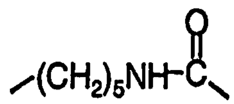 | 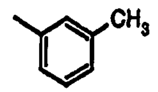 | H | CH | 92.7 |
| 816751 | 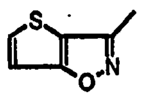 | 2 | 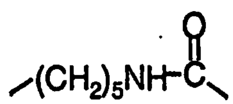 | 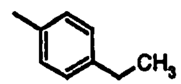 | H | CH | 131 |
| 816752 | 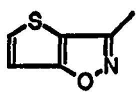 | 2 | 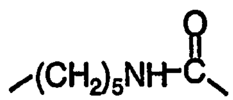 | 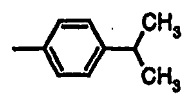 | H | CH | 157 |
| 816753 | 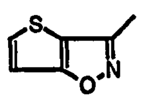 | 2 | 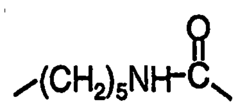 | 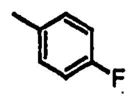 | H | CH | 115 |
| 816754 | 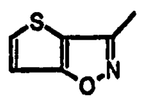 | 2 | 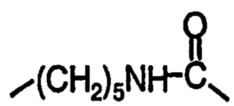 | 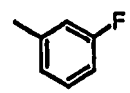 | H | CH | 108 |

(continued)

| COMPOUND NUMBER | R | n | 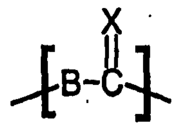 | $R_2$ | $R_3$ | Y | $d3K_i$ |
|---|---|---|---|---|---|---|---|
| 816755 | 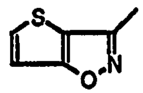 | 2 | 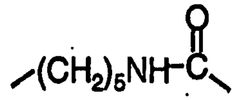 | 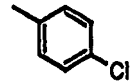 | H | CH | 80.8 |
| 816756 | 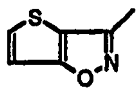 | 2 | 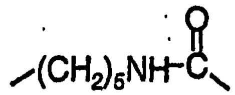 | 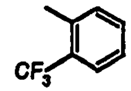 | H | CH | 96.1 |
| 816757 | 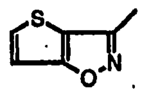 | 2 | 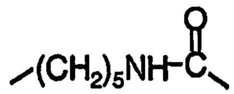 | 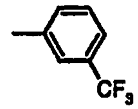 | H | CH | 92.6 |
| 816758 | 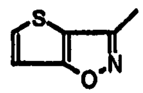 | 2 | 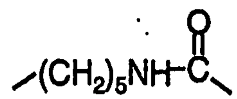 | 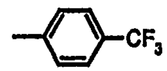 | H | CH | 116 |
| 816759 | 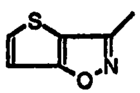 | 2 | 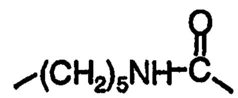 | 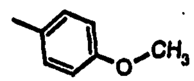 | H | CH | 107 |
| 816760 | 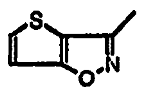 | 2 | 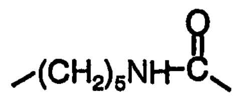 | 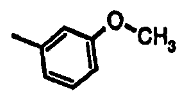 | H | CH | 121 |
| 816761 | 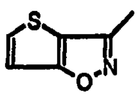 | 2 | 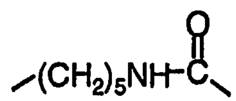 | 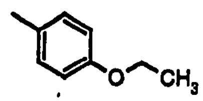 | H | CH | 82.9 |
| 816762 | 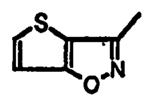 | 2 | 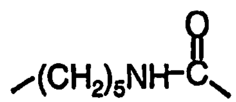 | 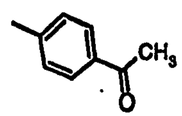 | H | CH | 57.1 |

(continued)

| COMPOUND NUMBER | R | n | 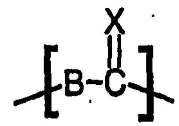 | $R_2$ | $R_3$ | Y | $d3K_i$ |
|---|---|---|---|---|---|---|---|
| 816763 | 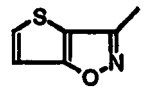 | 2 | 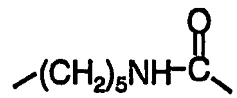 | 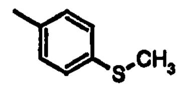 | H | CH | 78.9 |
| 816764 | 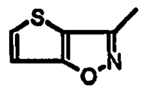 | 2 | 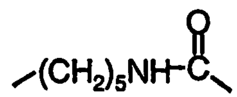 | 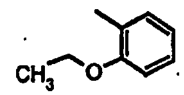 | H | CH | 85.4 |
| 816765 | 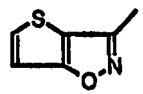 | 2 | 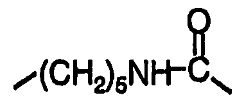 | 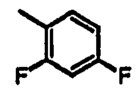 | H | CH | 104 |
| 816766 | 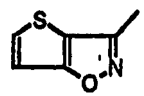 | 2 | 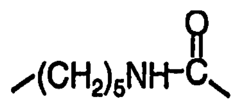 | 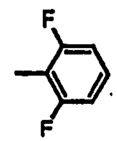 | H | CH | 42.9 |
| 816767 | 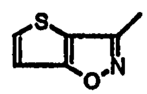 | 2 | 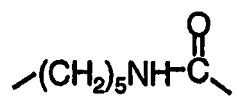 | 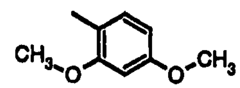 | H | CH | 60.1 |
| 816768 | 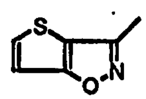 | 2 | 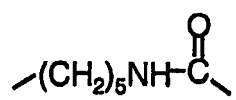 | 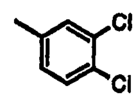 | H | CH | 23.4 |
| 816769 | 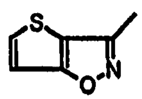 | 2 | 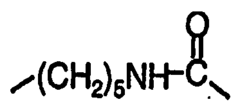 | 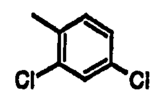 | H | CH | 57.8 |
| 816770 | 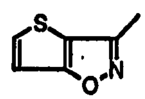 | 2 | 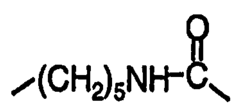 | 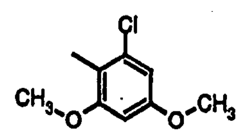 | H | CH | 28.2 |

(continued)

| COMPOUND NUMBER | R | n | 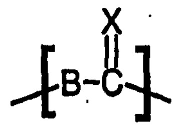 | $R_2$ | $R_3$ | Y | $d3K_i$ |
|---|---|---|---|---|---|---|---|
| 816771 | 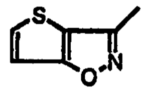 | 2 | 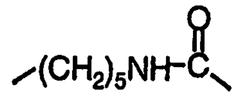 | 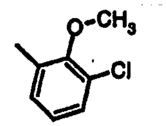 | H | CH | 74.1 |
| 816860 | 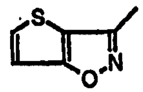 | 2 | 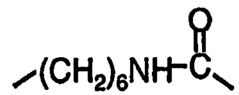 | 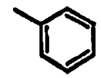 | H | CH | 11.8 |
| 816861 | 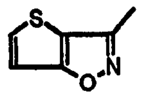 | 2 | 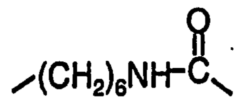 | 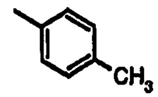 | H | CH | 14.8 |
| 816862 | 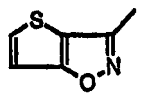 | 2 | 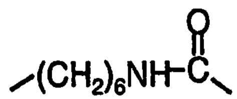 | 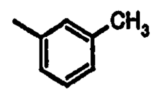 | H | CH | 19 |
| 816863 | 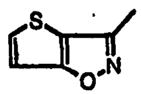 | 2 | 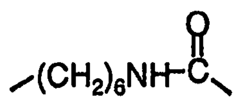 | 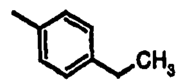 | H | CH | 20 |
| 816864 | 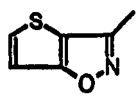 | 2 | 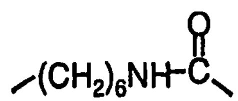 | 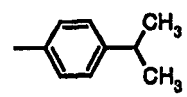 | H | CH | 21.5 |
| 816865 | 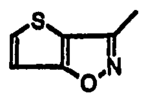 | 2 | 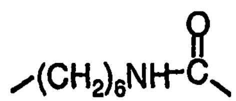 | 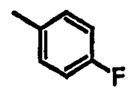 | H | CH | 11.9 |
| 816866 | 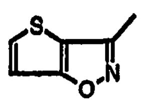 | 2 | 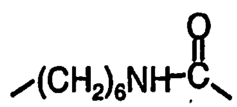 | 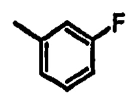 | H | CH | 8.25 |

(continued)

| COMPOUND NUMBER | R | n | 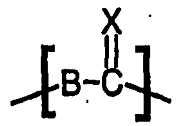 | $R_2$ | $R_3$ | Y | $d3K_i$ |
|---|---|---|---|---|---|---|---|
| 816867 | 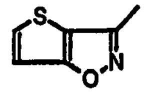 | 2 | 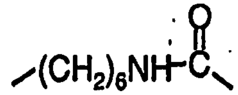 | 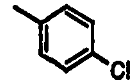 | H | CH | 7.38 |
| 816868 | 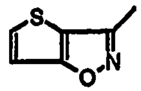 | 2 | 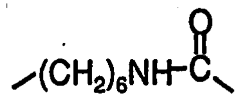 | 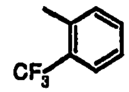 | H | CH | 58.6 |
| 816869 | 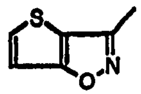 | 2 | 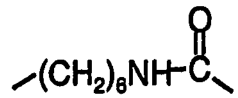 | 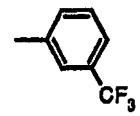 | H | CH | 23.5 |
| 816870 | 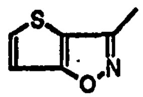 | 2 | 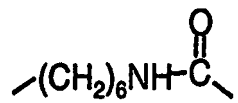 | 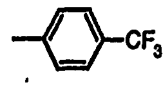 | H | CH | 17.6 |
| 816871 | 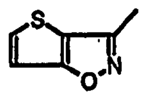 | 2 | 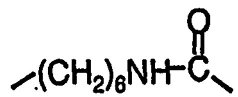 | 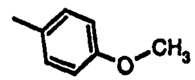 | H | CH | 17.3 |
| 816872 | 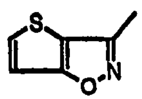 | 2 | 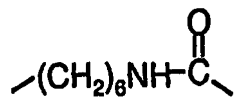 | 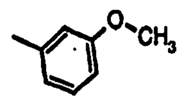 | H | CH | 19 |
| 816873 | 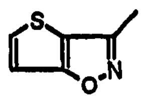 | 2 | 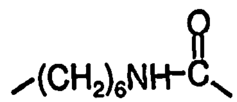 | 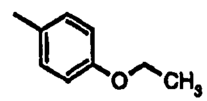 | H | CH | 22 |
| 816874 | 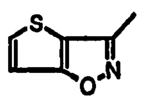 | 2 | 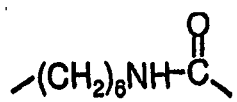 | 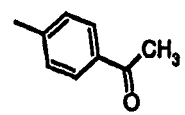 | H | CH | 6.84 |

(continued)

| COMPOUND NUMBER | R | n | 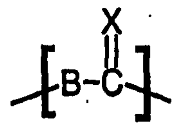 | R2 | R3 | Y | d3Ki |
|---|---|---|---|---|---|---|---|
| 816875 | 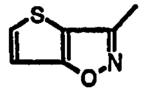 | 2 | 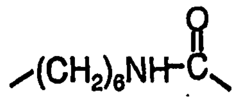 | 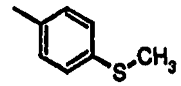 | H | CH | 35.4 |
| 816876 | 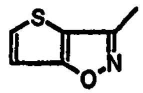 | 2 | 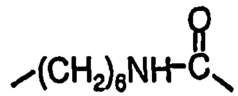 | 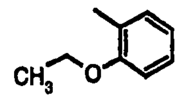 | H | CH | 46.9 |
| 816877 | 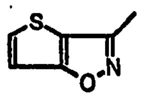 | 2 | 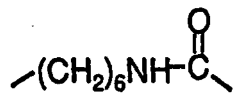 | 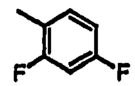 | H | CH | 19.1 |
| 816878 | 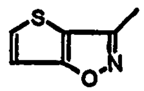 | 2 | 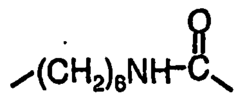 | 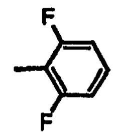 | H | CH | 33.8 |
| 816879 | 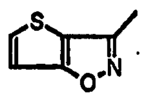 | 2 | 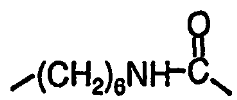 | 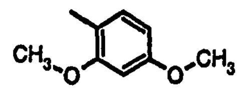 | H | CH | 47 |
| 816880 | 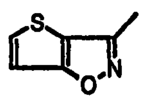 | 2 | 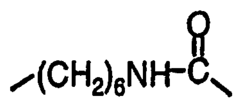 | 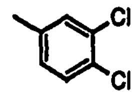 | H | CH | 14.7 |
| 816881 | 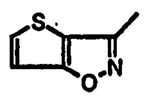 | 2 | 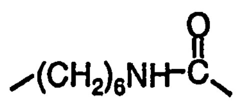 | 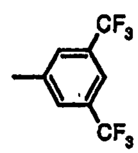 | H | CH | 23.8 |

(continued)

| COMPOUND NUMBER | R | n | 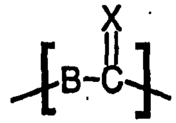 | $R_2$ | $R_3$ | Y | $d3K_i$ |
|---|---|---|---|---|---|---|---|
| 816882 | 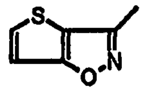 | 2 | 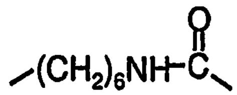 | 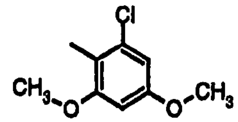 | H | CH | 58.6 |
| 816883 | 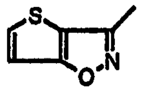 | 2 | 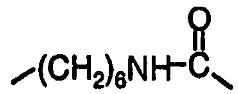 | 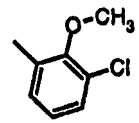 | H | CH | 35.5 |
| 816968 | 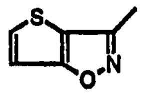 | 2 | 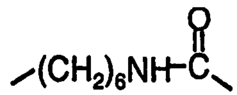 | 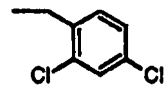 | H | CH | 24.2 |
| 817075 | 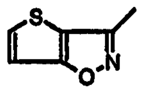 | 2 | 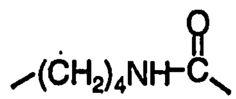 | 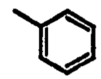 | H | CH | 12.7 |
| 818280 | 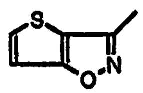 | 2 | 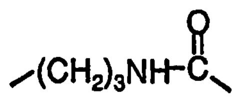 | 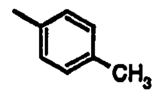 | H | CH | 217 |
| 818283 | 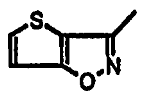 | 2 | 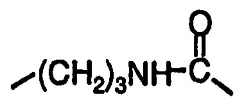 | 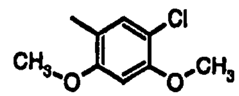 | H | CH | 131 |
| 818308 | 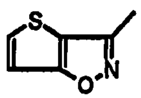 | 2 | 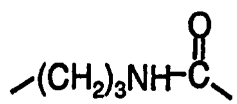 | 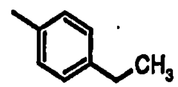 | H | CH | 351 |
| 818309 | 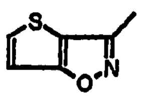 | 2 | 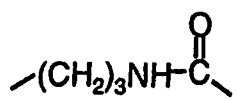 | 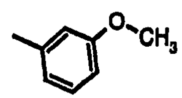 | H | CH | 390 |

(continued)

| COMPOUND NUMBER | R | n | 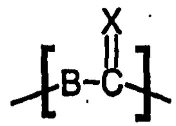 | $R_2$ | $R_3$ | Y | $d3K_i$ |
|---|---|---|---|---|---|---|---|
| 818310 | 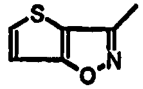 | 2 | 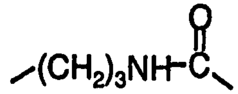 | 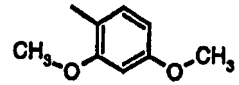 | H | CH | 133 |
| 818316 | 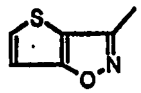 | 2 | 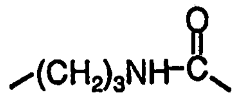 | 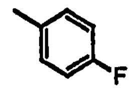 | H | CH | 426 |
| 818318 | 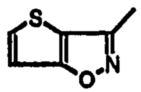 | 2 | 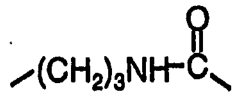 | 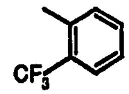 | H | CH | 384 |
| 818426 | 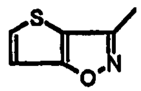 | 2 | 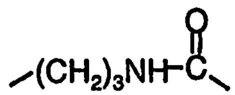 | 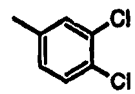 | H | CH | 158 |
| 817510 | 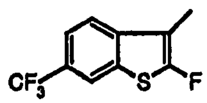 | 2 | 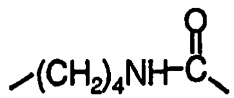 | 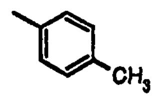 | H | N | 0.665 |
| 815158 | 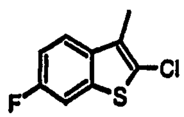 | 2 | 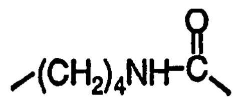 | 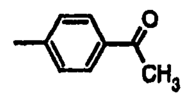 | H | N | 7.4 |
| 815159 | 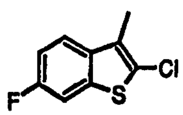 | 2 | 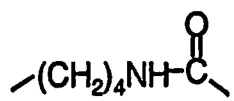 | 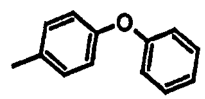 | H | N | 85.4 |
| 815160 | 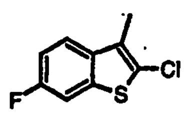 | 2 | 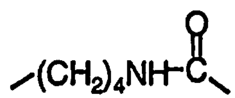 | 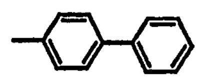 | H | N | 192 |

(continued)

| COMPOUND NUMBER | R | n | 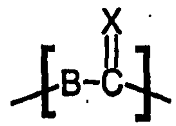 | $R_2$ | $R_3$ | Y | $d3K_i$ |
|---|---|---|---|---|---|---|---|
| 815161 | 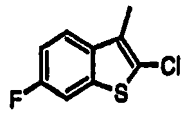 | 2 | 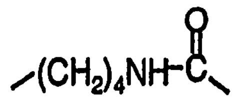 | 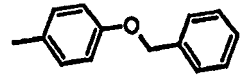 | H | N | 75.9 |
| 815162 | 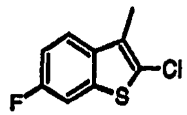 | 2 | 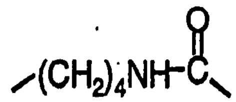 | 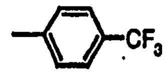 | H | N | 38 |
| 815163 | 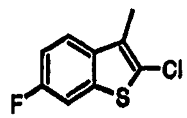 | 2 | 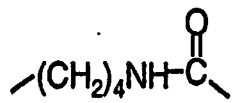 | 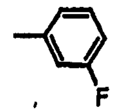 | H | N | 25.2 |
| 815164 | 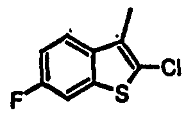 | 2 | 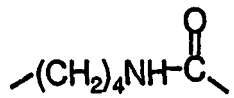 | 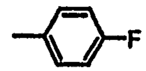 | H | N | 31.9 |
| 815165 | 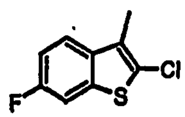 | 2 | 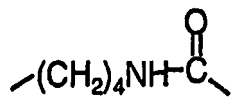 | 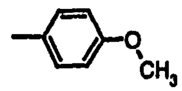 | H | N | 21.4 |
| 815166 | 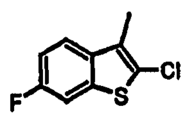 | 2 | 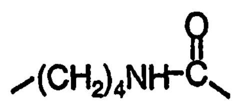 | 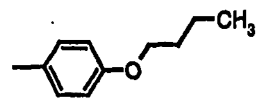 | H | N | 37 |
| 815167 | 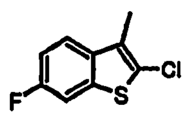 | 2 | 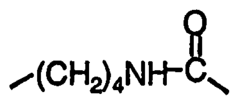 | 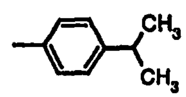 | H | N | 48 |

(continued)

| COMPOUND NUMBER | R | n | 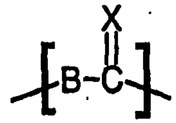 | $R_2$ | $R_3$ | Y | $d3K_i$ |
|---|---|---|---|---|---|---|---|
| 815168 | 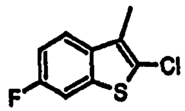 | 2 | 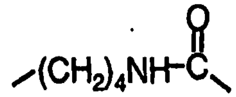 | 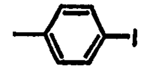 | H | N | 33.4 |
| 815169 | 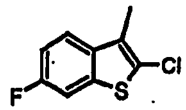 | 2 | 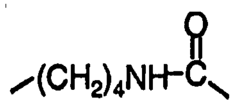 | 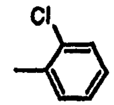 | H | N | 11.2 |
| 815170 | 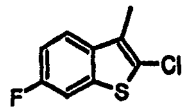 | 2 | 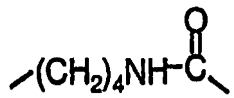 | 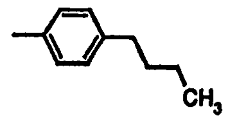 | H | N | 17.7 |
| 815171 | 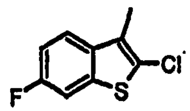 | 2 | 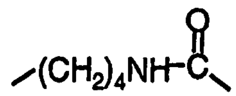 | 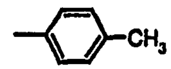 | H | N | 3.8 |
| 815172 | 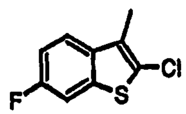 | 2 | 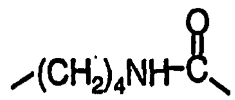 | 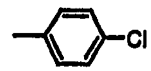 | H | N | 5.6 |
| 815173 | 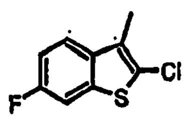 | 2 | 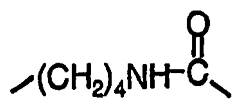 | 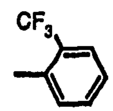 | H | N | 46.5 |
| 815174 | 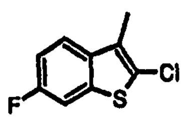 | 2 | 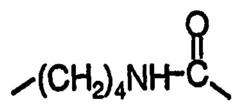 | 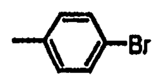 | H | N | 6 |

(continued)

| COMPOUND NUMBER | R | n | 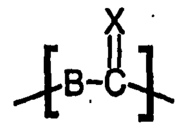 | $R_2$ | $R_3$ | Y | $d3K_i$ |
|---|---|---|---|---|---|---|---|
| 815175 | 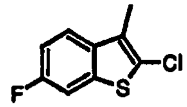 | 2 | 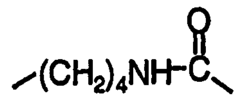 | 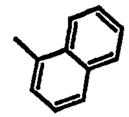 | H | N | 11.8 |
| 815176 | 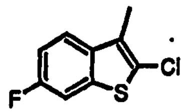 | 2 | 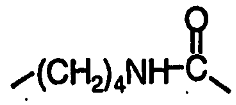 | 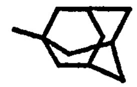 | H | N | 17.1 |
| 815177 | 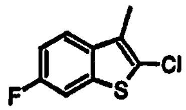 | 2 | 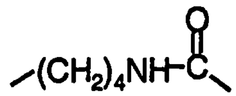 | 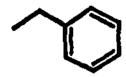 | H | N | 18 |
| 815993 | 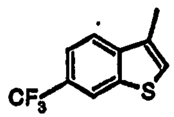 | 2 | 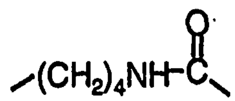 | 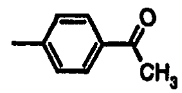 | H | N | 2.26 |
| 815994 | 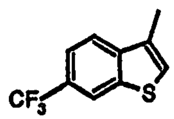 | 2 | 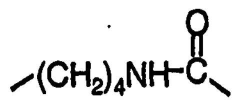 | 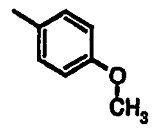 | H | N | 8.87 |
| 815995 | 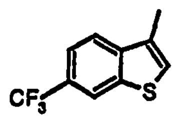 | 2 | 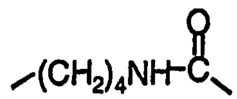 | 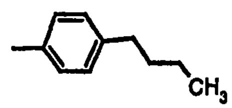 | H | N | 17.5 |
| 815996 | 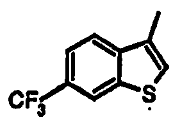 | 2 | 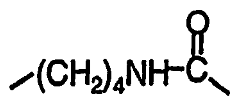 | 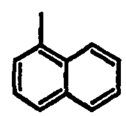 | H | N | 5.85 |

(continued)

| COMPOUND NUMBER | R | n | 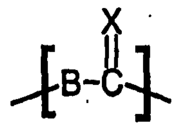 | $R_2$ | $R_3$ | Y | $d3K_i$ |
|---|---|---|---|---|---|---|---|
| 815997 | 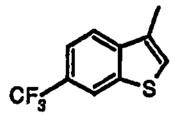 | 2 | 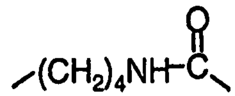 | 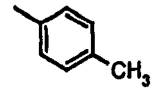 | H | N | 4.5 |
| 815998 | 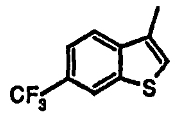 | 2 | 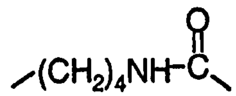 | 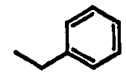 | H | N | 22.5 |
| 815999 | 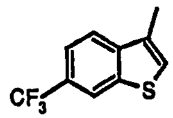 | 2 | 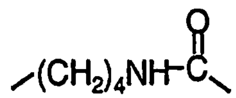 | 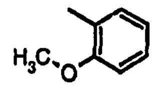 | H | N | 19 |
| 816000 | 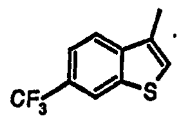 | 2 | 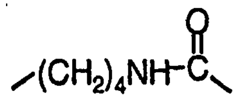 | 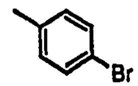 | H | N | 6.31 |
| 816001 | 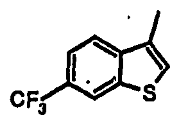 | 2 | 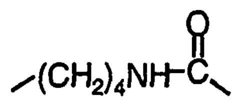 | 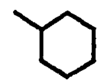 | H | N | 4.68 |
| 816002 | 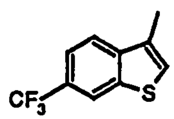 | 2 | 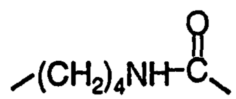 | 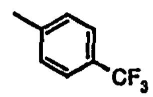 | H | N | 12.5 |
| 816003 | 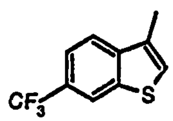 | 2 | 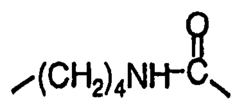 | 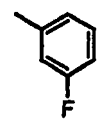 | H | N | 1.58 |

(continued)

| COMPOUND NUMBER | R | n | 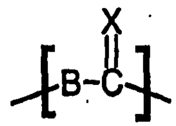 | $R_2$ | $R_3$ | Y | $d3K_i$ |
|---|---|---|---|---|---|---|---|
| 816004 | 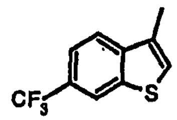 | 2 | 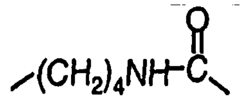 | 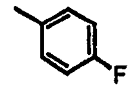 | H | N | 6.11 |
| 816005 | 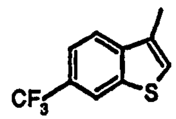 | 2 | 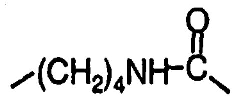 | 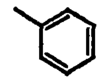 | H | N | 2.54 |
| 816006 | 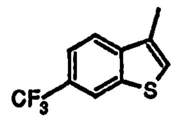 | 2 | 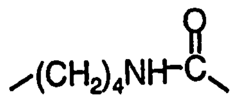 | 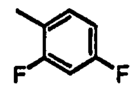 | H | N | 7.42 |
| 816007 | 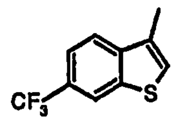 | 2 | 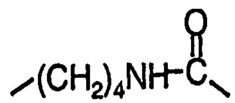 | 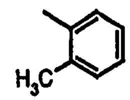 | H | N | 17.5 |
| 816008 | 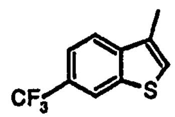 | 2 | 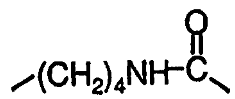 | 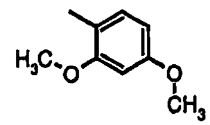 | H | N | 17 |
| 816226 | 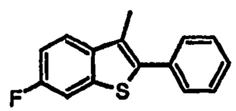 | 2 | 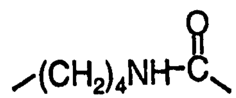 | 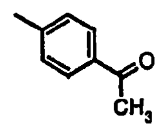 | H | N | 184 |
| 816232 | 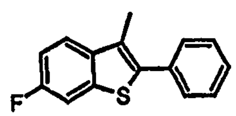 | 2 | 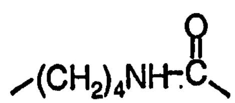 | 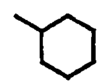 | H | N | 651 |

(continued)

| COMPOUND NUMBER | R | n | 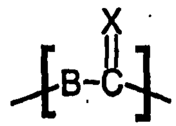 | $R_2$ | $R_3$ | Y | $d3K_i$ |
|---|---|---|---|---|---|---|---|
| 815994A HCl Salt | 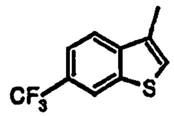 | 2 | 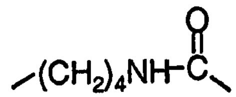 | 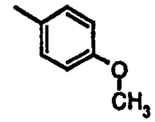 | H | N | 5.94 |
| 815997A HCl Salt | 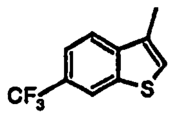 | 2 | 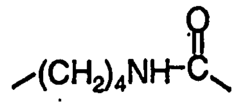 | 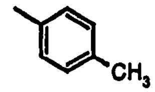 | H | N | 4.08 |
| 817510 | 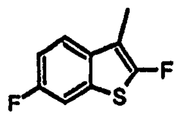 | 2 | 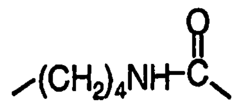 | 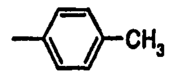 | H | N | 0.665 |
| 817511 | 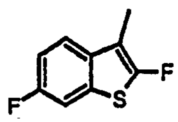 | 2 | 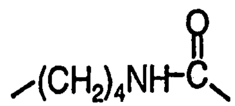 | 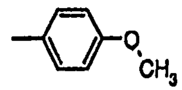 | H | N | 1 |
| 817512 | 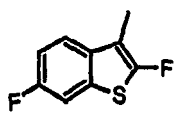 | 2 | 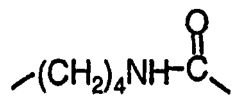 | 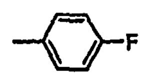 | H. | N | 1.09 |
| 817513 | 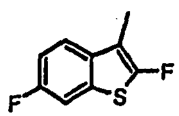 | 2 | 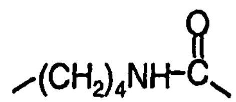 | 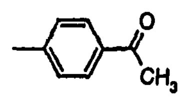 | H | N | 0.546 |

| 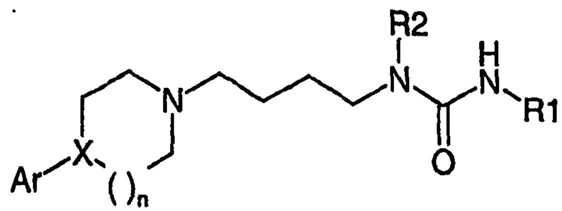 | | | | | | |
|---|---|---|---|---|---|---|
| **MDL #** | **D3 KI (nM)** | **n** | **X** | **Ar** | **R1** | **R2** |
| 833724 | 2.1 | 1 | N | 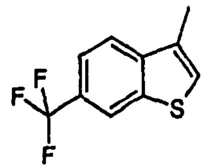 | 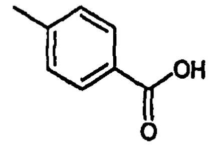 | H |
| 833725 | 0.602 | 1 | N | 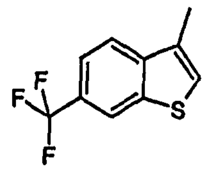 | 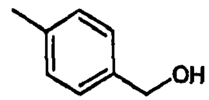 | H |
| 829191 | 43 | 2 | N | 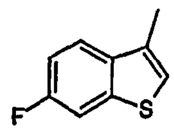 | 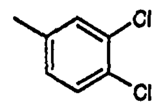 | H |
| 829192 | 4.03 | 2 | N | 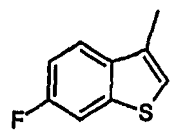 | 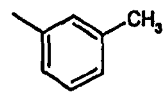 | H |
| 829193 | 7.86 | 2 | N | 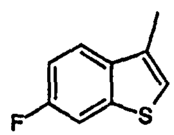 | 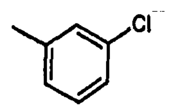 | H |

(continued)

| MDL # | D3 KI (nM) | n | X | Ar | R1 | R2 |
|---|---|---|---|---|---|---|
| 829195 | 21.1 | 2 | N | 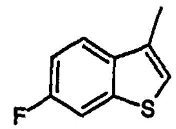 | 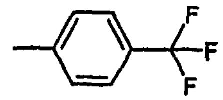 | H |
| 829196 | 10.9 | 2 | N | 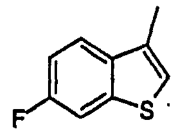 | 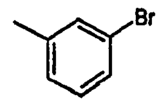 | H |
| 829197 | 34.8 | 2 | N | 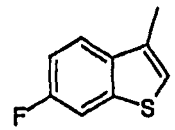 | 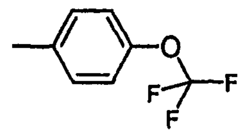 | H |
| 829198 | 65.6 | 2 | N | 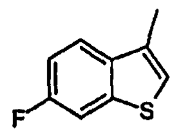 | 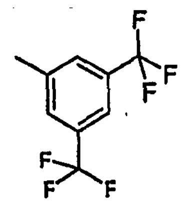 | H |
| 829199 | 6.51 | 2 | N | 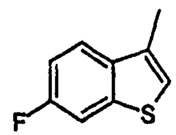 | 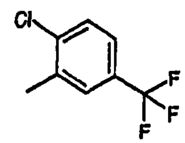 | H |
| 829200 | 47.8 | 2 | N | 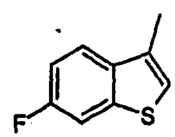 | 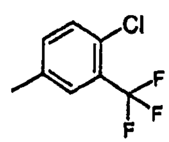 | H |
| 829202 | 16.6 | 2 | N | 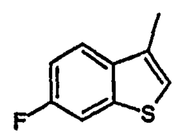 | 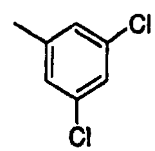 | H |

(continued)

| MDL # | D3 KI (nM) | n | X | Ar | R1 | R2 |
|---|---|---|---|---|---|---|
| 829203 | 5.64 | 2 | N | 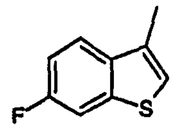 | 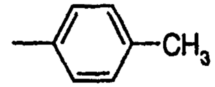 | H |
| 829204 | 5 | 2 | N | 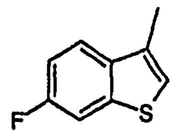 | 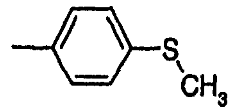 | H |
| 829206 | 2.5 | 2 | N | 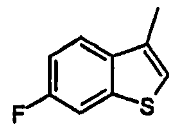 | 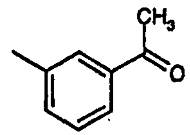 | H |
| 829207 | 141 | 2 | N | 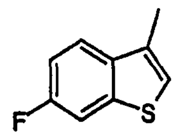 | 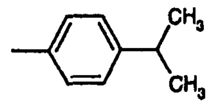 | H |
| 829208 | 0.0636 | 2 | N | 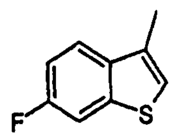 | 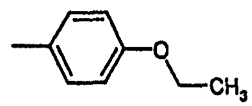 | H |
| 829210 | 1.99 | 2 | N | 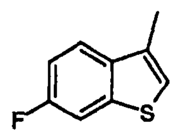 | 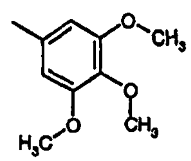 | H |

(continued)

| MDL # | D3 KI (nM) | n | X | Ar | R1 | R2 |
|---|---|---|---|---|---|---|
| 829211 | 16 | 2 | N | 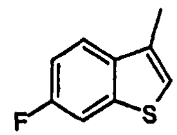 | 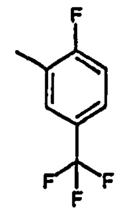 | H |
| 829212 | 49.9 | 2 | N | 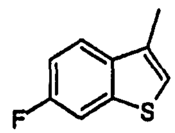 | 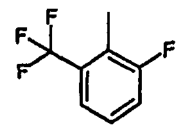 | H |
| 829213 | 181 | 2 | N | 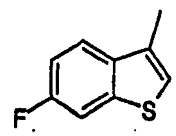 | 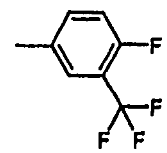 | H |
| 829214 | 14.1 | 2 | N | 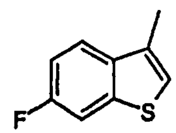 | 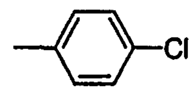 | H |
| 829215 | 6.59 | 2 | N | 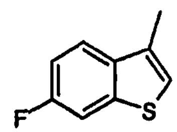 | 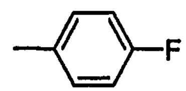 | H |
| 829216 | 0.178 | 2 | N | 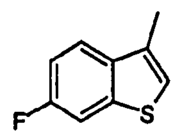 | 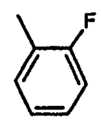 | H |

(continued)

| MDL # | D3 KI (nM) | n | X | Ar | R1 | R2 |
|---|---|---|---|---|---|---|
| 829217 | 3.51 | 2 | N | 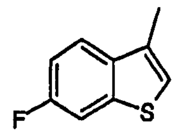 | 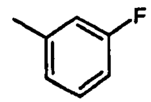 | H |
| 829218 | 0.165 | 2 | N | 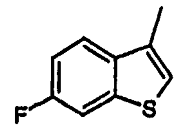 | 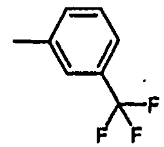 | H |
| 829219 | 0.0926 | 2 | N | 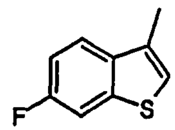 | 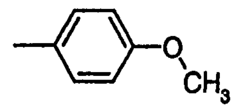 | H |
| 829220 | 18.1 | 2 | N | 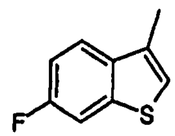 | 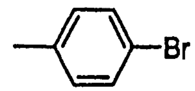 | H |
| 829221 | 0.0961 | 2 | N | 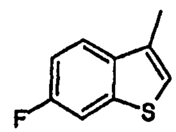 | 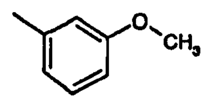 | H |
| 825154 | 35.5 | 1 | N | 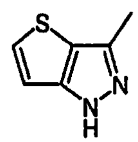 | 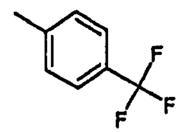 | H |
| 825155 | 84.8 | 1 | N | 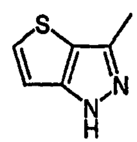 | 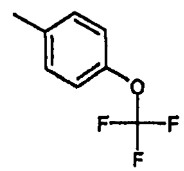 | H |

(continued)

| MDL # | D3 KI (nM) | n | X | Ar | R1 | R2 |
|---|---|---|---|---|---|---|
| 825156 | 63.2 | 1 | N | 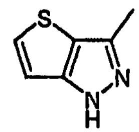 | 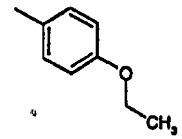 | H |
| 825160 | 202 | 1 | N | 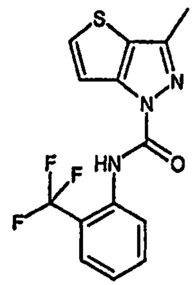 | 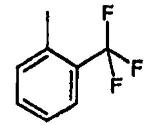 | H |
| 825167 | 374 | 1 | N | 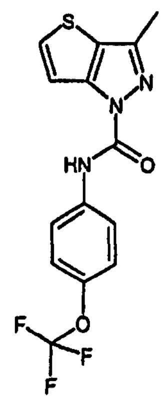 | 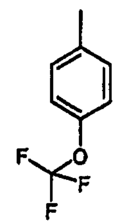 | H |

(continued)

| MDL # | D3 KI (nM) | n | X | Ar | R1 | R2 |
|---|---|---|---|---|---|---|
| 825168 | 47.4 | 1 | N | 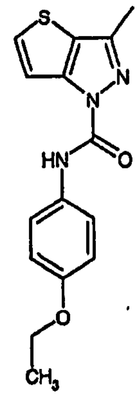 | 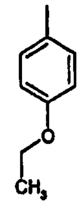 | H |
| 825170 | 136 | 1 | N | 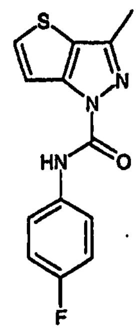 | 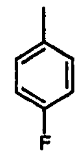 | H |
| 825171 | 496 | 1 | N | 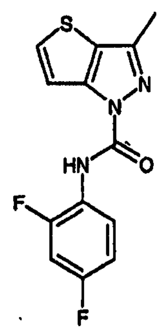 | 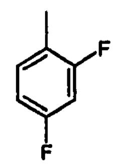 | H |

(continued)

| MDL # | D3 KI (nM) | n | X | Ar | R1 | R2 |
|---|---|---|---|---|---|---|
| 829369 | 44.2 | 1 | N | 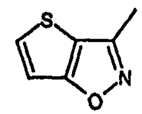 | 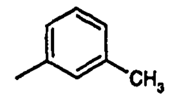 | H |
| 829370 | 4.91 | 1 | N | 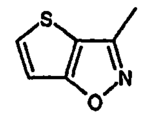 | 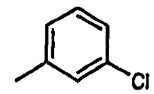 | H |
| 829371 | 55.2 | 1 | N | 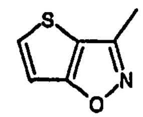 | 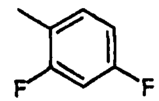 | H |
| 829372 | 31.9 | 1 | N | 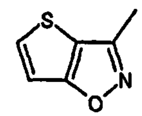 | 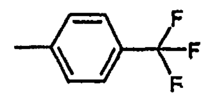 | H |
| 829373 | 14.9 | 1 | N | 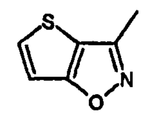 | 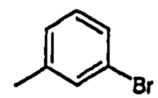 | H |
| 829374 | 103 | 1 | N | 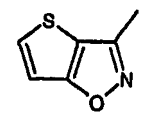 | 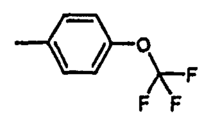 | H |
| 829375 | 176 | 1 | N | 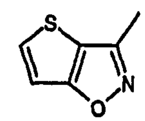 | 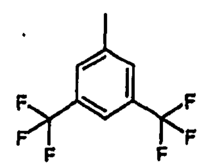 | H |
| 829377 | 87.9 | 1 | N | 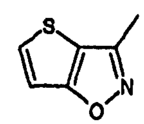 | 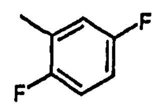 | H |

(continued)

| MDL # | D3 KI (nM) | n | X | Ar | R1 | R2 |
|---|---|---|---|---|---|---|
| 829378 | 184 | 1 | N | 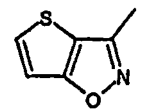 | 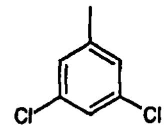 | H |
| 829379 | 54.9 | 1 | N | 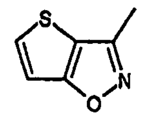 | 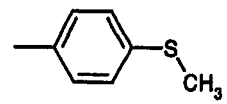 | H |
| 829380 | 26.9 | 1 | N | 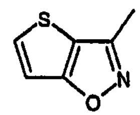 | 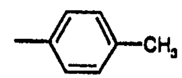 | H |
| 829381 | 46.5 | 1 | N | 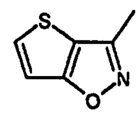 | 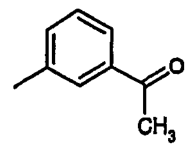 | H |
| 829382 | 42.7 | 1 | N | 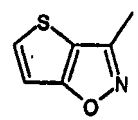 | 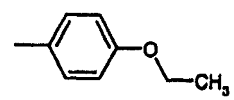 | H |
| 829383 | 102 | 1 | N | 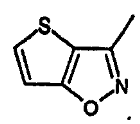 | 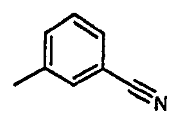 | H |
| 829384 | 190 | 1 | N | 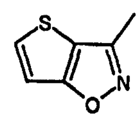 | 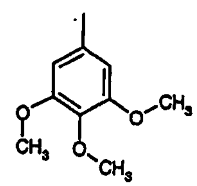 | H |

(continued)

| MDL # | D3 KI (nM) | n | X | Ar | R1 | R2 |
|---|---|---|---|---|---|---|
| 829385 | 142 | 1 | N | 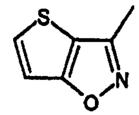 | 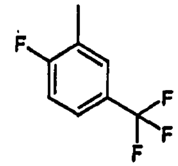 | H |
| 829387 | 114 | 1 | N | 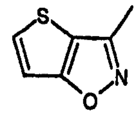 | 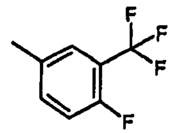 | H |
| 829388 | 44 | 1 | N | 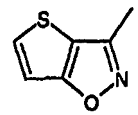 | 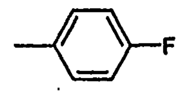 | H |
| 829389 | 50.5 | 1 | N | 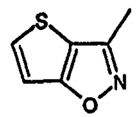 | 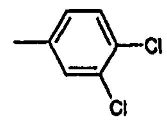 | H |
| 829390 | 37.5 | 1 | N | 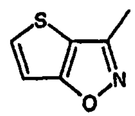 | 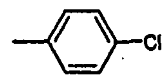 | H |
| 829392 | 487 | 1 | N | 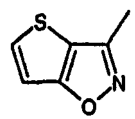 | 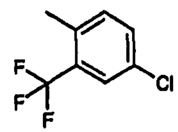 | H |
| 829393 | 190 | 1 | N | 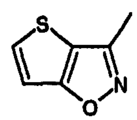 | 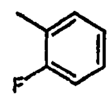 | H |

(continued)

| MDL # | D3 KI (nM) | n | X | Ar | R1 | R2 |
|---|---|---|---|---|---|---|
| 829394 | 77.1 | 1 | N | 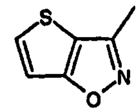 | 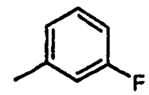 | H |
| 829395 | 148 | 1 | N | 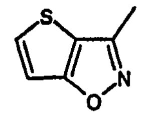 | 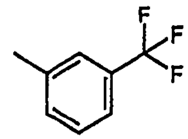 | H |
| 829396 | 121 | 1 | N | 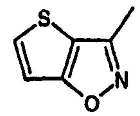 | 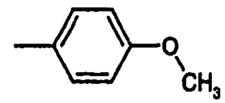 | H |
| 829397 | 86.6 | 1 | N | 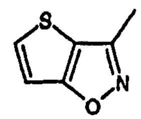 | 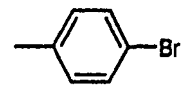 | H |
| 829398 | 44.1 | 1 | N | 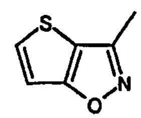 | 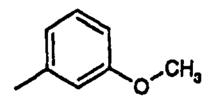 | H |
| 829407 | 452 | 1 | N | 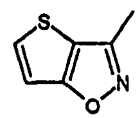 | 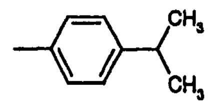 | H |
| 829409 | 379 | 2 | N | 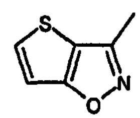 | 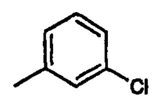 | H |
| 829411 | 670 | 2 | N | 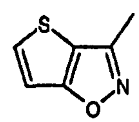 | 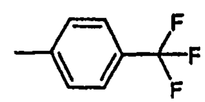 | H |

(continued)

| MDL # | D3 KI (nM) | n | X | Ar | R1 | R2 |
|---|---|---|---|---|---|---|
| 829412 | 142 | 2 | N | 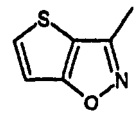 | 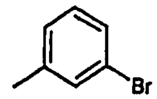 | H |
| 829414 | 546 | 2 | N | 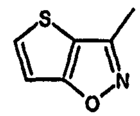 | 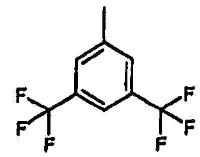 | H |
| 829415 | 345 | 2 | N | 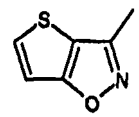 | 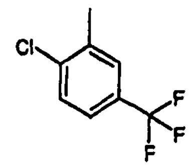 | H |
| 829416 | 327 | 2 | N | 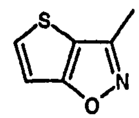 | 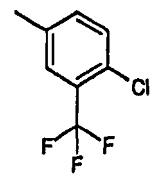 | H |
| 829417 | 338 | 2 | N | 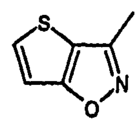 | 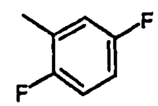 | H |
| 829418 | 52.9 | 2 | N | 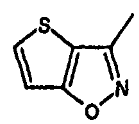 | 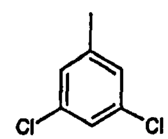 | H |
| 829419 | 138 | 2 | N | 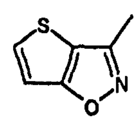 | 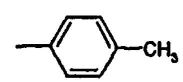 | H |

(continued)

| MDL # | D3 KI (nM) | n | X | Ar | R1 | R2 |
|---|---|---|---|---|---|---|
| 829420 | 233 | 2 | N | 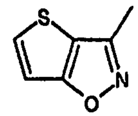 | 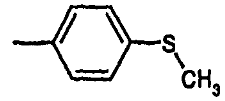 | H |
| 829421 | 173 | 2 | N | 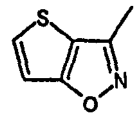 | 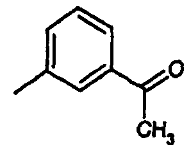 | H |
| 829422 | 466 | 2 | N | 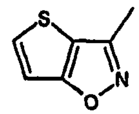 | 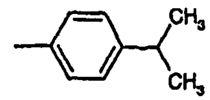 | H |
| 829425 | 539 | 2 | N | 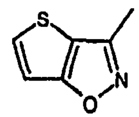 | 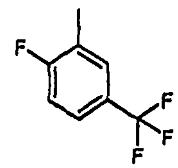 | H |
| 829427 | 459 | 2 | N | 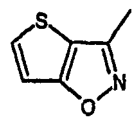 | 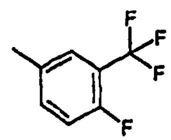 | H |
| 829428 | 747 | 2 | N | 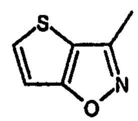 | 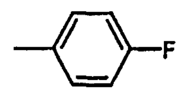 | H |
| 829429 | 111 | 2 | N | 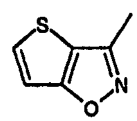 | 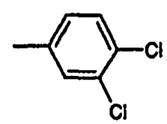 | H |

(continued)

| MDL # | D3 KI (nM) | n | X | Ar | R1 | R2 |
|---|---|---|---|---|---|---|
| 829431 | 153 | 2 | N | 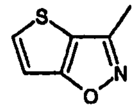 | 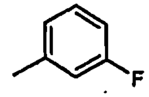 | H |
| 829432 | 226 | 2 | N | 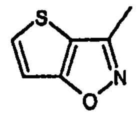 | 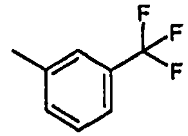 | H |
| 829433 | 216 | 2 | N | 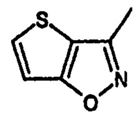 | 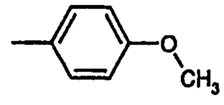 | H |
| 829434 | 197 | 2 | N | 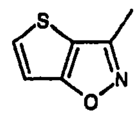 | 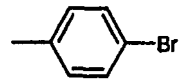 | H |
| 829873 | 27.7 | 1 | CH | 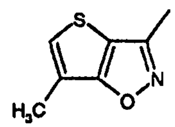 | 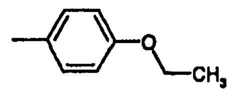 | H |
| 829874 | 360 | 1 | CH | 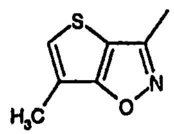 | 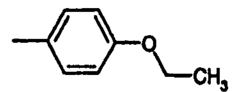 | 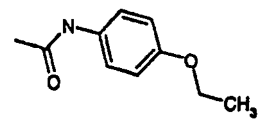 |
| 829875 | 94.7 | 1 | CH | 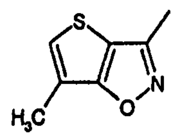 | 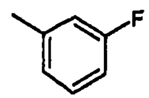 | 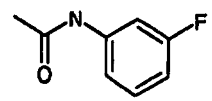 |
| 829876 | 77.1 | 1 | CH | 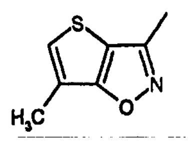 | 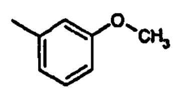 | 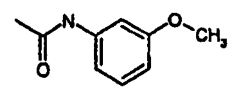 |

(continued)

| MDL # | D3 KI (nM) | n | X | Ar | R1 | R2 |
|---|---|---|---|---|---|---|
| 829878 | 8.78 | 1 | CH | 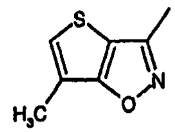 | 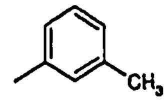 | H |
| 829879 | 354 | 1 | CH | 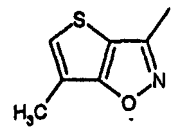 | 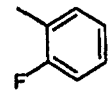 | H |
| 829882 | 143 | 1 | CH | 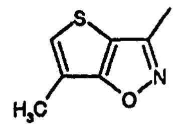 | 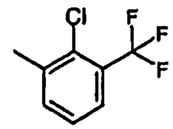 | H |
| 830517 | 9.04 | 1 | CH | 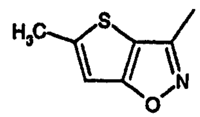 | 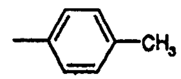 | H |
| 830518 | 9.16 | 1 | CH | 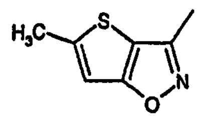 | 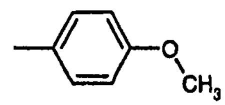 | H |

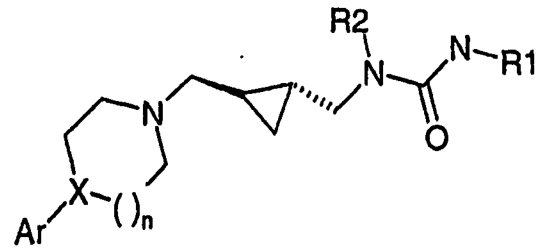

| MDL# | D3 KI (nM) | Chirality | n | X | Ar | R1 | R2 |
|---|---|---|---|---|---|---|---|
| 830389 | 2.97 | Racemic | 2 | N | 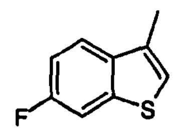 | 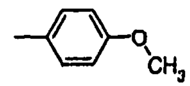 | $-CH_2CH=CH_2$ |
| 830400 | 41.3 | Racemic | 2 | N | 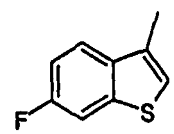 | 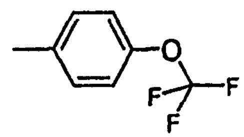 | Bn |
| 830401 | 29.7 | Racemic | 2 | N | 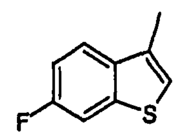 | 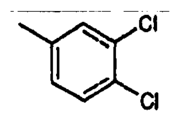 | Bn |
| 830408 | 3.43 | Racemic | 2 | N | 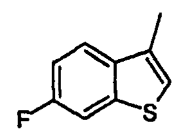 | 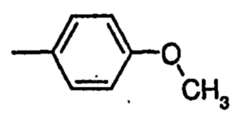 | H |
| 830409 | 47.4 | Racemic | 2 | N | 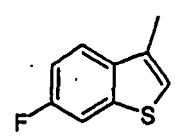 | 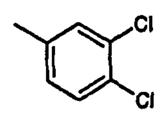 | H |

(continued)

| MDL# | D3 KI (nM) | Chirality | n | X | Ar | R1 | R2 |
|---|---|---|---|---|---|---|---|
| 830410 | 137 | Racemic | 2 | N | 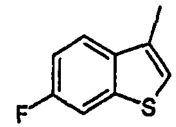 | 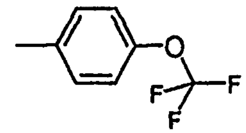 | H |
| 832786 | 61.56 | R,R | 1 | N | 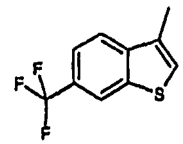 | 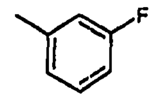 | H |
| 832810 | 33.5 | R,R | 1 | N | 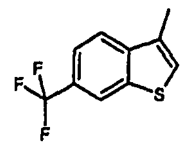 | 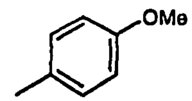 | H |
| 832811 | 18.2 | R,R | 1 | N | 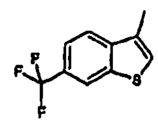 | 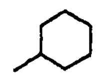 | H |
| 832812 | 39.5 | R,R | 1 | N | 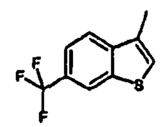 | 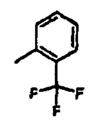 | H |
| 832813 | 50.84 | R,R | 1 | N | 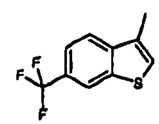 | 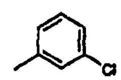 | H |
| 832819 | 46.24 | R,R | 1 | N | 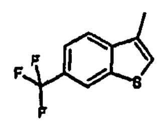 | 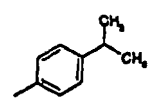 | H |

(continued)

| MDL# | D3 KI (nM) | Chirality | n | X | Ar | R1 | R2 |
|---|---|---|---|---|---|---|---|
| 832820 | 3.9 | R,R | 1 | N | 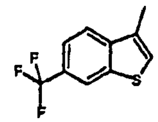 | 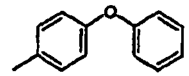 | H |
| 833203 | 18.19 | R,R | 1 | N | 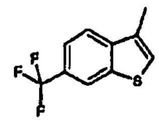 | 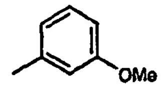 | H |
| 831498 | 43.6 | Racemic | 1 | CH | 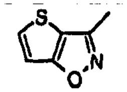 | t-Bu | H |

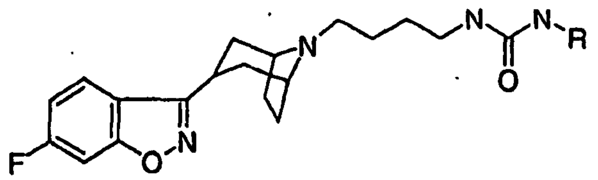

| MDL # | D3 KI (nM) | R |
|---|---|---|
| 817228 | 179 | 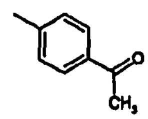 |
| 817229 | 365 | 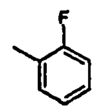 |
| 817230 | 182 | 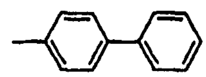 |
| 817231 | 433 | 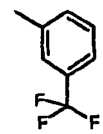 |
| 817233 | 146 | 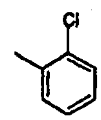 |
| 817234 | 256 | 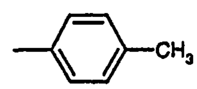 |

## Claims

**1.** A compound of the formula (I):

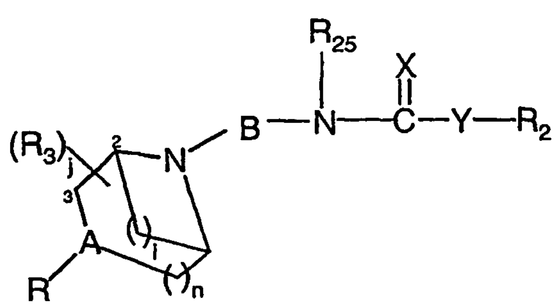

I

wherein

A is CH or N;
Y is O or $NR_1$

wherein $R_1$ is

a) hydrogen;
b) $C_1$-$C_6$alkyl; or
c)

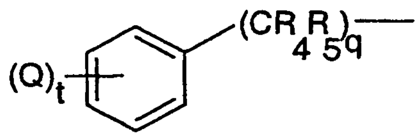

wherein
each Q is independently hydrogen, $C_1$-$C_6$alkyl, halogen or trifluoromethyl;
each $R_4$, and $R_5$ is independently hydrogen or $C_1$-$C_6$alkyl;
t is 0 or 1; and
q is 0 or 1;

j is 0, 1 or 2;
n is 1 or 2;
when n is 1, i is 0 or 2;
when n is 2, i is 0;
$R_3$ is hydrogen, $C_1$-$C_6$alkyl or

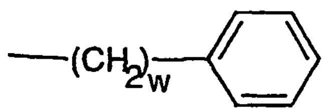

wherein w is 1, 2 or 3;
X is O or S;
$R_{25}$ is hydrogen or

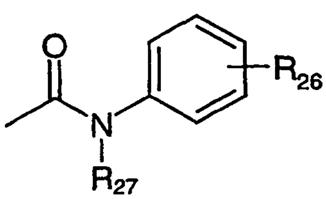

wherein $R_{27}$ is hydrogen or $C_1$-$C_6$alkyl and $R_{26}$ is hydrogen, $C_1$-$C_6$alkoxy, halogen or $C_1$-$C_6$alkyl that is unsaturated or saturated;
-B- is a group selected from (a) - (e):

(a) -($CR_{21}R_{22}$)m-
(b)

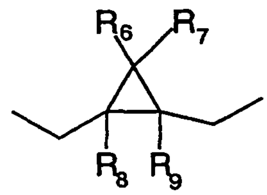

(c)

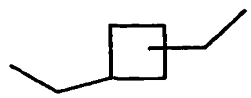

(d)

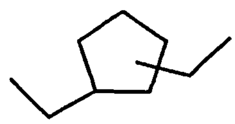

(e)

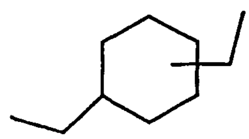

wherein
m is 3, 4, 5, or 6;
each $R_{21}$ and $R_{22}$ is independently hydrogen, $C_1$-$C_6$alkyl or
$-(CH_2)_aOH$ wherein a is 0, 1 or 2;
$R_6$, $R_7$, $R_8$ and $R_9$ are each independently hydrogen, halogen or $C_1$-$C_3$alkyl with the proviso that when $R_8$ is $C_1$-$C_3$alkyl, $R_9$ is not $C_1$-$C_3$alkyl;

R is a group selected from (a) - (c):

a)

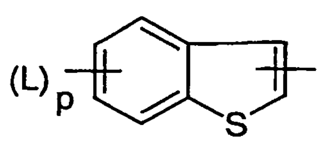

b)

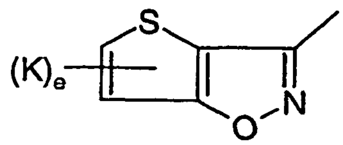

c)

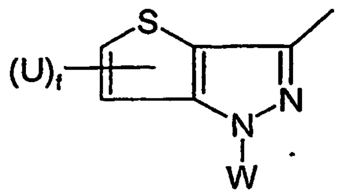

wherein

each L is independently hydrogen, $C_1$-$C_6$alkyl, halogen or trifluoromethyl;
each U is independently hydrogen, $C_1$-$C_6$alkyl, CHO, halogen, $-(CH_2)_bOH$, or $-(CH_2)_bOC_1$-$C_6$alkyl wherein b is 1 or 2;
each K is independently hydrogen, $C_1$-$C_6$alkyl, CHO, halogen, $-(CH_2)_bOH$, or $-(CH_2)_bOC_1$-$C_6$alkyl wherein b is as hereinbefore defined;
W is hydrogen or

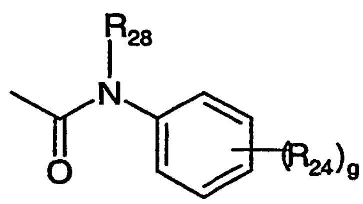

wherein $R_{28}$ is hydrogen or $C_1$-$C_6$alkyl;
each $R_{24}$ is independently trifluoromethyl, trifluoromethoxy, $C_1$-$C_6$alkoxy or halogen; and
g is 0, 1 or 2;
p is 0, 1 or 2;
e is 0, 1 or 2;
f is 0, 1, or 2;

$R_2$ is a group selected from (a) - (t):

(a)

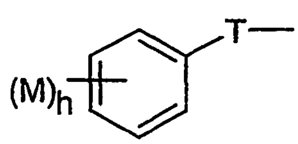

(b)

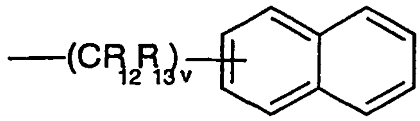

(c)

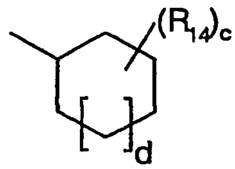

(d)

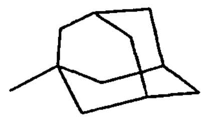

(e)

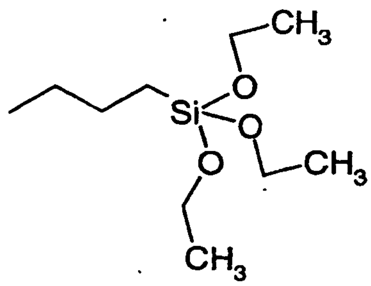

(f) $-C_1-C_{12}alkyl$
(g) $-(CR_{15}R_{16})_s-CO_2C_1-C_6alkyl$
(h)

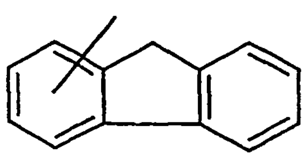

(i)

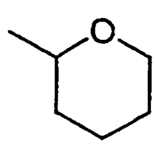

(j)

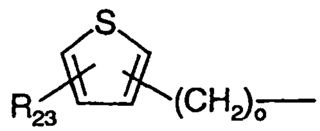

(k)

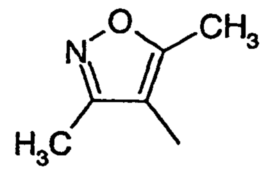

(l) $-CH_2CH_2SO_2CH_3$
(m) $-(CR_{17}R_{18})_r-OC_1-C_2$alkyl
(n) $-CH_2CH_2OPh$
(o) $-(CR_{19}R_{20})_qCZ_3$
(p) $-CO_2Ph$
(q) $-COCV_3$
(r)

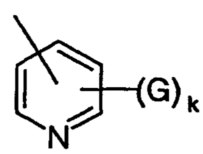

(s)

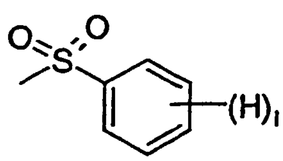

(t)

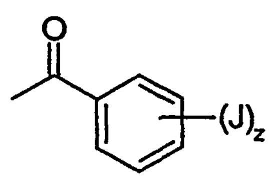

wherein

-T- is selected from (i) or (ii):

(i) $-(CR_{10}R_{11})_u-$

wherein
u is 0, 1 or 2; and
each $R_{10}$ and $R_{11}$ is independently hydrogen or $C_1$-$C_6$alkyl;
(ii)

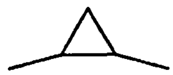 ;

each M, G, H and J is independently selected from:

(1) hydrogen;
(2) halogen;
(3) $C_1$-$C_6$alkyl;
(4) $C_1$-$C_6$alkoxy;
(5) phenoxy;
(6) phenyl;
(7) trifluoromethyl;
(8) trifluoromethoxy;
(9) $-CO_2-R_{19}$ wherein $R_{19}$ is hydrogen or $C_1$-$C_6$alkyl;
(10) $-COC_1$-$C_6$alkyl;
(11) hydroxy;
(12) $-(CH_2)-OR_{20}$ wherein $R_{20}$ is hydrogen or $C_1$-$C_6$alkyl;
(13) $-C(CH_2)CH_3$;
(14) $-NO_2$;
(15) $-SCH_3$;
(16) benzyloxy; and
(17) -CN;

each $R_{12}$ and $R_{13}$ is independently hydrogen or $C_1$-$C_6$alkyl;

v is 0 or 1;
$R_{14}$ is hydrogen or $C_1$-$C_4$alkyl;
c is 0, 1 or 2;
d is 0 or 1;

each $R_{15}$ and $R_{16}$ is independently hydrogen or $C_1$-$C_6$alkyl;

s is 0, 1, 2, 3, 4 or 5;
o is 1 or 2;
each $R_{17}$ and $R_{18}$ is independently hydrogen, $C_1$-$C_6$alkyl or

$CO_2C_1$-$C_2$alkyl;
r is 2 or 3;

each $R_{19}$ and $R_{20}$ is independently hydrogen or

$C_1$-$C_2$ alkyl;
$R_{23}$ is hydrogen or $C_1$-$C_4$alkyl;
Z is chlorine or fluorine;
q is 0 or 1;
V is chlorine or fluorine; and
h, k, l, and z are 0, 1, 2, or 3.

**2.** A compound according to claim 1 wherein X is O, Y is NH and -B- is group (a).

**3.** A compound according to claim 2 wherein $R_2$ is group (a).

**4.** A compound according to claim 3 wherein m is 4, M is hydrogen, $C_1$-$C_6$alkyl, $C_1$-$C_6$alkoxy, or phenyl and T is (i) wherein u is 0 or 1.

**5.** A compound according to claim 2 wherein $R_2$ is group (c).

**6.** A compound according to claim 5 wherein wherein m is 4, d is 1, and $R_{14}$ is $C_1$-$C_6$alkyl.

**7.** A compound according to claim 1 wherein X is O, Y is NH and -B- is group (b).

**8.** A compound according to claim 6 wherein $R_2$ is group (a).

**9.** A compound according to claim 7 wherein $R_6$, $R_7$, $R_8$ or $R_9$ are hydrogen; M is hydrogen, $C_1$-$C_6$alkyl, $C_1$-$C_6$alkoxy or phenyl, and T is 0 or 1.

**10.** A compound according to claim 6 wherein $R_2$ is group (c).

**11.** A compound according to claim 9 wherein $R_6$, $R_7$, $R_8$ or $R_9$ are hydrogen, d is 1, and $R_{14}$ is $C_1$-$C_6$alkyl

**12.** The compound of claim 1 which is 1-(4-methoxy-phenyl)-3-{4-[4-(6-trifluoromethyl-benzo[b]thiophen-3-yl)-piperazin-1-yl]-butyl}-urea.

**13.** The compound of claim 1 which is 1-p-tolyl-3-{4-[4-(6-trifluoromethyl-benzo[b]thiophen-3-yl)-piperazin-1-yl]-butyl}-urea.

**14.** The compound of claim 1 which is 1-{4-[4-(2,6-difluoro-benzo[b]thiophen-3-yl)-piperazin-1-yl]-butyl}-3-p-tolyl-urea.

**15.** The compound of claim 1 which is 1-{4-[4-(2-chloro-6-fluoro-benzo[b]thiophen-3-yl)-piperazin-1-yl]-butyl}-3-(4-chloro-phenyl)-urea.

**16.** The compound of claim 1 which is 1-(4-acetyl-phenyl)-3-[4-(4-thieno[2,3-d]isoxazol-3-yl-piperidin-1-yl)-butyl]-urea.

**17.** The compound of claim 1 which is 1-(4-ethoxy-phenyl)-3-[4-(4-thieno[2,3-d]isoxazol-3-yl-piperidin-1-yl)-butyl]-urea.

**18.** The compound of claim 1 which is 1-(4-ethoxy-phenyl)-3-{4-[4-(6-fluoro-benzo[b]thiophen-3-yl)-[1,4]diazepan-1-yl]-butyl}-urea.

**19.** The compound of claim 1 which is 1-{4-[4-(6-fluoro-benzo[b]thiophen-3-yl)-[1,4]diazepan-1-yl]-butyl}-3-(2-fluoro-phenyl)-urea.

**20.** The compound of claim 1 which is 1-{4-[4-(6-fluoro-benzo[b]thiophen-3-yl)-[1,4]diazepan-1-yl]-butyl}-3-(3-trifluoromethyl-phenyl)-urea.

**21.** The compound of claim 1 which is 1-{4-[4-(6-fluoro-benzo[b]thiophen-3-yl)-[1,4]diazepan-1-yl]-butyl}-3-(4-methoxy-phenyl)-urea.

**22.** The compound of claim 1 which is 1-(3-Imidazol-1-yl-propyl)-3-{(1R, 2R)-2-[4-(6-trifluoromethyl-benzo[b]thiophen-3-yl)-piperazin-1-ylmethyl]-cyclopropylmethyl}-urea.

**23.** Use of a compound of claim 1 for the manufacture of a medicament for treating conditions or disorders of the central nervous system, wherein the central nervous system disorder is selected from Psychotic Disorders, Substance Dependence, Substance Abuse, Dyskinetic Disorders, Dementia, Anxiety Disorders, Sleep Disorders, Circadium Rhythm Disorders, Mood Disorders and Nausea.

**24.** Use of a compound of claim 1 for the manufacture of a medicament for treating Schizophrenia.

**25.** A pharmaceutical composition comprising an effective amount of a compound of claim 1 with a pharmaceutically-acceptable carrier or diluent.

**26.** A pharmaceutical composition comprising an effective amount of a compound of claim 1 with a pharmaceutically-acceptable carrier or diluent in conjunction with one or more dopamine $D_1$, $D_2$, $D_4$, $D_5$ or 5HT receptor antagonists.

**27.** A depot pharmaceutical composition, which comprises a pharmaceutically acceptable carrier and a therapeutically effective amount of the compound of claim 1, wherein the compound contains an acylated hydroxy group, or an acylated amino group.

**28.** The depot pharmaceutical composition of claim 27, wherein the hydroxy group is acylated, or the amino group is acylated with $(C_4\text{-}C_{18})$alkanoyl group or a $(C_4\text{-}C_{18})$alkoxycarbonyl group.

**29.** The composition of claim 25 which contains a pharmaceutically acceptable oil.

**30.** The composition of claim 28 wherein the oil is selected from the group consisting of coconut oil, peanut oil, sesame oil, cotton seed oil, corn oil, soybean oil, olive oil, and synthetic esters of fatty acids and polyfunctional alcohols.

**31.** The depot pharmaceutical composition of claim 28, wherein the composition provides a long acting antipsychotic effect upon injection into a mammal.

**32.** The depot pharmaceutical composition of claim 29, wherein the composition provides a long acting antipsychotic effect upon injection into a mammal.

**33.** The depot pharmaceutical composition of claim 30 wherein the composition provides a long acting antipsychotic effect upon injection into a mammal.

**34.** A compound of claim 1 wherein one or more of the atoms contained therein is a radionuclide.

**35.** A compound of claim 1 wherein R is group (a) with a radiolabeled $^{14}C$ in the 3-position of the benzo[b]thiophene ring system, L is trifluoromethyl, p is 1, $R_3$ is hydrogen, n is 1, i is 0, and A is N.

**36.** A process for preparing a compound of claim 1 wherein Y is N which comprises: reacting a compound of formula (II)

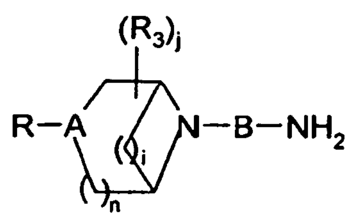

(II)

wherein R, $R_3$, j, n, i, B and A are as defined in formula I of claim 1 with a compound of formula (III)

$X{=}C{=}N\text{-}R_2$ (III)

wherein X and $R_2$ are as defined in formula I of claim 1.

**37.** A process for preparing a compound of formula I wherein Y is O which comprises: reacting a compound of formula (II)

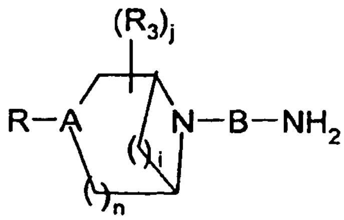

(II)

wherein R, $R_3$, j, n, i, B and A are as defined in formula I of claim 1 with a compound of formula IV

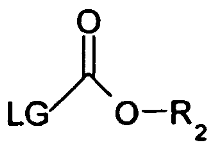

(IV) .

wherein "LG" is a suitable leaving group selected from bromine, chlorine or iodine and $R_2$ is as defined in formula I of claim 1.

**38.** A process for preparing compounds of formula I which comprises reacting a compound of formula (V)

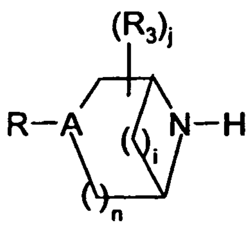

(V)

wherein $R_3$, j, R, A, i and n are as defined in formula I of claim 1 with a compound of formula (VI)

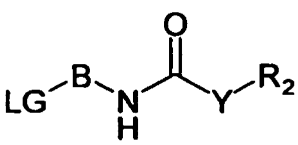

(VI)

wherein "LG" is a suitable leaving selected from chlorine, bromine, iodine or mesyl and B, Y and $R_2$ are defined in formula I of claim 1.

**39.** A compound according to claim 1 wherein R is group (a).

**40.** A compound according to claim 1 wherein R is group (b).

**41.** A compound according to claim 1 wherein R is group (c).

**42.** Use of a compound of claim 1 for the manufacture of a medicament for treating renal dysfunction.

## Patentansprüche

**1.** Verbindung der Formel (I):

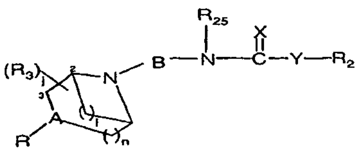

I

worin

A für CH oder N steht;
Y für 0 oder $NR_1$,
worin $R_1$

a) Wasserstoff;
b) $C_1$-$C_6$-Alkyl oder
c)

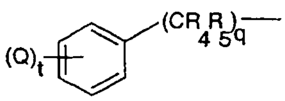

worin

Q jeweils unabhängig voneinander für Wasserstoff, $C_1$-$C_6$-Alkyl, Halogen oder Trifluormethyl steht;
$R_4$ und $R_5$ jeweils unabhängig voneinander für Wasserstoff oder $C_1$-$C_6$-Alkyl stehen;
t für 0 oder 1 steht und
q für 0 oder 1 steht;

bedeutet;

steht;
j für 0, 1 oder 2 steht;
n für 1 oder 2 steht;
i für 0 oder 2 steht, wenn n für 1 steht;
i für 0 steht, wenn n für 2 steht;
$R_3$ für Wasserstoff, $C_1$-$C_6$-Alkyl oder

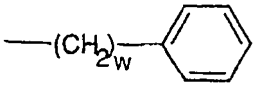

worin w für 1, 2 oder 3 steht;
steht;

X für O oder S steht;
$R_{25}$ für Wasserstoff oder

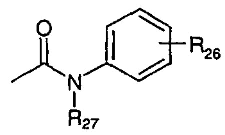

worin $R_{27}$ Wasserstoff oder $C_1$-$C_6$-Alkyl bedeutet und $R_{26}$ Wasserstoff, $C_1$-$C_6$-Alkoxy, Halogen oder gesättigtes oder ungesättigtes $C_1$-$C_6$-Alkyl bedeutet;
steht;
-B- für eine unter (a) - (e) ausgewählte Gruppe steht:

(a) - $(CR_{21}R_{22})_m$-
(b)

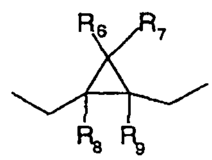

(c)

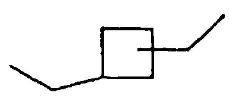

(d)

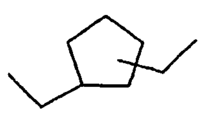

(e)

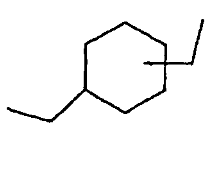

worin

m für 3, 4, 5 oder 6 steht;
$R_{21}$ und $R_{22}$ jeweils unabhängig voneinander für Wasserstoff, $C_1$-$C_6$-Alkyl oder - $(CH_2)_aOH$, worin a 0, 1 oder 2 bedeutet, stehen;
$R_6$, $R_7$, $R_8$ und $R_9$ jeweils unabhängig voneinander für Wasserstoff, Halogen oder $C_{1-3}$-Alkyl stehen,

mit der Maßgabe, daß $R_9$ nicht für $C_{1-3}$-Alkyl steht, wenn $R_8$ für $C_{1-3}$-Alkyl steht;

R für eine unter (a) - (c) ausgewählte Gruppe steht:

a)

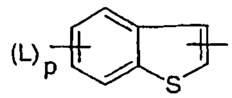

b)

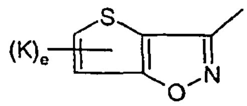

c)

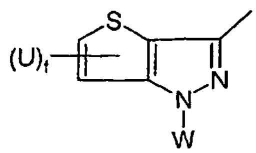

worin

L jeweils unabhängig voneinander für Wasserstoff, $C_1$-$C_6$-Alkyl, Halogen oder Trifluormethyl steht;
U jeweils unabhängig voneinander für Wasserstoff, $C_1$-$C_6$-Alkyl, CHO, Halogen, $-(CH_2)_bOH$ oder $-(CH_2)_bO$-$C_1$-$C_6$-Alkyl, worin b 1 oder 2 bedeutet, steht;
K jeweils unabhängig voneinander für Wasserstoff, $C_1$-$C_6$-Alkyl, CHO, Halogen, $(CH_2)_bOH$ oder $-(CH_2)_bO$-$C_1$-$C_6$-Alkyl, worin b die oben angegebene Bedeutung besitzt, steht;
W für Wasserstoff oder

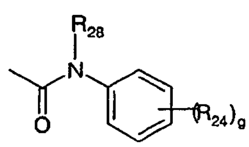

worin $R_{28}$ Wasserstoff oder $C_1$-$C_6$-Alkyl bedeutet;
$R_{24}$ jeweils unabhängig voneinander Trifluormethyl, Trifluormethoxy, $C_1$-$C_6$-Alkoxy oder Halogen bedeutet und
g 0, 1 oder 2 bedeutet;

steht;
p für 0, 1 oder 2 steht;
e für 0, 1 oder 2 steht;
f für 0, 1 oder 2 steht;

$R_2$ für eine unter (a) - (t) ausgewählte Gruppe steht:

(a)

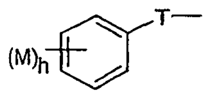

(b)

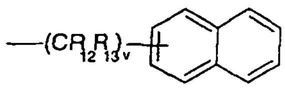

(c)

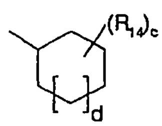

(d)

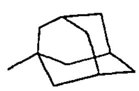

(e)

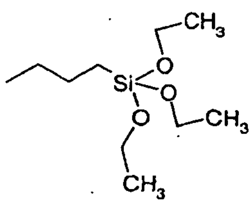

(f) -$C_{1-12}$-Alkyl
(g) - $(CR_{15}R_{16})_s$-$CO_2$-$C_{1-6}$-Alkyl
(h

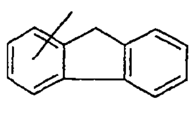

(i)

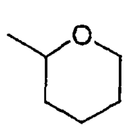

(j)

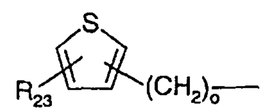

(k)

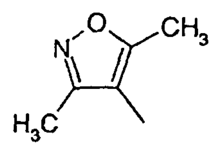

(l)-$CH_2CH_2SO_2CH_3$
(m) - $(CR_{17}R_{18})_r$-$OC_1$-$C_2$-Alkyl
(n) - $CH_2CH_2OPh$
(o) - $(CR_{19} R_{20})_qCZ_3$
(p) -$CO_2Ph$
(q) -$COCV_3$
(r)

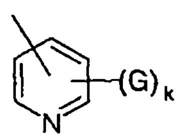

(s)

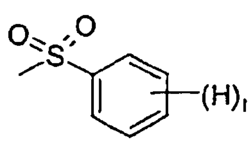

(t)

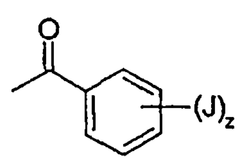

worin

-T- unter (i) oder (ii) ausgewählt ist:

(i) -$(CR_{10}R_{11})_u$-
worin

u für 0, 1 oder 2 steht und
$R_{10}$ und $R_{11}$ jeweils unabhängig voneinander für Wasserstoff oder $C_{1-6}$-Alkyl stehen;

(ii)

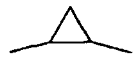;

M, G, H und J jeweils unabhängig voneinander unter:

(1) Wasserstoff;
(2) Halogen;
(3) $C_{1-6}$-Alkyl ;
(4) $C_{1-6}$-Alkoxy;
(5) Phenoxy;
(6) Phenyl;
(7) Trifluormethyl;
(8) Trifluormethoxy;
(9) $-CO_2-R_{19}$, worin $R_{19}$ für Wasserstoff oder $C_{1-6}$-Alkyl steht;
(10) -CO-$C_{1-6}$-Alkyl ;
(11) Hydroxy;
(12) - $-(CH_2)-OR_{20}$, worin $R_{20}$ für Wasserstoff oder $C_{1-6}$-Alkyl steht;
(13) $-C\ (CH_2)CH_3$;
(14) $-NO_2$;
(15) $-SCH_3$;
(16) Benzyloxy und
(17) -CN

ausgewählt sind;
$R_{12}$ und $R_{13}$ jeweils unabhängig voneinander für Wasserstoff oder $C_{1-6}$-Alkyl stehen;
v für 0 oder 1 steht;
$R_{14}$ für Wasserstoff oder $C_{1-4}$-Alkyl steht;
c für 0, 1 oder 2 steht;
d für 0 oder 1 steht;
$R_{15}$ und $R_{16}$ jeweils unabhängig voneinander für Wasserstoff oder $C_{1-6}$-Alkyl stehen;
s für 0, 1, 2, 3, 4 oder 5 steht;
o für 1 oder 2 steht;
$R_{17}$ und $R_{18}$ jeweils unabhängig voneinander für Wasserstoff, $C_{1-6}$-Alkyl oder $CO_2$-$C_{1-2}$-Alkyl stehen;
r für 2 oder 3 steht;
$R_{19}$ und $R_{20}$ jeweils unabhängig voneinander für Wasserstoff oder $C_{1-2}$-Alkyl stehen;
$R_{23}$ für Wasserstoff oder $C_{1-4}$-Alkyl steht;
Z für Chlor oder Fluor steht;
q für 0 oder 1 steht;
V für Chlor oder Fluor steht und
h, k, 1 und z für 0, 1, 2 oder 3 stehen.

**2.** Verbindung nach Anspruch 1, worin X für O steht, Y für NH steht und -B- für die Gruppe (a) steht.

**3.** Verbindung nach Anspruch 2, worin $R_2$ für die Gruppe (a) steht.

**4.** Verbindung nach Anspruch 3, worin m für 4 steht, M für Wasserstoff, $C_{1-6}$-Alkyl, $C_{1-6}$-Alkoxy oder Phenyl steht und T für (i), worin u 0 oder 1 bedeutet, steht.

**5.** Verbindung nach Anspruch 2, worin $R_2$ für die Gruppe (c) steht.

**6.** Verbindung nach Anspruch 5, worin m für 4 steht, d für 1 steht und $R_{14}$ für $C_{1-6}$-Alkyl steht.

**7.** Verbindung nach Anspruch 1, worin X für O steht, Y für NH steht und -B- für die Gruppe (b) steht.

**8.** Verbindung nach Anspruch 6, worin $R_2$ für die Gruppe (a) steht.

**9.** Verbindung nach Anspruch 7, worin $R_6$, $R_7$, $R_8$ oder $R_9$ für Wasserstoff stehen; M für Wasserstoff, $C_{1-6}$-Alkyl, $C_{1-6}$-Alkoxy oder Phenyl steht und T für 0 oder 1 steht.

**10.** Verbindung nach Anspruch 6, worin $R_2$ für die Gruppe (c) steht.

**11.** Verbindung nach Anspruch 9, worin $R_6$, $R_7$, $R_8$ oder $R_9$ für Wasserstoff stehen, d für 1 steht und $R_{14}$ für $C_{1-6}$-Alkyl steht.

**12.** Verbindung nach Anspruch 1, bei der es sich um 1-(4-Methoxyphenyl)-3-{4-[4-(6-trifluormethylbenzo-[b] thiophen-3-yl)piperazin-1-yl]butyl}harnstoff handelt.

**13.** Verbindung nach Anspruch 1, bei der es sich um 1-p-Tolyl-3-{4-[4-(6-trifluormethylbenzo[b]thiophen-3-yl)piperazin-1-yl]butyl}harnstoff handelt.

**14.** Verbindung nach Anspruch 1, bei der es sich um 1-{4-[4-(2,6-Difluorbenzo[b]thiophen-3-yl)piperazin-1-yl]butyl}-3-p-tolylharnstoff handelt.

**15.** Verbindung nach Anspruch 1, bei der es sich um 1-{4-[4-(2-Chlor-6-fluorbenzo[b]thiophen-3-yl)-piperazin-1-yl]butyl}-3-(4-chlorphenyl)harnstoff handelt.

**16.** Verbindung nach Anspruch 1, bei der es sich um 1-(4-Acetylphenyl) -3- [4- (4-thieno [2, 3-d] isoxazol-3-ylpiperidin-1-yl)butyl]harnstoff handelt.

**17.** Verbindung nach Anspruch 1, bei der es sich um 1-(4-Ethoxyphenyl)-3-[4-(4-thieno[2,3-d]isoxazol-3-ylpiperidin-1-yl)butyl]harnstoff handelt.

**18.** Verbindung nach Anspruch 1, bei der es sich um 1-(4-Ethoxyphenyl)-3-{4-[4-(6-fluorbenzo[b]thiophen-3-yl) [1,4] diazepan-1-yl]butyl}harnstoff handelt.

**19.** Verbindung nach Anspruch 1, bei der es sich um 1-{4- [4-(6-fluorbenzo [b] thiophen-3-yl) [1, 4] diazepan-1-yl]butyl}-3-(2-fluorphenyl)harnstoff handelt.

**20.** Verbindung nach Anspruch 1, bei der es sich um 1-{4- [4- (6-fluorbenzo [b] thiophen-3-yl) [1, 4] diazepan-1-yl] butyl}-3-(3-trifluormethylphenyl) harnstoff handelt.

**21.** Verbindung nach Anspruch 1, bei der es sich um 1-{4-[4-(6-fluorbenzo[b]thiophen-3-yl) [1,4] diazepan-1-yl]butyl}-3-(4-methoxyphenyl)harnstoff handelt.

**22.** Verbindung nach Anspruch 1, bei der es sich um 1-(3-Imidazol-1-ylpropyl)-3-{(1R,2R)-2-[4-(6-trifluormethylbenzo [b]thiophen-3-yl)piperazin-1-yl-methyl]cyclopropylmethyl}harnstoff handelt.

**23.** Verwendung einer Verbindung nach Anspruch 1 zur Herstellung eines Arzneimittels zur Behandlung von Zuständen oder Störungen des Zentralnervensystems, bei der die Störung des Zentralnervensystems unter psychotischen Störungen, Substanzabhängigkeit, Substanzmißbrauch, dyskinetischen Störungen, Demenz, Angststörungen, Schlafstörungen, Störungen des 24-Stunden-Rhythmus, Stimmungsstörungen und Übelkeit ausgewählt ist.

**24.** Verwendung einer Verbindung nach Anspruch 1 zur Herstellung eines Arzneimittels zur Behandlung von Schizophrenie.

**25.** Pharmazeutische Zusammensetzung, enthaltend eine wirksame Menge einer Verbindung nach Anspruch 1 mit einem pharmazeutisch verträglichen Träger oder Verdünnungsmittel.

**26.** Pharmazeutische Zusammensetzung, enthaltend eine wirksame Menge einer Verbindung nach Anspruch 1 mit einem pharmazeutisch verträglichen Träger oder Verdünnungsmittel in Verbindung mit einem oder mehreren Dopamin-$D_1$-, -$D_2$-, -$D_4$-, -$D_5$- oder 5HT-Rezeptorantagonisten.

**27.** Pharmazeutische Depotzusammensetzung, die einen pharmazeutisch unbedenklichen Träger und eine therapeutisch wirksame Menge der Verbindung nach Anspruch 1 enthält, wobei die Verbindung eine acylierte Hydroxygruppe oder eine acylierte Aminogruppe enthält.

**28.** Pharmazeutische Depotzusammensetzung nach Anspruch 27, bei der die Hydroxygruppe bzw. die Aminogruppe mit einer ($C_4$-$C_{18}$)-Alkanoylgruppe oder einer ($C_4$-$C_{18}$)-Alkoxycarbonylgruppe acyliert ist.

**29.** Zusammensetzung nach Anspruch 25, die ein pharmazeutisch unbedenkliches Öl enthält.

**30.** Zusammensetzung nach Anspruch 29, bei der das Öl aus der Gruppe bestehend aus Kokosnußöl, Erdnußöl, Sesamöl, Baumwollsaatöl, Maisöl, Sojabohnenöl, Olivenöl und synthetischen Estern von Fettsäuren und polyfunktionellen Alkoholen ausgewählt ist.

**31.** Pharmazeutische Depotzusammensetzung nach Anspruch 28, bei der die Zusammensetzung bei Injektion in ein Säugetier eine lang anhaltende antipsychotische Wirkung bereitstellt.

**32.** Pharmazeutische Depotzusammensetzung nach Anspruch 29, bei der die Zusammensetzung bei Injektion in ein Säugetier eine lang anhaltende antipsychotische Wirkung bereitstellt.

**33.** Pharmazeutische Depotzusammensetzung nach Anspruch 30, bei der die Zusammensetzung bei Injektion in ein Säugetier eine lang anhaltende antipsychotische Wirkung bereitstellt.

**34.** Verbindung nach Anspruch 1, worin es sich bei einem oder mehreren der darin enthaltenden Atome um ein Radionuklid handelt.

**35.** Verbindung nach Anspruch 1, worin R für die Gruppe (a) mit einem radioaktiv markierten $^{14}C$ in der 3-Stellung des Benzo[b]thiophenringsystems steht, L für Trifluormethyl steht, p für 1 steht, $R_3$ für Wasserstoff steht, n für 1 steht, i für 0 steht und A für N steht.

**36.** Verfahren zur Herstellung einer Verbindung nach Anspruch 1, worin Y für N steht, bei dem man: eine Verbindung der Formel (II)

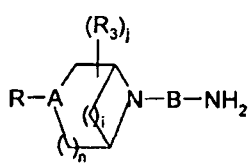

(II)

worin R, $R_3$, j, n, i, B und A die unter Formel I von Anspruch 1 angegebene Bedeutung besitzen, mit einer Verbindung der Formel (III)

$$X{=}C{=}N{-}R_2 \qquad (III)$$

worin X und $R_2$ die unter Formel I von Anspruch 1 angegebene Bedeutung besitzen, umsetzt.

**37.** Verfahren zur Herstellung einer Verbindung der Formel I, worin Y für O steht, bei dem man: eine Verbindung der Formel (II)

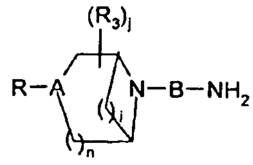

(II)

worin R, $R_3$, j, n, i, B und A die unter Formel I von Anspruch 1 angegebene Bedeutung besitzen, mit einer Verbindung der Formel IV

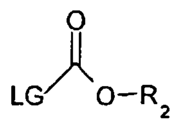

(IV)

worin "LG" für eine geeignete, unter Brom, Chlor oder Iod ausgewählte Abgangsgruppe steht und $R_2$ die unter Formel I von Anspruch 1 angegebene Bedeutung besitzt, umsetzt.

**38.** Verfahren zur Herstellung von Verbindungen der Formel I, bei dem man eine Verbindung der Formel (V)

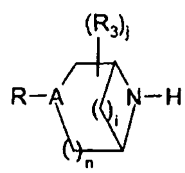

(V)

worin $R_3$, j, R, A, i und n die unter Formel I von Anspruch 1 angegebene Bedeutung besitzen, mit einer Verbindung der Formel (VI)

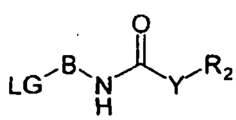

(VI)

worin "LG" für eine geeignete, unter Chlor, Brom, Iod oder Mesyl ausgewählte Abgangsgruppe steht und B, Y und $R_2$ die unter Formel I von Anspruch 1 angegebene Bedeutung besitzen, umsetzt.

**39.** Verbindung nach Anspruch 1, worin R für die Gruppe (a) steht.

**40.** Verbindung nach Anspruch 1, worin R für die Gruppe (b) steht.

**41.** Verbindung nach Anspruch 1, worin R für die Gruppe (c) steht.

**42.** Verwendung einer Verbindung nach Anspruch 1 zur Herstellung eines Arzneimittels zur Behandlung von Nierenfunktionsstörungen.

## Revendications

**1.** Composé de formule (I) :

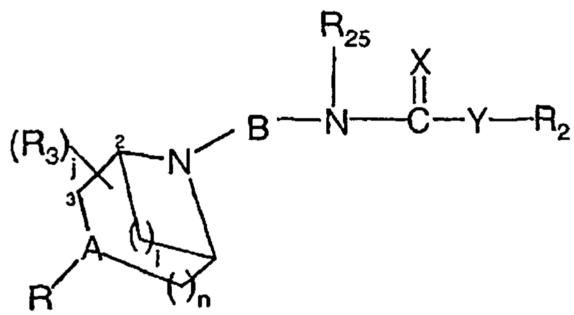

**(I)**

dans laquelle :

A est un groupe CH ou un atome d'azote ;
Y est un atome d'oxygène ou un groupe $NR_1$
dans lequel $R_1$ est :

a) un atome d'hydrogène ;
b) un groupe alkyle en $C_1$-$C_6$ ; ou
c) un groupe

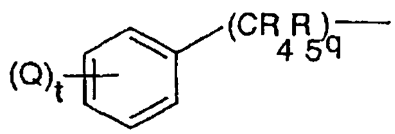

dans lequel :

chaque Q est indépendamment un atome d'hydrogène, un groupe alkyle en $C_1$-$C_6$, un atome d'halogène ou groupe trifluorométhyle ;
chaque $R_4$ et chaque $R_5$ sont indépendamment un atome d'hydrogène ou un groupe alkyle en $C_1$-$C_6$ ;
t vaut 0 ou 1 ; et
q vaut 0 ou 1 ;

j vaut 0, 1 ou 2 ;
n vaut 1 ou 2 ;
lorsque n vaut 1, i vaut 0 ou 2 ;
lorsque n vaut 2, i vaut 0 ;
$R_3$ est un atome d'hydrogène, un groupe alkyle en $C_1$-$C_6$ ou un groupe

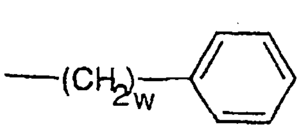

dans lequel :

w vaut 1, 2, ou 3 ;
X est un atome d'oxygène ou un atome de soufre ;
$R_{25}$ est un atome d'hydrogène ou un groupe

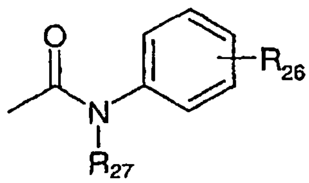

dans lequel $R_{27}$ est un atome d'hydrogène ou un groupe alkyle en $C_1$-$C_6$ et $R_{26}$ est un atome d'hydrogène, un groupe alcoxy en $C_1$-$C_6$, un atome d'halogène ou un groupe alkyle en $C_1$-$C_6$ qui est insaturé ou saturé ;
-B- est un groupe choisi parmi (a) - (e) :

(a) - $(CR_{21}R_{22})m$-
(b) un groupe

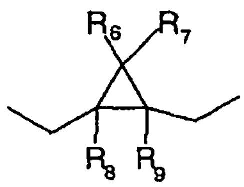

(c) un groupe

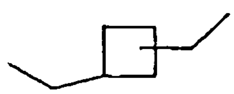

(d) un groupe

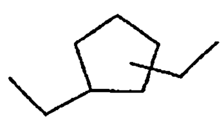

(e) un groupe

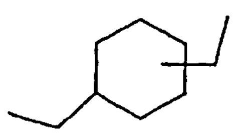

dans lesquels :

m vaut 3, 4, 5, ou 6 ;
chaque $R_{21}$ et chaque $R_{22}$ est indépendamment un atome d'hydrogène, un groupe alkyle en $C_1$-$C_6$ ou un groupe - $(CH_2)_aOH$ dans lequel a vaut 0, 1 ou 2 ;

$R_6$, $R_7$, $R_8$ et $R_9$ sont chacun indépendamment un atome d'hydrogène, un atome d'halogène ou un groupe alkyle en $C_1$-$C_3$, à condition que lorsque $R_8$ est un groupe alkyle en $C_1$-$C_3$, $R_9$ ne soit pas un groupe alkyle en $C_1$-$C_3$ ;

R est un groupe choisi parmi (a)-(c) :

(a) un groupe

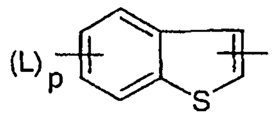

(b) un groupe

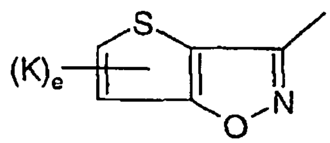

(c) un groupe

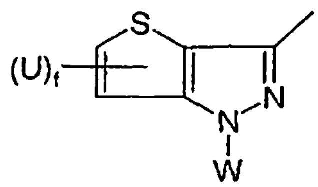

dans lesquels :

chaque L est indépendamment un atome d'hydrogène, un groupe alkyle en $C_1$-$C_6$, un atome d'halogène ou un groupe trifluorométhyle ;
chaque U est indépendamment un atome d'hydrogène, un groupe alkyle en $C_1$-$C_6$, un groupe CHO, un atome d'halogène, un groupe - $(CH_2)_bOH$ ou un groupe - $(CH_2)_bO$-$C_1$-$C_6$-alkyle, dans lesquels b vaut 1 ou 2 ;
chaque K est indépendamment un atome d'hydrogène, un groupe alkyle en $C_1$-$C_6$, un groupe CHO, un atome d'halogène, un groupe -$(CH_2)_bOH$ ou un groupe -$(CH_2)_bO$-$C_1$-$C_6$-alkyle, dans lesquels b est tel que défini ci-dessus ;
W est un atome d'hydrogène ou un groupe

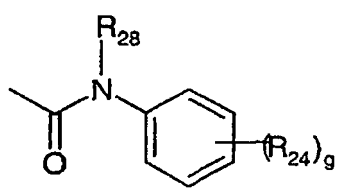

dans lequel :

$R_{28}$ est un atome d'hydrogène ou un groupe alkyle en $C_1$-$C_6$ ;

chaque $R_{24}$ est indépendamment un groupe trifluorométhyle, un groupe trifluorométhoxy, un groupe alcoxy en $C_1$-$C_6$ ou un atome d'halogène ; et
g vaut 0, 1 ou 2 ;

p vaut 0, 1 ou 2 ;
e vaut 0, 1 ou 2 ;
f vaut 0, 1 ou 2 ;

$R_2$ est un groupe choisi parmi (a)-(t) :

(a) un groupe

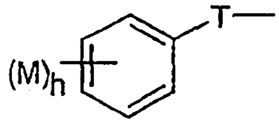

(b) un groupe

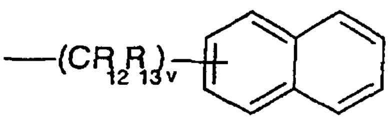

(c) un groupe

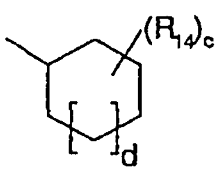

(d) un groupe

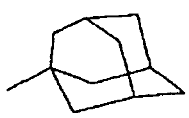

(e) un groupe

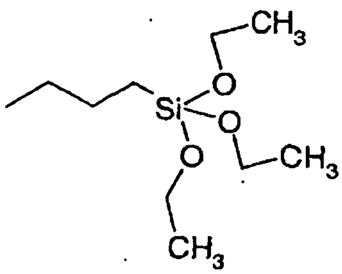

(f) un groupe -$C_1$-$C_{12}$-alkyle
(g) un groupe - $(CR_{15}$-$R_{16})_s$-$CO_2$-$C_1$-$C_6$-alkyle ;
(h) un groupe

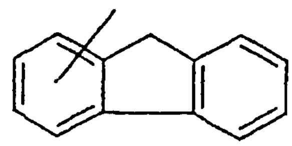

(i) un groupe

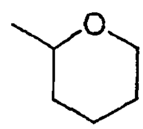

(j) un groupe

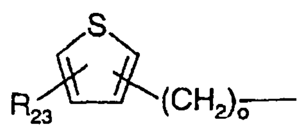

(k) un groupe

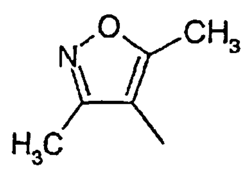

(l) un groupe -$CH_2CH_2SO_2CH_3$
(m) un groupe - $(CR_{17}$-$R_{18})_r$-O-$C_1$-$C_2$-alkyle
(n) un groupe -$CH_2CH_2OPh$
(o) un groupe - $(CR_{19}R_{20})_q CZ_3$
(p) un groupe -$CO_2Ph$
(q) un groupe -$COCV_3$
(r) un groupe

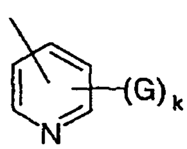

(s) un groupe

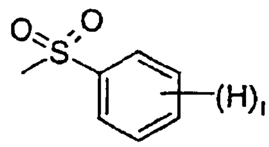

(t) un groupe

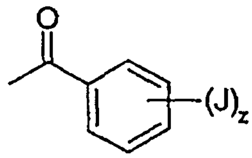

dans lesquels :

-T- est choisi parmi (i) ou (ii) :

(i) un groupe - $(CR_{10}\text{-}R_{11})_u$-
dans lequel :

u vaut 0, 1 ou 2 ; et
chaque $R_{10}$ et chaque $R_{11}$ sont indépendamment un atome d'hydrogène ou un groupe alkyle en $C_1$-$C_6$ ;

(ii) un groupe

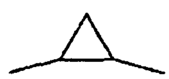

chaque M, chaque G, chaque H et chaque J sont choisis indépendamment parmi :

(1) un atome d'hydrogène ;
(2) un atome d'halogène ;
(3) un groupe alkyle en $C_1$-$C_6$ ;
(4) un groupe alcoxy en $C_1$-$C_6$ ;
(5) un groupe phénoxy ;
(6) un groupe phényle ;
(7) un groupe trifluorométhyle ;
(8) un groupe trifluorométhoxy ;
(9) un groupe $-CO_2R_{19}$, dans lequel $R_{19}$ est un atome d'hydrogène ou un groupe alkyle en $C_1$-$C_6$ ;
(10) un groupe -CO-$C_1$-$C_6$-alkyle ;
(11) un groupe hydroxy ;
(12) un groupe - $(CH_2)$-$OR_{20}$, dans lequel $R_{20}$ est un atome d'hydrogène ou un groupe alkyle en $C_1$-$C_6$ ;
(13) un groupe - $C(CH_2)CH_3$ ;
(14) un groupe - $NO_2$ ;
(15) un groupe - $SCH_3$ ;
(16) un groupe benzyloxy ; et
(17) un groupe -CN ;

chaque $R_{12}$ et chaque $R_{13}$ sont indépendamment un atome d'hydrogène ou un groupe alkyle en $C_1$-$C_6$ ;
v vaut 0 ou 1 ;

$R_{14}$ est un atome d'hydrogène ou un groupe alkyle en $C_1$-$C_4$ ;
c vaut 0, 1 ou 2 ;
d vaut 0 ou 1 ;
chaque $R_{15}$ et chaque $R_{16}$ sont indépendamment un atome d'hydrogène ou un groupe alkyle en $C_1$-$C_6$ ;
s vaut 0, 1, 2, 3, 4 ou 5 ;
o vaut 1 ou 2 ;
chaque $R_{17}$ et chaque $R_{18}$ sont indépendamment un atome d'hydrogène, un groupe alkyle en $C_1$-$C_6$ ou un groupe $CO_2$-$C_1$-$C_2$ alkyle ;
r vaut 2 ou 3 ;
chaque $R_{19}$ et chaque $R_{20}$ sont indépendamment un atome d'hydrogène ou un groupe alkyle en $C_1$-$C_2$ ;
$R_{23}$ est un atome d'hydrogène ou un groupe alkyle en $C_1$-$C_4$ ;
Z est un atome de chlore ou un atome de fluor ;
q vaut 0 ou 1 ;
V est un atome de chlore ou un atome de fluor ; et
h, k, l et z valent 0, 1, 2 ou 3.

**2.** Composé selon la revendication 1, dans lequel X est un atome d'oxygène, Y est un groupe NH, et -B- est un groupe (a).

**3.** Composé selon la revendication 2, dans lequel $R_2$ est un groupe (a).

**4.** Composé selon la revendication 3, dans lequel m vaut 4, M est un atome d'hydrogène, un groupe alkyle en $C_1$-$C_6$, un groupe alcoxy en $C_1$-$C_6$ ou un groupe phényle, et T est un groupe (i) dans lequel u vaut 0 ou 1.

**5.** Composé selon la revendication 2, dans lequel $R_2$ est un groupe (c).

**6.** Composé selon la revendication 5, dans lequel m vaut 4, d vaut 1 et $R_{14}$ est un groupe alkyle en $C_1$-$C_6$.

**7.** Composé selon la revendication 1, dans lequel X est un atome d'oxygène, Y est un groupe NH, et -B- est un groupe (b).

**8.** Composé selon la revendication 6, dans lequel $R_2$ est un groupe (a).

**9.** Composé selon la revendication 7, dans lequel $R_6$, $R_7$, $R_8$ ou $R_9$ sont un atome d'hydrogène ; M est un atome d'hydrogène, un groupe alkyle en $C_1$-$C_6$, un groupe alcoxy en $C_1$-$C_6$ ou un groupe phényle, et T vaut 0 ou 1.

**10.** Composé selon la revendication 6, dans lequel $R_2$ est un groupe (c).

**11.** Composé selon la revendication 9, dans lequel $R_6$, $R_7$, $R_8$ ou $R_9$ sont un atome d'hydrogène, d vaut 1, et $R_{14}$ est un groupe alkyle en $C_1$-$C_6$.

**12.** Composé selon la revendication 1, qui est la 1-(4-méthoxy-phényl)-3-{4-[4-(6-trifluorométhyl-benzo[b]thiophén-3-yl)-pipérazin-1-yl]-butyl}-urée.

**13.** Composé selon la revendication 1, qui est la 1-p-tolyl-3-{4-[4-(6-trifluorométhyl-benzo[b]thiophén-3-yl)-pipérazin-1-yl]-butyl}-urée.

**14.** Composé selon la revendication 1, qui est la 1-{4-[4-(2,6-difluoro-benzo[b]thiophén-3-yl)-pipérazin-1-yl]-butyl}-3-p-tolyl-urée.

**15.** Composé selon la revendication 1, qui est la 1-{4-[4-(2-chloro-6-fluoro-benzo[b]thiophén-3-yl)-pipérazin-1-yl]-butyl}-3-(4-chloro-phényl)-urée.

**16.** Composé selon la revendication 1, qui est la 1- (4-acétyl-phényl) -3- [4- (4-thiéno [2, 3-d] isoxazol-3-yl-pipéridin-1-yl)-butyl]-urée.

**17.** Composé selon la revendication 1, qui est la 1-(4-éthoxy-phényl)-3-[4-(4-thiéno[2,3-d] isoxazol-3-yl-pipéridin-1-yl)-butyl]-urée.

**18.** Composé selon la revendication 1, qui est la 1-(4-éthoxy-phényl)-3-{4-[4-(6-fluoro-benzo[b]thiophén-3-yl)-[1,4]diazépan-1-yl]-butyl}-urée.

**19.** Composé selon la revendication 1, qui est la 1-{4-[4-(6-fluoro-benzo[b]thiophén-3-yl)-[1,4]diazépan-1-yl]-butyl}-3-(2-fluoro-phényl)-urée.

**20.** Composé selon la revendication 1, qui est la 1-{4- [4- (6-fluoro-benzo [b] thiophén-3-yl) - [1,4] diazépan-1-yl]-butyl}-3-(3-trifluorométhyl-phényl)-urée.

**21.** Composé selon la revendication 1, qui est la 1-{4- [4- (6-fluoro-benzo [b] thiophén-3-yl) - [1,4]diazépan-1-yl]-butyl}-3-(4-méthoxy-phényl)-urée.

**22.** Composé selon la revendication 1, qui est la 1-(3-imidazol-1-yl-propyl)-3-{(1R, 2R)-2-[4-(6-trifluorométhyl-benzo[b]thiophén-3-yl)-pipérazin-1-ylméthyl]-cyclopropylméthyl}-urée.

**23.** Utilisation d'un composé selon la revendication 1 pour la fabrication d'un médicament destiné au traitement de pathologies ou de troubles du système nerveux central, dans laquelle le trouble du système nerveux central est choisi parmi des troubles psychotiques, une dépendance à des substances toxiques, un abus de substances toxiques, des troubles dyskinétiques, la démence, des troubles d'anxiété, des troubles du sommeil, des troubles du rythme circadien, des troubles de l'humeur et la nausée.

**24.** Utilisation d'un composé selon la revendication 1 pour la fabrication d'un médicament destiné au traitement de la schizophrénie.

**25.** Composition pharmaceutique comprenant une quantité efficace d'un composé selon la revendication 1 avec un support ou un diluant pharmaceutiquement acceptable.

**26.** Composition pharmaceutique comprenant une quantité efficace d'un composé selon la revendication 1 avec un support ou un diluant pharmaceutiquement acceptable, conjointement avec un ou plusieurs antagonistes de récepteurs dopaminergiques $D_1$, $D_2$, $D_4$, $D_5$ ou 5HT.

**27.** Composition pharmaceutique à effet retard, comprenant un support pharmaceutiquement acceptable et une quantité thérapeutiquement efficace du composé selon la revendication 1, dans laquelle le composé renferme un groupe hydroxy-acylé ou un groupe amino-acylé.

**28.** Composition pharmaceutique à effet retard selon la revendication 27, dans laquelle le groupe hydroxy est acylé, ou le groupe amino est acylé avec un groupe alcanoyle en $C_4$-$C_{18}$ ou un groupe $C_4$-$C_{18}$-alcoxycarbonyle.

**29.** Composition selon la revendication 25, contenant une huile pharmaceutiquement acceptable.

**30.** Composition selon la revendication 28, dans laquelle l'huile est choisie dans le groupe constitué de l'huile de coprah, de l'huile d'arachide, de l'huile de sésame, de l'huile de graines de coton, de l'huile de maïs, de l'huile de soja, de l'huile d'olive et d'esters synthétiques d'acides gras et d'alcools polyfonctionnels.

**31.** Composition pharmaceutique à effet retard selon la revendication 28, la composition procurant un effet antipsychotique à longue durée d'action par injection chez un mammifère.

**32.** Composition pharmaceutique à effet retard selon la revendication 29, la composition procurant un effet antipsychotique à longue durée d'action par injection chez un mammifère.

**33.** Composition pharmaceutique à effet retard selon la revendication 30, la composition procurant un effet antipsychotique à longue durée d'action par injection chez un mammifère.

**34.** Composé selon la revendication 1, dans lequel un ou plusieurs des atomes qui y sont présents est (sont) un radionucléide.

**35.** Composé selon la revendication 1, dans lequel R est un groupe (a) avec un atome $^{14}C$ radio-marqué en position 3 du système de noyau benzo[b]thiophène, L est un groupe trifluorométhyle, p vaut 1, $R_3$ est un atome d'hydrogène, n vaut 1, i vaut 0, et A est un atome d'azote.

**36.** Procédé de préparation d'un composé selon la revendication 1, dans lequel Y est un atome d'azote, comprenant :

la réaction d'un composé de formule (II)

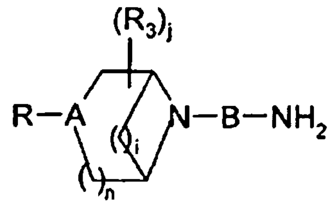

(II)

dans laquelle R, $R_3$, j, n, i, B et A sont tels que définis dans la formule (I) selon la revendication 1, avec un composé de formule (III)

X=C=N-$R_2$ (III)

dans laquelle X et $R_2$ sont tels que définis dans la formule (I) selon la revendication 1.

**37.** Procédé de préparation d'un composé de formule (I), dans laquelle Y est un atome d'oxygène, comprenant :

la réaction d'un composé de formule (II)

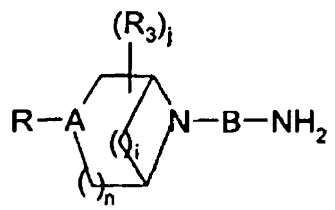

(II)

dans laquelle R, $R_3$, j , n, i, B et A sont tels que définis dans la formule (I) selon la revendication 1, avec un composé de formule (IV)

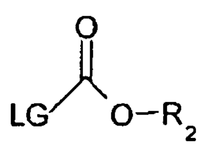

(IV)

dans laquelle "LG" est un groupe partant approprié choisi parmi un atome de brome, un atome de chlore et un atome d'iode, et $R_2$ est tel que défini dans la formule (I) selon la revendication 1.

**38.** Procédé de préparation de composés de formule (I) comprenant la réaction d'un composé de formule (V)

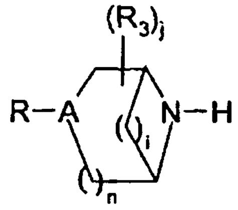

(V)

dans laquelle $R_3$, j, R, A, i et n sont tels que définis dans la formule (I) selon la revendication 1, avec un composé de formule (VI)

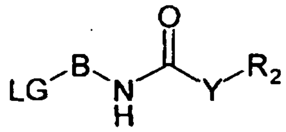

(VI)

dans laquelle "LG" est un groupe partant approprié choisi parmi un atome de chlore, un atome de brome, un atome d'iode et un groupe mésyle, et B, Y et $R_2$ sont tels que définis dans la formule (I) selon la revendication 1.

**39.** Composé selon la revendication 1, dans lequel R est un groupe (a).

**40.** Composé selon la revendication 1, dans lequel R est un groupe (b).

**41.** Composé selon la revendication 1, dans lequel R est un groupe (c).

**42.** Utilisation d'un composé selon la revendication 1 pour la fabrication d'un médicament destiné au traitement d'un dysfonctionnement rénal.